# EUROPEAN PATENT APPLICATION

(11) **EP 4 591 884 A1**
(43) Date of publication of application: **30.07.2025**
(21) Application number: 23867498.0
(22) Date of filing: 19.09.2023
(51) Int. Cl.: A61K 47/18, A61K 48/00, A61P 35/00

(54) **LIPID COMPOUND FOR NUCLEIC ACID DELIVERY, AND RELATED USE AND MEDICAMENT COMPRISING SAME**

(30) Priority: 20.09.2022 CN 202211144019; 16.06.2023 CN 202310721211; 16.08.2023 CN 202311034019
(71) Applicant: Beijing Baishihekang Pharmaceutical Technology (Bsjpharma) Co., Ltd., Beijing 100192 (CN)
(72) Inventor: JIANG, Chengyu, Beijing 100005 (CN); DU, Xinyi, Beijing 100005 (CN); LIU, Jiaqi, Beijing 100005 (CN)
(74) Representative: Jones Day
(86) International application number: PCT/CN2023/119703
(87) International publication number: WO 2024/061211

(57) **Abstract**

Provided are a lipid compound for nucleic acid delivery, and related use and a medicament comprising same. Particularly, provided are use of a lipid compound comprising one or more compounds represented by the following formula (I) in preparing a product for nucleic acid delivery, a pharmaceutical composition comprising the lipid compound and a nucleic acid molecule, and use of the pharmaceutical composition.

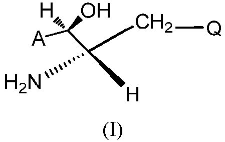

## Description

### TECHNICAL FIELD

The present application relates to an artificially synthesized lipid capable of promoting nucleic acid delivery, and a specific combination of the artificially synthesized lipid and a nucleic acid that is capable of promoting nucleic acid target cells and target organs.

### BACKGROUND

Over the past decades, the concept of using nucleic acid molecules, including RNA molecules, as therapeutic medicaments has moved from concept to clinical reality. In fact, nucleic acid molecules have many properties that make them useful as therapeutic medicaments. They can fold into complex conformations that allow them to interact with proteins, small molecules, or other nucleic acids, and some can even form catalytic centers. Usually, people generally refer to siRNA, miRNA, and other non-coding small RNAs as small nucleic acids or small RNA (sRNA) without distinction. However, nucleic acids are generally considered a poor choice as therapeutic medicaments because they are easily degraded and have a short half-life in vivo.

Therefore, how to effectively deliver nucleic acid molecules, including small RNAs, to cells and target organs in a body to achieve their biological activities and therapeutic or preventive effects is an issue that those skilled in the art need to consider.

### SUMMARY OF THE INVENTION

The present invention is based in part on the inventors' discovery of the delivery functionality of a series of sphingosine lipids. The inventors unexpectedly found that a combination of a sphingosine lipid within a certain carbon number range and a nucleic acid or a combination of a sphingosine and a helper lipid within a certain carbon number range and a nucleic acid can effectively deliver nucleic acids into cells and deliver nucleic acids into target organs.

In a first aspect, the present invention provides use of a lipid composition in the preparation of a product/reagent for delivering nucleic acids, the lipid composition comprising one or more compounds having the following formula (I): 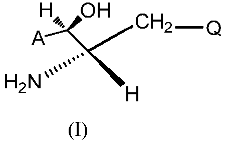 wherein
A is selected from linear C₁₀₋₃₄ alkyl groups and linear C₁₀₋₃₄ alkenyl groups;
and Q is -OH.

In a specific embodiment, the present invention provides use of a lipid composition in the preparation of a product/reagent for delivering nucleic acids, the lipid composition comprising one or more compounds having the following formula (I): 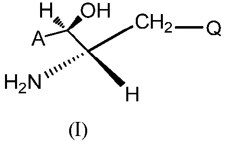 wherein
A is selected from linear C₁₀₋₃₂ alkyl groups and linear C₁₀₋₃₂ alkenyl groups;
and Q is -OH.

In a specific embodiment, the present invention provides use of a lipid composition in the preparation of a product/reagent for delivering nucleic acids, the lipid composition comprising one or more compounds having the following formula (I): 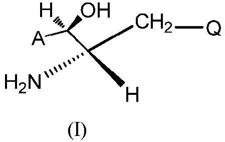 wherein
A is selected from linear C₁₁₋₃₁ alkyl groups and linear C₁₁₋₃₁ alkenyl groups;
and Q is -OH.

In a specific embodiment, the present invention provides use of a lipid composition in the preparation of a product/reagent for delivering nucleic acids, the lipid composition comprising one or more compounds having the following formula (I): 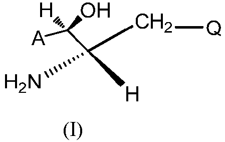 wherein
A is selected from linear C₂₁₋₃₄ alkyl groups or linear C₂₁₋₃₄ alkenyl group;
and Q is -OH.

Preferably, the A of the present invention is selected from linear C₂₁₋₃₂ alkyl groups and linear C₂₅₋₃₂ alkenyl group; more preferably, the A is selected from linear C₂₅₋₃₀ alkyl groups and linear C₂₅₋₃₀ alkenyl groups.

In a specific embodiment, the product/reagent for delivering nucleic acids provided by the present invention delivers a nucleic acid into the body of a subject, and preferably, the reagent is used to deliver a nucleic acid into the body of a subject via oral, intramuscular, intravenous, subcutaneous, transdermal, intraarterial, intraperitoneal, intrapulmonary, intracerebrospinal, intraarticular, intrasynovial, intrathecal, intraventricular, and/or inhalation route.

In a specific embodiment, the product/reagent for nucleic acid delivery provided by the present invention delivers nucleic acids to cells in vitro, and preferably, delivers nucleic acids to cells in vitro by direct contact.

In a specific embodiment, the lipid composition provided by the present invention may be used to deliver small RNA molecules, preferably, the small RNA molecule is a small RNA molecule with a length of 14-32 nucleotides, and preferably, the nucleic acid molecule is a small RNA molecule with a length of 14, 15, 16, 17, 18, 19, 20, 21, 22, 23, 24, 25, 26, 27, 28, 29, 30, 31, or 32 nucleotides.

In another aspect, the present invention provides a pharmaceutical composition comprising a lipid composition and a nucleic acid molecule, wherein the lipid composition comprises one or more compounds of formula (I): 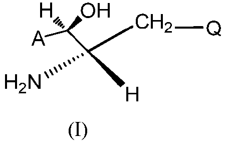 wherein
A is selected from linear C₁₀₋₃₄ alkyl groups and linear C₁₀₋₃₄ alkenyl groups;
and Q is -OH.

In a specific embodiment, the present invention provides a pharmaceutical composition comprising a lipid composition and a nucleic acid molecule, wherein the lipid composition comprises one or more compounds of formula (I): 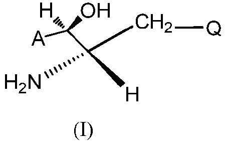 wherein
A is selected from linear C₁₀₋₃₂ alkyl groups and linear C₁₀₋₃₂ alkenyl groups;
and Q is -OH.

In a specific embodiment, the present invention provides a pharmaceutical composition comprising a lipid composition and a nucleic acid molecule, wherein the lipid composition comprises one or more compounds of formula (I): 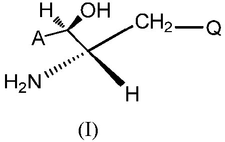 wherein
A is selected from linear C₁₁₋₃₁ alkyl groups and linear C₁₁₋₃₁ alkenyl groups;
and Q is -OH.

In a specific embodiment, the present invention provides a pharmaceutical composition comprising a lipid composition and a nucleic acid molecule, wherein the lipid composition comprises one or more compounds of formula (I): 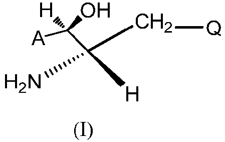 wherein
A is selected from linear C₂₁₋₃₄alkyl groups or linear C₂₁₋₃₄alkenyl groups;
and Q is -OH.

Preferably, the A of the present invention is selected from linear C₂₁₋₃₂ alkyl groups and linear C₂₅₋₃₂ alkenyl groups; and more preferably, the A is selected from linear C₂₅₋₃₀ alkyl groups and linear C₂₅₋₃₀ alkenyl groups.

In a specific embodiment, the nucleic acid molecule is an RNA molecule or a DNA molecule, preferably, the RNA molecule is a small RNA molecule, preferably, it is a small RNA molecule with a length of 14-32 nucleotides, and preferably, it is a small RNA molecule with a length of 14, 15, 16, 17, 18, 19, 20, 21, 22, 23, 24, 25, 26, 27, 28, 29, 30, 31, or 32 nucleotides.

In another aspect, the present invention provides a method for preparing the pharmaceutical composition of the present invention as described above, the method comprising a step of mixing the lipid composition with a nucleic acid molecule.

In another aspect, the present invention provides use of the pharmaceutical composition described herein in the preparation of a medicament for treating a disease in a subject, and preferably, the medicament is administered to the subject by oral, intramuscular, intravenous, subcutaneous, transdermal, intraarterial, intraperitoneal, intrapulmonary, intracerebrospinal, intraarticular, intrasynovial, intrathecal, intraventricular, and/or inhalation route.

In another aspect, the present invention provides a compound as shown in formula (I): 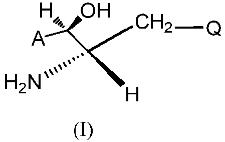 wherein
A is selected from linear C₁₀₋₃₄ alkyl groups and linear C₁₀₋₃₄ alkenyl groups;
and Q is -OH.

In a specific embodiment, the present invention provides a compound as shown in formula (I): 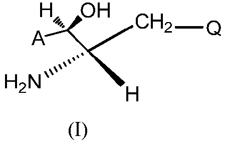 wherein
A is selected from linear C₁₀₋₃₂ alkyl groups and linear C₁₀₋₃₂ alkenyl groups;
and Q is -OH.

In a specific embodiment, the present invention provides a compound as shown in formula (I): 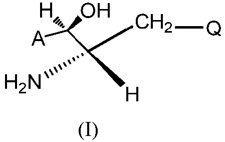 wherein
A is selected from linear C₁₁₋₃₁ alkyl groups and linear C₁₁₋₃₁ alkenyl groups;
and Q is -OH.

In another aspect, the present invention provides a composition for in vitro cell transfection, comprising a lipid composition and a nucleic acid molecule, wherein the lipid composition comprises one or more compounds of formula (I): 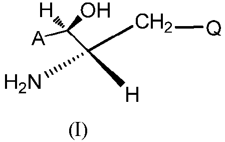 wherein
A is selected from linear C₁₀₋₃₄ alkyl groups or linear C₁₀₋₃₄ alkenyl groups, preferably linear C₁₀₋₃₂ alkyl groups or linear C₁₀₋₃₂ alkenyl groups;
and Q is -OH.

Preferably, the A of the present invention is selected from linear C₂₁₋₃₂ alkyl groups and linear C₂₅₋₃₂ alkenyl groups; and more preferably, the A is selected from linear C₂₅₋₃₀ alkyl groups and linear C₂₅₋₃₀ alkenyl groups.

Specifically, the lipid composition comprises one or more compounds shown in Table 1.

In a specific embodiment, the nucleic acid molecule is an RNA molecule or a DNA molecule, preferably, the RNA molecule is a small RNA molecule, preferably, it is a small RNA molecule with a length of 14-32 nucleotides, and preferably, it is a small RNA molecule with a length of 14, 15, 16, 17, 18, 19, 20, 21, 22, 23, 24, 25, 26, 27, 28, 29, 30, 31, or 32 nucleotides.

Exemplarily, the small RNA molecule of the present invention is selected from the small RNA molecule PGY-ssRNA-26.

In a specific embodiment, the present invention provides a compound as shown in formula (I): 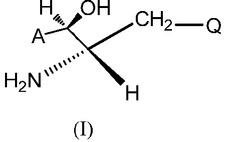 wherein
A is selected from linear C₂₁₋₃₄ alkyl groups and linear C₂₁₋₃₄ alkenyl groups; preferably, the A is selected from linear C₂₁₋₃₂ alkyl groups and linear C₂₅₋₃₂ alkenyl groups; and more preferably, the A is selected from linear C₂₅₋₃₀ alkyl groups and linear C₂₅₋₃₀ alkenyl groups;
and Q is -OH,
and preferably, the compound is a compound shown in Table 1.

In one embodiment of the present invention, the present invention provides a package set, comprising a compound represented by formula (I) as described herein, or a lipid composition, or a product/reagent for nucleic acid delivery, or a pharmaceutical composition, and a nucleic acid, wherein the compound represented by formula (I), or a lipid composition, or a reagent for nucleic acid delivery, or a pharmaceutical composition and the nucleic acid are each independently provided in a first container and a second container, and the first container and the second container are the same or different. Preferably, the package set contains any one or more of the above compounds, preferably any one or more of the compounds selected from Table 1.

In one embodiment of the present invention, the present invention provides a method for nucleic acid delivery, which comprises administering to a subject a compound represented by formula (I), or a lipid composition, or a product/reagent for nucleic acid delivery, or a pharmaceutical composition as described herein.

In one embodiment of the present invention, the present invention provides a method for treating a disease in a subject, which comprises administering to the subject a compound represented by formula (I), or a lipid composition, or a product/reagent for nucleic acid delivery, or a pharmaceutical composition as described herein.

In a specific embodiment, the disease comprises cancer, inflammation, fibrotic diseases, autoimmune diseases, infections, congenital and hereditary diseases, connective tissue diseases, digestive system diseases, endocrine diseases, eye diseases, reproductive diseases, cardiovascular diseases, renal and urinary diseases, respiratory diseases, metabolic disorders, musculoskeletal diseases, nervous system diseases, and blood system diseases.

In a specific embodiment, the cancer is selected from lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, kidney cancer, squamous cell carcinoma, or blood system cancer.

In a specific embodiment, the medicament is administered to a subject by oral, intramuscular, intravenous, subcutaneous, transdermal, intraarterial, intraperitoneal, intrapulmonary, intracerebrospinal, intraarticular, intrasynovial, intrathecal, intraventricular, and/or inhalation routes.

In another aspect, the present invention provides a method for preparing a compound as shown in formula (I), wherein the method is selected from any one of the following:

### Method a.

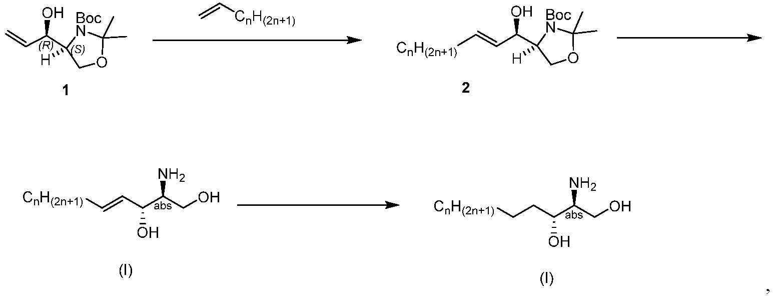

Step a1. reacting a compound represented by formula 1 with an olefin to generate a compound represented by formula 2;
Step a2. converting the compound represented by formula 2 to a compound represented by formula (I) in which A is a linear alkenyl group;

Optional step a3. reducing the compound represented by formula (I) in which A is a linear alkenyl group to obtain the compound represented by formula (I) in which A is a linear alkyl group;
wherein n=9, 10, 11, 12, 13, 14, 15, 16, 17, 18, 19, 20, 21, 22, 23, 24, 25, 26, 27, 28, 29, 30, 31, or 32.

Preferably, n=9, 10, 14, 16, 18, 19, or 24.

In one embodiment, step a1 is carried out under the catalysis by benzal-[1,3-bis(2,4,6-trimethylphenyl)imidazolidine-2-methylene]-dichloro-ruthenium and tricyclohexylphosphine.

Preferably, the reaction conditions of step a1 are Grubbs 2^{nd}, DCM, 40°C.

In one embodiment, step a2 is carried out in the presence of TFA.

Preferably, the reaction conditions of step a2 are TFA, ACN, H₂O, 80°C.

In one embodiment, step a3 is hydrogenation reduction, preferably Pd/C catalyzed hydrogenation reduction.

Preferably, the reaction conditions of step a3 are Pd/C, H₂, MeOH/THF, 50°C.

### Method b.

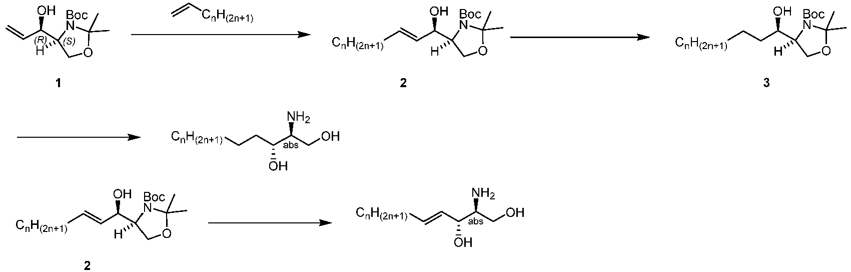

Step b1. reacting a compound represented by formula 1 with an olefin to generate a compound represented by formula 2;
Step b2-1. reducing the compound represented by formula 2 to obtain a compound represented by formula 3;
Step b3. converting the compound represented by formula 3 to a compound represented by formula (I) in which A is a linear alkyl group;
   or
Step b2-2. converting the compound represented by formula 2 to a compound represented by formula (I) in which A is a linear alkenyl group;
wherein n=9, 10, 11, 12, 13, 14, 15, 16, 17, 18, 19, 20, 21, 22, 23, 24, 25, 26, 27, 28, 29, 30, 31, or 32.

Preferably, n=20, 21, 22, 23, 25, 26, 27, 28, 29, 30, or 31.

In one embodiment, step b1 is the same as step a1.

In one embodiment, step b2-1 is hydrogenation reduction, preferably Pd/C catalyzed hydrogenation reduction.

Preferably, the reaction conditions of step b2-1are Pd/C, H₂, MeOH/THF, 50°C.

In one embodiment, step b3 is carried out in the presence of TFA.

Preferably, the reaction conditions of step b3 are TFA, ACN, H₂O, 80°C.

In one embodiment, step 2-2 is carried out in the presence of TFA.

Preferably, the reaction conditions of step b3 are TFA, ACN, H₂O, 80°C.

### Method c.

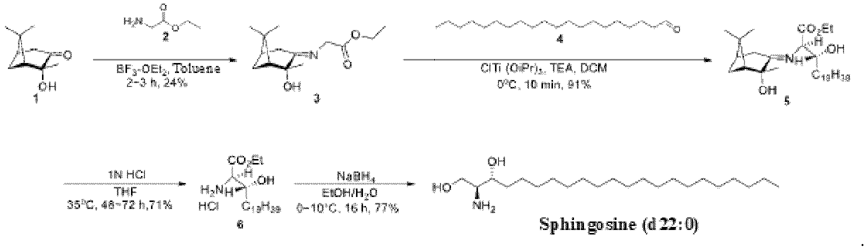

Preferably, step c further comprises:

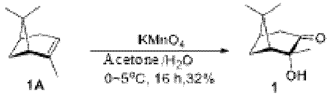

Preferably, step c further comprises:

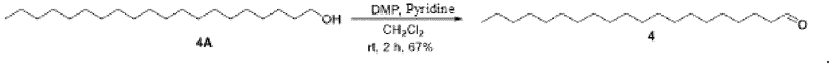

### BRIEF DESCRIPTION OF THE DRAWINGS

FIG. 1 shows results of in vitro small nucleic acid delivery in HPAC cells.
FIG. 2 shows results of in vitro small nucleic acid delivery in H460 cells.
FIG. 3 shows results of in vitro small nucleic acid delivery in 293T cells.

### DETAILED DESCRIPTION

In order to explain the present invention in more detail, this specification provides the following specific embodiments, and illustrates these specific embodiments in conjunction with the accompanying drawings, but the solutions in the present disclosure are not limited thereto. Those skilled in the art can make appropriate changes to the methods, uses, and small RNAs of the present invention in combination with common knowledge in the art. As long as they can achieve the functions described in the present invention, they shall fall within the scope of the present invention.

Unless otherwise defined, all technical and scientific terms as used herein have the same meaning as commonly understood by those skilled in the art.

In a specific embodiment of the present invention, the present invention provides use of a lipid composition in the preparation of a reagent for delivering nucleic acids, the lipid composition comprising one or more compounds having formula (I): 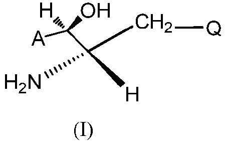
wherein A is selected from linear C₁₀₋₃₂ alkyl groups and linear C₁₀₋₃₂ alkenyl groups; optionally, A is a linear alkyl group containing 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, 20 carbon atoms, 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, 32 carbon atoms, 33 carbon atoms, or 34 carbon atoms, or is a linear alkenyl group containing 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, or 32 carbon atoms;
and Q is -OH.

In a specific embodiment, the present invention provides use of a lipid composition in the preparation of a reagent for delivering nucleic acids, the lipid composition comprising one or more compounds having formula (I): 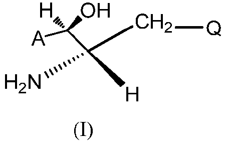 wherein
A is selected from linear C₂₁₋₃₄ alkyl groups and linear C₂₁₋₃₄ alkenyl groups; optionally, A is a linear alkyl group containing 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, 32 carbon atoms, 33 carbon atoms, or 34 carbon atoms, or is a linear alkenyl group containing 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, 32 carbon atoms, 33 carbon atoms, or 34 carbon atomss;
and Q is -OH.

In a specific embodiment, the lipid composition provided by the present invention may comprise one or more compounds of formula (I) provided by the present application or their salts, hydrates, or solvates; it may further comprise one or more lipid compounds other than the compounds provided by the present application. The other lipid compounds may be, for example, neutral lipids, charged lipids, steroids, and polymer-conjugated lipids. "Neutral lipid" refers to a lipid compound that exists in an uncharged form or in a neutral zwitterionic form at a selected pH value (e.g., a physiological pH value). "Charged lipid" refers to a lipid compound that exists in either a positively or negatively charged form regardless of a pH value within the useful physiological range (e.g., pH from about 3 to about 9).

In certain embodiments, the lipid composition may further comprise one or more solvents, which can be mixed with the compound provided by the present application or its salts, hydrates, or solvates to form a homogeneous mixture.

The lipid composition may further comprise an organic solvent or solvent mixture, such as chloroform, dichloromethane, diethyl ether, cyclohexane, cyclopentane, benzene, toluene, methanol, or other aliphatic alcohols, such as ethanol, propanol, isopropanol, butanol, tert-butanol, isobutanol, pentanol, and hexanol. These solvents may be used alone, in admixture, and/or optionally together with a suitable buffer as the solvent in the lipid composition. The selection of a solvent can generally be based on the polarity of the solvent, the ease with which the solvent can be removed at a later stage of the formation of the lipid-nucleic acid mixture, and/or pharmaceutically acceptable properties. In certain embodiments, the solvent is non-toxic or pharmaceutically acceptable. Exemplary pharmaceutically acceptable solvents comprise lower alcohols (1-6 carbon atoms), such as methanol, ethanol, n-propanol, isopropanol, and n-butanol. In certain embodiments, an appropriate amount of a solvent may be used to enable a nucleic acid and a lipid to form a clear single-phase mixture.

In specific embodiments, the lipid composition provided by the present invention comprises two or more compounds of formula (I).

In a specific embodiment of the present invention, the present invention provides a pharmaceutical composition comprising a lipid composition and a nucleic acid molecule, wherein the lipid composition comprises one or more compounds of formula (I): 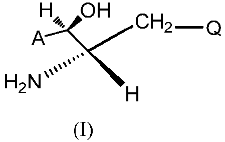
wherein A is selected from linear C₁₀₋₃₂ alkyl groups and linear C₁₀₋₃₂ alkenyl groups; optionally, A is a linear alkyl group containing 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, 20 carbon atoms, 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, 32 carbon atoms, 33 carbon atoms, or 34 carbon atoms, or is a linear alkenyl group containing 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, or 32 carbon atoms;
Q is -OH;
and preferably, the nucleic acid molecule is an RNA molecule or a DNA molecule, preferably, it is a small RNA molecule, preferably, it is a small RNA molecule with a length of 14-32 nucleotides, and preferably, it is a small RNA molecule with a length of 14, 15, 16, 17, 18, 19, 20, 21, 22, 23, 24, 25, 26, 27, 28, 29, 30, 31, or 32 nucleotides.

In a specific embodiment, the present invention provides a pharmaceutical composition comprising a lipid composition and a nucleic acid molecule, wherein the lipid composition comprises one or more compounds of formula (I): 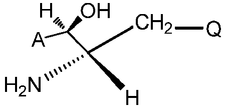 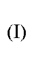 wherein
A is selected from a linear C₂₁₋₃₄alkyl group or a linear C₂₁₋₃₄alkenyl group;
Q is -OH;
and preferably, the nucleic acid molecule is an RNA molecule or a DNA molecule, preferably, it is a small RNA molecule, preferably, it is a small RNA molecule with a length of 14-32 nucleotides, and preferably, it is a small RNA molecule with a length of 14, 15, 16, 17, 18, 19, 20, 21, 22, 23, 24, 25, 26, 27, 28, 29, 30, 31, or 32 nucleotides.

In an embodiment of the pharmaceutical composition provided by the present invention, the mass ratio of the lipid composition to the nucleic acid molecule is 0.1:1, 0.2:1, 0.3:1, 0.4:1, 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1, 2:1, 2.1:1, 2.2:1, 2.3:1, 2.4:1, 2.5:1, 2.6:1, 2.7:1, 2.8:1, 2.9:1, 3:1, 4:1, 5:1, 6:1, 7:1, 8:1, 9:1, or 10:1, or a range between any of the above ratios.

In an embodiment of the pharmaceutical composition provided by the present invention, the mass ratio of the lipid composition to the nucleic acid molecule is 1:100, 1:30, 1:10, 1:3, 1:1, 3:1, 10:1, 30:1, 100:1, or a range between any of the above ratios.

In a specific embodiment, the nucleic acid molecule is an RNA molecule or a DNA molecule for treatment; preferably, the nucleic acid molecule is used to treat a disease by targeting a specific target; and optionally, the nucleic acid molecule may be used to treat cancer, inflammation, fibrotic diseases, autoimmune diseases, infections, congenital and hereditary diseases, connective tissue diseases, digestive system diseases, endocrine diseases, eye diseases, reproductive diseases, cardiovascular diseases, renal and urinary diseases, respiratory diseases, metabolic disorders, musculoskeletal diseases, nervous system diseases, and blood system diseases.

In a specific embodiment, the cancer is selected from lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, kidney cancer, squamous cell carcinoma, or blood system cancer.

In a specific embodiment, the present invention provides a method for preparing the pharmaceutical composition of the present invention, the method comprising a step of mixing the lipid composition with a nucleic acid molecule.

In a specific embodiment, the method for preparing the pharmaceutical composition of the present invention comprises:
1) a step of mixing the lipid composition with a nucleic acid molecule; and
2) at 25°C to 150°C,

preferably, at 25°C, 30°C, 35°C, 36°C, 37°C, 40°C, 45°C, 50°C, 55°C, 60°C, 65°C, 70°C, 75°C, 80°C, 85°C, 90°C, 95°C, 100°C, 110°C, 120°C, 130°C, 140°C, or 150°C,
or a temperature in any range between these points, heating the mixture obtained in step 1) for at least 5 min, for example, for 6, 7, 8, 9, 10, 11, 12, 13, 14, 15, 16, 17, 18, 19, 20, 21, 22, 23, 24, 25, 26, 27, 28, 29, or 30 min.

### Terms

The term "nucleic acid" as used herein includes "polynucleotide," "oligonucleotide," and "nucleic acid molecule," and generally refers to a DNA or RNA polymer that may be single-stranded or double-stranded, synthetic or obtained (e.g., isolated and/or purified) from a natural source; which may contain natural, non-natural, or altered nucleotides. In some embodiments, the nucleic acid does not comprise any insertions, deletions, inversions, and/or substitutions. However, as discussed herein, in some cases it may be appropriate for a nucleic acid to include one or more insertions, deletions, inversions, and/or substitutions.

The term "vector" as used herein refers to a recombinant expression vector that incorporates the nucleic acid described herein. The recombinant expression vector may be any suitable recombinant expression vector, and may be used to transform or transfect any suitable host cell, including but not limited to plant expression vectors, animal expression vectors, and viral vectors, such as retroviral vectors or lentiviral vectors. These vectors are well known to those skilled in the art and may be commercially purchased.

The term "host cell" as used herein refers to any type of cells that can be transfected with the recombinant expression vector of the present invention. The host cell may be a eukaryotic cell, such as plant, animal, fungus, or algae, or may be a prokaryotic cell, such as bacteria or protozoa.

A variety of transfection techniques are well known in the art and include, but are not limited to, calcium phosphate co-precipitation, direct microinjection into cultured cells, electroporation, liposome-mediated gene transfer, lipid-mediated transduction, and nucleic acid delivery using high-speed microprojectiles.

The term "Cᵢ₋ⱼ" as used herein represents a range of carbon atom numbers, wherein i and j are integers and j is greater than i, and the range of carbon atom numbers includes the endpoints (i.e., i and j) and each integer point between the endpoints. For example, C₂₁₋₃₄ means a range of 21 to 34 carbon atoms, including 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, 32 carbon atoms, 33 carbon atoms, or 34 carbon atoms.

The term "alkyl" as used herein refers to a saturated linear or branched hydrocarbyl group. The term "linear Cᵢ₋ⱼ alkyl groups" refers to linear alkyl groups having i to j carbon atoms. In some embodiments, an alkyl group contains 21 to 34 carbon atoms. In some embodiments, an alkyl group contains 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, 32 carbon atoms, 33 carbon atoms, or 34 carbon atoms.

In some embodiments, an alkyl group contains 10 to 34 carbon atoms, preferably 10 to 32 carbon atoms. In some embodiments, an alkyl group contains 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, 20 carbon atoms, 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, 32 carbon atoms, 33 carbon atoms, or 34 carbon atoms.

The term "alkenyl" as used herein refers to a linear or branched hydrocarbyl group having at least one carbon-carbon double bond, which may optionally be substituted independently with one or more substituents described herein, and includes groups having "cis" and "trans" orientations or "E" and "Z" orientations. In some embodiments, an alkenyl group contains 21 to 34 carbon atoms. In some embodiments, an alkenyl group contains 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, 32 carbon atoms, 33 carbon atoms, or 34 carbon atoms.

In some embodiments, an alkenyl group contains 10 to 34 carbon atoms, preferably 10 to 32 carbon atoms. In some embodiments, an alkyl group contains 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, 20 carbon atoms, 21 carbon atoms, 22 carbon atoms, 23 carbon atoms, 24 carbon atoms, 25 carbon atoms, 26 carbon atoms, 27 carbon atoms, 28 carbon atoms, 29 carbon atoms, 30 carbon atoms, 31 carbon atoms, 32 carbon atoms, 33 carbon atoms, or 34 carbon atoms.

In some embodiments, an alkenyl group contains more than one carbon-carbon double bond. It should be appreciated that, when the alkenyl group contains more than one carbon-carbon double bond, the double bonds may be separated from or conjugated with one another. In some embodiments, an alkenyl group is α-alkenyl.

The term "delivery" as used herein encompasses both local and systemic delivery. "Local delivery" refers to the delivery of a therapeutic agent (e.g., a nucleic acid) to be delivered directly to a target site within an organism. For example, a reagent can be delivered locally by direct injection into a target site (e.g., a disease site, such as a tumor or inflammation site) or target organ (e.g., the heart, spleen, lung, kidney, etc.). "Systemic delivery" refers to delivery that results in widespread biodistribution of a therapeutic agent (e.g., a nucleic acid) within an organism, thereby exposing an effective amount of the therapeutic agent to most parts of the body. To achieve broad biodistribution, a blood lifetime is generally desired in such a way that these therapeutic agents are not rapidly degraded or cleared before reaching target sites distant from the site of administration. Systemic delivery of a lipid composition can be by any suitable means, including, for example, oral, inhalation, gastrointestinal, intravenous, subcutaneous, and intraperitoneal.

The term "lipid" as used herein refers to a class of organic compounds that include, but are not limited to, esters of fatty acids, and are characterized by being insoluble in water (e.g., with the solubility in water less than about 0.01% by weight) but soluble in many organic solvents. The lipid may be, for example, simple lipids (e.g., fats, oils, and waxes), compound lipids (e.g., phospholipids and glycolipids), and derivatized lipids (e.g., steroids).

The term "treating" as used herein includes treating a disease state in a mammal (particularly in humans), and includes: (a) inhibiting the disease state, i.e., preventing its development; and/or (b) alleviating the disease state, i.e., causing remission of the disease state.

The term "subject" as used herein refers to any human or non-human organism that could potentially benefit from treatment with a nucleic acid molecule comprised in a pharmaceutical composition of the present invention. An exemplary subject comprises a patient with a disease, and in particular, a patient with cancer, inflammation, fibrotic diseases, autoimmune diseases, infections, congenital and hereditary diseases, connective tissue diseases, digestive system diseases, endocrine diseases, eye diseases, reproductive diseases, cardiovascular diseases, renal and urinary diseases, respiratory diseases, metabolic disorders, musculoskeletal diseases, nervous system diseases, and blood system diseases.

### Examples

The following examples are merely illustrative of the invention disclosed herein and should not be construed as limiting the scope of protection of the appended claims in any cases.

The following solutions describe the proposed synthetic route. By using these solutions, the following guidelines, and the examples, those skilled in the art may develop similar methods for preparing compounds within the scope of the present invention.

### Example 1. Synthesis and mass spectrometry confirmation of sphingosine Sphingosine (d14:0)

### Synthesis route map:

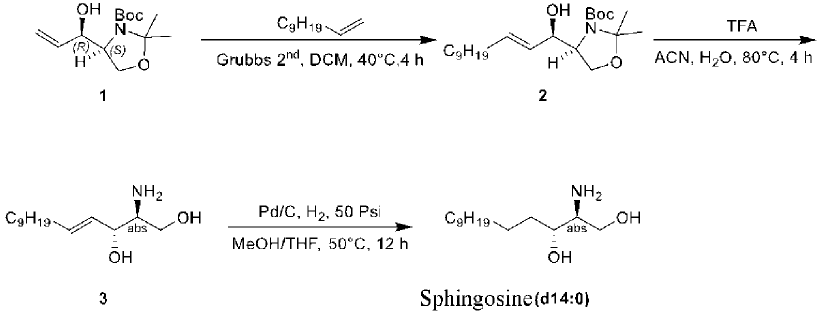

### Synthesis route:

### Step 1

### Tert-butyl (4S)-4-[(E,1R)-1-hydroxydodecyl-2-enyl]-2,2-dimethyl-oxazolidine-3-carboxylate

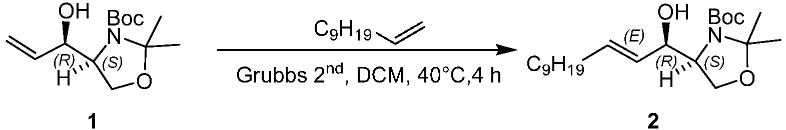

Degas a mixture of tert-butyl (4S)-4-[(1R)-1-hydroxyallyl]-2,2-dimethyl-oxazolidine-3-carboxylate (400 mg, 1.55 mmol), undec-1-ene (311.79 mg, 2.02 mmol), benzal-[1,3-bis(2,4,6-trimethylphenyl)imidazolidin-2-ylidene]-dichloro-ruthenium; and a mixture of tricyclohexylphosphine (131.97 mg, 155.45 µmol) in dichloromethane (6 mL), and replace with nitrogen three times. Stir the mixture at 40°C for 4 h under nitrogen protection. Filter, and spin dry the filtrate. Purify the crude product by silica gel flash column chromatography (eluent: 20% ethyl acetate in petroleum ether) to obtain tert-butyl (4S)-4-[(E,1R)-1-hydroxydodecyl-2-enyl]-2,2-dimethyl-oxazolidine-3-carboxylate (250 mg, 651.78 µmol, yield 41.93%), which is brown and oily.

**¹H NMR** (400 MHz, CDCl₃) δ = 5.81 - 5.71 (m, 1H), 5.15 - 4.90 (m, 1H), 2.15 - 2.01 (m, 2H), 1.28 (m, 36H), 0.91 (t, *J* = 6.8 Hz, 3H).

### Step 2

### (E,2S,3R)-2-aminotetradecyl-4-ene-1,3-diol

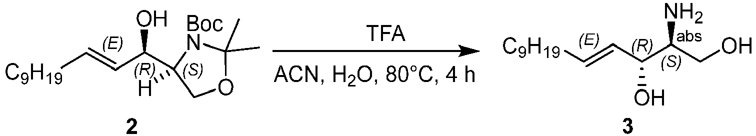

Add an aqueous solution (2 mL) of trifluoroacetic acid (46.05 mg, 403.87 µmol, 30 µL) to an acetonitrile solution (2 mL) of tert-butyl (4S)-4-[(E,1R)-1-hydroxydodecyl-2-enyl]-2,2-dimethyl-oxazolidine-3-carboxylate (250 mg, 651.78 µmol). Stir the mixture at 80°C for 4 h. Add a saturated aqueous solution of sodium bicarbonate to adjust the pH value to 8. Extract three times with ethyl acetate (10 mL). Wash the combined organic phases with saturated brine (5 mL), dry with anhydrous Na₂SO₄, and filter. Concentrate the filtrate under reduced pressure to obtain (E, 2S, 3R)-2-aminotetradecyl-4-ene-1,3-diol (150 mg, 616.31 µmol, yield 94.56%), which is a colorless solid.

**LCMS** Rt = 3.756 min in 7 min chromatography, 10-80AB ESI calculated value C₁₄H₃₀NO₂ [M+H]⁺ 244.3, and measured value 244.1

### Step 3

### (2S,3R)-2-aminotetradecane-1,3-diol

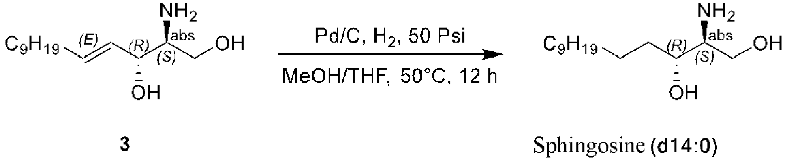

Under nitrogen protection, add wet palladium on carbon (327.94 mg, 308.15 µmol, 10% purity) to a solution of (E,2R,3R)-2-aminotetradecyl-4-ene-1,3-diol (150 mg, 616.31 µmol) in methanol (2 mL) and tetrahydrofuran (2 mL). Degas the mixture, and replace with hydrogen three times. Stir the mixture under hydrogen (50 psi) atmosphere at 50°C for 12 h. Add to the mixture tetrahydrofuran (10 mL), and filter. Wash the filter cake three times with tetrahydrofuran (10 mL). Purify the crude product after concentration of the filtrate by prep-HPLC (chromatographic column: Phenomenex Luna C8 50*40 mm*5 µm; mobile phase: [water(formic acid)-methanol]; gradient: 45%-95% B in 20 min) to obtain (2S,3R)-2-aminotetradecane-1,3-diol (35.8 mg, 145.88 µmol, yield 23.67%), which is a white solid.
**LCMS** Rt = 5.995 min in 7 min Chromatography, 10-80AB ESI calculated value C₁₄H₃₂NO₂ [M+H]⁺ 246.4, and measured value 246.2
**¹H NMR** (400 MHz, CDCl₃) δ = 5.50 - 5.40 (m, 1H), 4.05 - 3.95 (m, 1H), 3.87 - 3.70 (m, 2H), 3.60 - 3.45 (m, 1 H), 2.66 - 2.39 (m, 2H), 1.75 - 1.40 (m, 16H), 1.39 - 1.30 (m, 4H), 0.90 (t, *J* = 6.4 Hz, 3H).

### Sphingosine (d15:0)

### Synthesis route map:

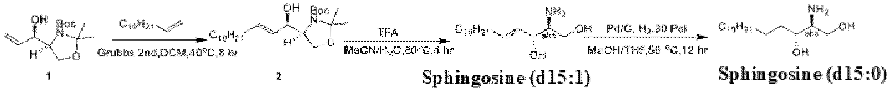

### Synthesis route:

### Step 1

### Tert-butyl (4S)-4-[(E,1R)-1-hydroxytridecyl-2-enyl]-2,2-dimethyl-oxazolidine-3-carboxylate

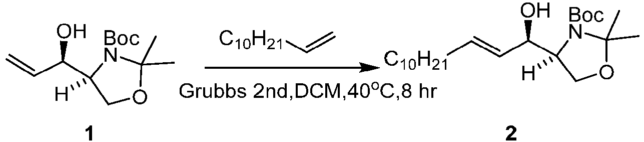

Degas a mixed liquid of tert-butyl (4S)-4-[(1R)-1-hydroxyallyl]-2,2-dimethyl-oxazolidine-3-carboxylate (400 mg, 1.55 mmol), dodec-1-ene (340.14 mg, 2.02 mmol), and benzal-[1,3-bis(2,4,6-trimethylphenyl)imidazolidin-2-ylidene]-dichloro-ruthenium; and tricyclohexylphosphine (131.97 mg, 155.45 µmol) in dichloromethane (6 mL), and replace with nitrogen three times. Stir the mixture at 40°C for 8 h under nitrogen protection. Purify, by silica gel flash column chromatography (eluent: 0 to 14% ethyl acetate in petroleum ether), the crude product after concentration under reduced pressure to obtain tert-butyl (4S)-4-[(E,1R)-1-hydroxytridecyl-2-enyl]-2,2-dimethyl-oxazolidine-3-carboxylate (380 mg, 955.76 µmol, yield 61.49%), which is a yellow oily matter.

**¹H NMR** (400 MHz, CDCl₃) δ = 5.83 - 5.57 (m, 1H), 5.52 - 5.32 (m, 1H), 4.24 - 3.74 (m, 4H), 2.10 - 1.90 (m, 2H), 1.53- 1.43 (m, 15H), 1.40 - 1.14 (m, 16H), 0.90 - 0.84 (m, 3H).

### Step 2

### (E,2S,3R)-2-aminopentadecyl-4-ene-1,3-diol

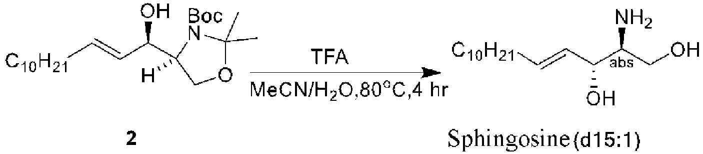

Add an aqueous solution (2 mL) of trifluoroacetic acid (544.89 mg, 4.78 mmol, 354.98 µL) to an acetonitrile solution (2 mL) of (4S)-4-[(E,1R)-1-hydroxytridecyl-2-enyl]-2,2-dimethyl-oxazolidine-3-carboxylate (380 mg, 955.76 µmol). Stir the mixture at 80°C for 4 h. Add a saturated aqueous solution of sodium bicarbonate to adjust the pH value to 8. Filter the mixture, wash the filter cake with water (10 mL) three times, and dry under reduced pressure to obtain (E,2S,3R)-2-aminopentadecyl-4-ene-1,3-diol (100 mg, 388.48 µmol, yield 40.65%), which is a brown solid. The solid is used directly in the next step with no need for further purification.

**¹H NMR** (400 MHz, CDCl₃) δ = 5.82 - 5.69 (m, 1H), 5.51 - 5.41 (m, 1H), 4.85 - 4.41 (m, 2H), 4.13 - 3.95 (m, 1H), 3.77 - 3.55 (m, 2H), 2.97 - 2.77 (m, 1H), 2.12 - 2.00 (m, 2H), 1.40 - 1.35 (m, 2H), 1.28 - 1.24 (m, 14H), 0.88 (t, *J =* 6.8 Hz, 3H).

### Step 3

### (2S,3R)-2-aminopentadecane-1,3-diol

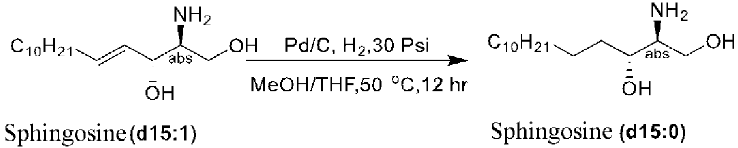

Under nitrogen protection, add wet palladium on carbon (206.71 mg, 194.24 µmol, 10% purity) to a solution of (E,2S,3R)-2-aminopentadecyl-4-ene-1,3-diol (100 mg, 388.48 µmol) in methanol (2 mL) and tetrahydrofuran (2 mL). Degas the suspension, and replace with hydrogen three times. Stir the mixture under hydrogen atmosphere (30 psi) at 50°C for 12 h. Added tetrahydrofuran (10 mL) into the mixture, and filter. Wash the filter cake three times with tetrahydrofuran (10 mL). Purify the crude product after concentration of the filtrate by prep-HPLC (chromatographic column: Phenomenex Luna C8 50*40 mm*5 µm; mobile phase: [water(formic acid)-methanol]; gradient: 10% to 40% B in 20 min) to obtain (2S,3R)-2-aminopentadecane-1,3-diol (10.6 mg, 34.70 µmol, yield 8.93%, formate), which is a white solid.

**¹H NMR** (400 MHz, CD₃OD) δ = 8.54 (br s, 1H), 3.85 - 3.65 (m, 3H), 3.20 - 3.11 (m, 1H), 1.56 - 1.46 (m, 2H), 1.41 - 1.26 (m, 20H), 0.90 (t, *J* = 6.8 Hz, 3H).

**LCMS** Rt = 0.830 min in 1.5 min Chromatography, 5-95AB_E ESI calculated value C₁₅H₃₄NO₂ [M+H]⁺ 260.3, and measured value 260.1

**HPLC** Rt = 0.790 min, in 8 min chromatography, ELSD, purity 98.862%.

### Sphingosine (d19:1) & (d19:0)

### Synthesis route map:

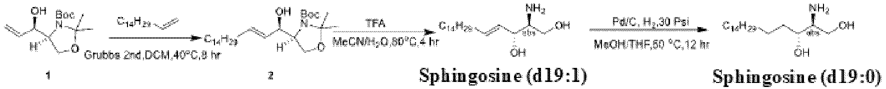

### Synthesis route:

### Step 1

### Tert-butyl (4S)-4-[(E,1R)-1-hydroxyheptadecyl-2-enyl]-2,2-dimethyl-oxazolidine-3-carboxylate

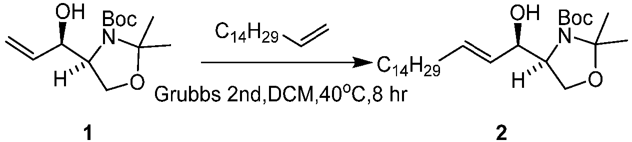

Degas a mixed liquid of tert-butyl (4S)-4-[(1R)-1-hydroxyallyl]-2,2-dimethyl-oxazolidine-3-carboxylate (400 mg, 1.55 mmol), hexadec-1-ene (453.51 mg, 2.02 mmol, 580.68 µL), and benzal-[1,3-bis(2,4,6-trimethylphenyl)imidazolidin-2-ylidene]-dichloro-ruthenium; and tricyclohexylphosphine (131.97 mg, 155.45 µmol) in dichloromethane (6 mL), and replace with nitrogen three times. Stir the mixture at 40°C for 8 h under nitrogen protection. Purify, by silica gel flash column chromatography (eluent: 0 to 14% ethyl acetate in petroleum ether), the crude product after concentration under reduced pressure to obtain tert-butyl (4S)-4-[(E,1R)-1-hydroxyheptadecyl-2-enyl]-2,2-dimethyl-oxazolidine-3-carboxylate (300 mg, 661.24 µmol, yield 42.54%), which is a yellow oily matter.

**¹H NMR** (400 MHz, CDCl₃) δ = 5.76 - 5.67 (m, 1H), 5.58 - 5.36 (m, 1H), 4.13 - 3.79 (m, 4H), 2.04 - 1.96 (m, 2H), 1.70 - 1.30 (m, 23H), 1.27 - 1.24 (m, 16H), 0.93 - 0.83 (m, 3H).

### Step 2

### (E,2S,3R)-2-aminononadecyl-4-ene-1,3-diol

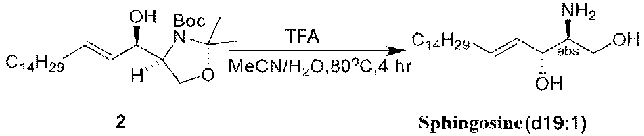

Add trifluoroacetic acid (753.94 mg, 6.61 mmol, 491.17 µL) to a mixed solution of tert-butyl (4S)-4-[(E,1R)-1-hydroxyheptadecan-2-enyl]-2,2-dimethyl-oxazolidine-3-carboxylate (300 mg, 661.24 µmol) in acetonitrile (2 mL) and water (2 mL). Stir the mixture at 80°C for 4 h. Concentrate under reduced pressure to obtain the crude product (200 mg, 637.92 µmol, yield 96.47%), which is a yellow oily matter. Purify (100 mg, 318.96 µmol) thereof by prep-HPLC (chromatographic column: Phenomenex Luna C8 50*40 mm*5 µm; mobile phase: [water (formic acid)-methanol]; gradient: 50% to 80% B in 20 min) to obtain (E,2S,3R)-2-aminononadecyl-4-ene-1,3-diol (10.9 mg, 25.49 µmol, yield 7.99%, trifluoroacetate), which is a white solid.
**¹H NMR** (400 MHz, CD₃OD) δ = 8.53 (s, 0.5H), 5.96 - 5.76 (m, 1H), 5.53 - 5.40 (m, 1H), 4.26 (t, *J =* 6.0 Hz, 1H), 3.82 - 3.75 (m, 1H), 3.70 - 3.62 (m, 1H), 3.20 - 3.14 (m, 2H), 2.15 - 2.05 (m, 2H), 1.47 - 1.40 (m, 2H), 1.29 (s, 22H), 0.90 (t, *J* = 6.8 Hz, 3H).
**¹⁹F NMR** (376.5 MHz, CD₃OD) δ = -76.926.
**LCMS** Rt = 1.764 min in 7 min Chromatography, 50-100AB ESI calculated value C₁₉H₃₉NO₂ [M+H]⁺ 314.3, and measured value 314.3
**HPLC** Rt = 2.253 min, in 8 min chromatography, ELSD, purity 97.530%.

### Step 3

### (2S,3R)-2-aminononadecane-1,3-diol

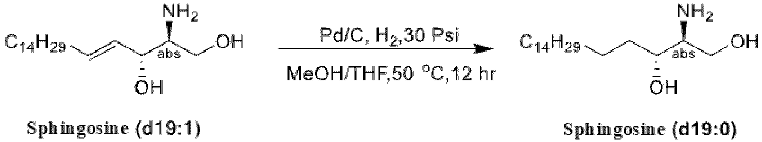

Under nitrogen protection, add wet palladium on carbon (169.72 mg, 159.48 µmol, 10% purity) to a solution of (E,2S,3R)-2-aminononadecyl-4-ene-1,3-diol (100 mg, 318.96 µmol) in methanol (2 mL) and tetrahydrofuran (2 mL). Degas the suspension, and replace with hydrogen three times. Stir the mixture under hydrogen atmosphere (30 psi) at 50°C for 12 h. Add tetrahydrofuran (10 mL) to the mixture, and filter. Wash the filter cake three times with tetrahydrofuran (10 mL). Purify the crude product after concentration of the filtrate by prep-HPLC (chromatographic column: Phenomenex Luna C8 50*40 mm*5 µm; mobile phase: [water(formic acid)-methanol]; gradient: 20% to 50% B in 20 min) to obtain (2S,3R)-2-aminononadecane-1,3-diol (25.7 mg, 71.08 µmol, yield 22.43%, formate), which is a white solid.
**¹H NMR** (400 MHz, CD₃OD) δ = 8.54 (br s, 1H), 3.84 - 3.65 (m, 3H), 3.23 - 3.09 (m, 1H), 1.55 - 1.45 (m, 2H), 1.35 - 1.26 (m, 28H), 0.90 (t, *J* = 6.8 Hz, 3H).
LCMS Rt = 0.962 min in 1.5 min Chromatography, 5-95AB ESI calculated value C₁₉H₄₂NO₂ [M+H]⁺ 316.3, and measured value 316.2
**HPLC** Rt = 2.105 min, in 8 min chromatography, ELSD, purity 99.824%.

### Sphingosine (d21:1) & (d21:0)

### Synthesis route map

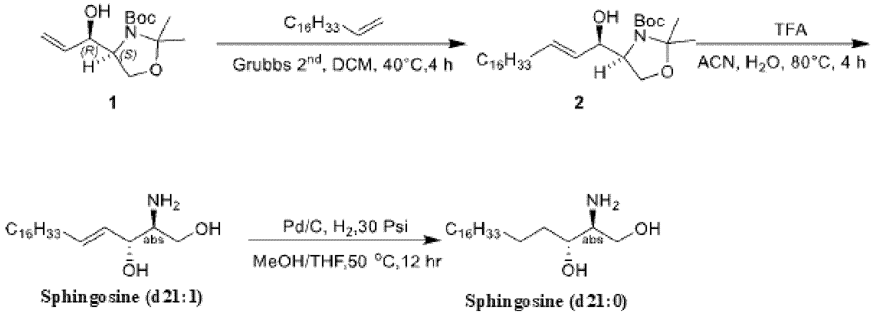

### Synthesis route:

### Step 1

### (4S)-4-[(E,1R)-1-hydroxynonadecyl-2-enyl]-2,2-dimethyl-oxazolidine-3-carboxylate

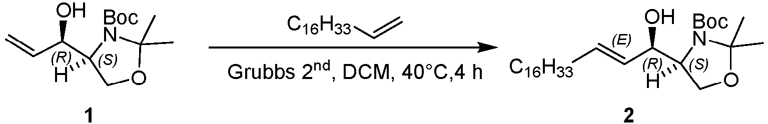

Degas a mixed liquid of tert-butyl (4S)-4-[(1R)-1-hydroxyallyl]-2,2-dimethyl-oxazolidine-3-carboxylate (800 mg, 3.11 mmo), octadec-1-ene (1.02 g, 4.04 mmol), and benzal-[1,3-bis(2,4,6-trimethylphenyl)imidazolidin-2-ylidene]-dichloro-ruthenium; and tricyclohexylphosphine (263.94 mg, 310.89 µmol) in dichloromethane (12 mL), and replace with nitrogen three times. Stir the mixture at 40°C for 4 h under nitrogen protection. Filter the reaction solution, and purify, by silica gel flash column chromatography (eluent: 20% ethyl acetate in petroleum ether), the crude product after concentration of the filtrate under reduced pressure to obtain (4S)-4-[(E,1R)-1-hydroxynonadecyl-2-enyl]-2,2-dimethyl-oxazolidine-3-carboxylate (600 mg, 1.25 mmol, yield 40.06%), which is a brown oily matter.

**¹H NMR** (400 MHz, CDCl₃) δ = 5.75 - 5.61 (m, 1H), 5.45 - 5.30 (m, 1H), 4.20 - 3.50 (m, 4H), 1.97 - 1.81 (m, 2H), 1.42 (s, 15H), 1.18 (s, 28H), 0.81 (t, *J* = 6.4 Hz, 3H).

### Step 2

### (E,2S,3R)-2-aminohenicosane-4-ene-1,3-diol

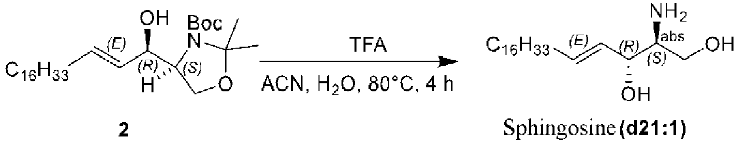

Add an aqueous solution (4 mL) of trifluoroacetic acid (1.42 g, 12.45 mmol, 925.13 µL) to an acetonitrile solution (4 mL) of (4S)-4-[(E,1R)-1-hydroxynonadec-2-enyl]-2,2-dimethyl-oxazolidine-3-carboxylate (600 mg, 1.25 mmol). Stir the mixture at 80°C for 4 h. Purify the reaction solution by prep-HPLC (chromatographic column: Phenomenex Luna C8 50*40 mm*5 µm; mobile phase: [water (formic acid)-methanol]; gradient: 27% to 57% B in 20 min), and freeze dry to obtain (E,2S,3R)-2-aminohenicosane-4-ene-1,3-diol (15.6 mg, 34.24 µmol, yield 2.75%, trifluoroacetate), which is a white solid.
**LCMS** Rt = 3.223 min in 4 min Chromatography, 10-80AB ESI calculated value C₂₁H₄₄NO₂ [M+H]⁺ 342.3, and measured value 342.3
**¹H NMR** (400 MHz, CD₃OD) δ = 5.95 - 5.80 (m, 1H), 5.61 - 5.40 (m, 1H), 4.32 - 4.20 (m, 1H), 3.86 - 3.75 (m, 1H), 3.70 - 3.61 (m, 1H), 3.20 - 3.10 (m, 1H), 2.21 - 2.05 (m, 2 H), 1.31 (s, 28H), 0.96 - 0.75 (m, 3H).

### Step 3

### (2S,3R)-2-aminohenicosane-1,3-diol

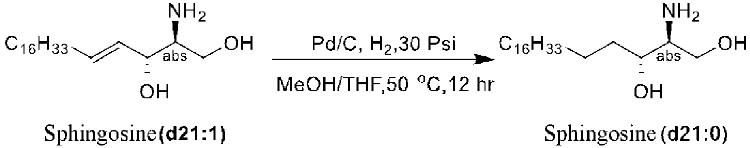

Under nitrogen protection, add wet palladium on carbon (155.78 mg, 146.38 µmol, 10% purity) to a solution of (E,2S,3R)-2-aminohenicosane-4-ene-1,3-diol (100 mg, 292.77 µmol) in methanol (2 mL) and tetrahydrofuran (2 mL). Degas the suspension, and replace with hydrogen three times. Stir the mixture under hydrogen atmosphere (30 psi) at 50°C for 12 h. Add tetrahydrofuran (10 mL) to the mixture, and filter. Wash the filter cake three times with tetrahydrofuran (10 mL). Purify the crude product after concentration of the filtrate by prep-HPLC (chromatographic column: Phenomenex Luna C8 50*40 mm*5 µm; mobile phase: [water(formic acid)-methanol]; gradient: 50% to 80% B in 20 min) to obtain (2S,3R)-2-aminohenicosane-1,3-diol (13.0 mg, 33.37 µmol, yield 11.39%, formate), which is a white solid.
**¹H NMR** (400 MHz, CD₃OD) δ = 8.54 (br s, 1H), 3.85 - 3.79 (m, 1H), 3.77 - 3.64 (m, 2H), 3.18 - 3.05 (m, 1H), 1.51 - 1.48 (m, 2H), 1.38 - 1.25 (m, 32H), 0.90 (t, *J=* 6.4 Hz, 3H).
**LCMS** Rt = 3.082 min in 7 min Chromatography, 50-100AB ESI calculated value C₂₁H₄₆NO₂ [M+H]⁺ 344.4, and measured value 344.3
**HPLC** Rt = 3.162 min, in 8 min chromatography, ELSD, purity 96.003%.

### Sphingosine (d22:0)

### Synthesis route map

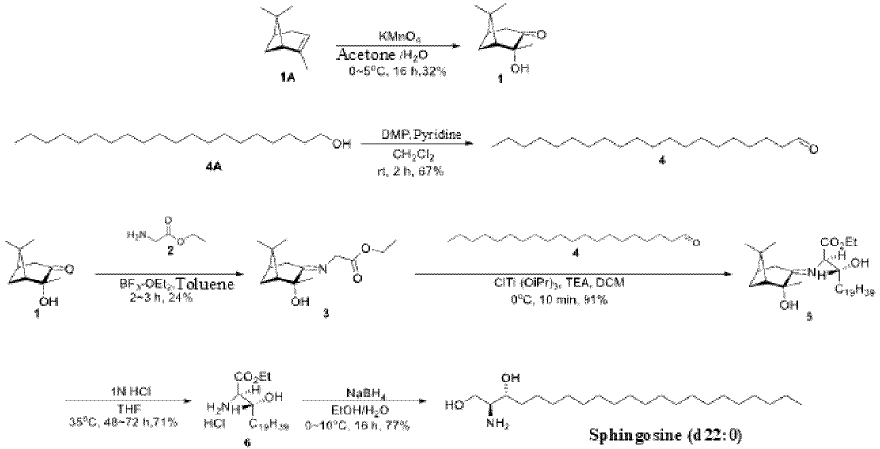

### Synthesis route:

### Step 1

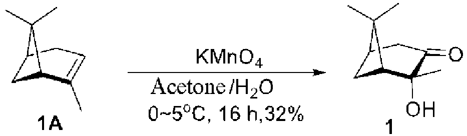

Dissolve *α*-pinene (**1A**, 3.0 kg, 22.4 mol, 1.0 eq) in acetone (30 L) and water (3 L), add potassium permanganate (6.0 kg, 38.5 mol, 1.7 eq) in batches within 2 h at a temperature in a range of 0-5 °C. After the addition is completed, stir at 0-5°C overnight, and the reaction solution turns dark black. GC shows that the starting material has been completely consumed, filter the reaction solution to remove manganese dioxide, and concentrate the filtrate to obtain a crude product. Dissolve the crude product in ethyl acetate (5 L), filter again to remove insoluble matters, wash the filtrate with water (5 L) and saturated sodium bicarbonate (5 L), dry over anhydrous sodium sulfate, filter and concentrate to obtain a crude oily matter (1.7 kg), and after distillation (collecting 3-4 mmHg, 100-104°C fractions) to obtain compound 1 (1.2 kg, yield 32%) with a GC purity >97%.

### Step 2

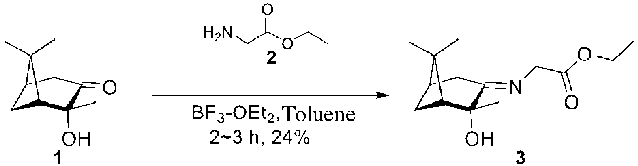

Compound**1**(1.0 kg, 5.9 mol, 1.0 eq) was dissolved in toluene (10 L), and glycine ethyl ester hydrochloride (**2**, 1.7 kg, 11.9 mol, 2.0 eq), triethylamine (1.3 kg, 13.1 mol, 2.2 eq) and boron trifluoride etherate (8.9 g, 59.5 mmol, 0.01 eq) were added. Under argon protection, heat to reflux, and react for 2 to 3 h. LC-MS shows that about 5% of compound **1** remains. Cool the reaction solution to room temperature, filter, rinse the filter cake with ethyl acetate (5 L), and concentrate the filtrate to obtain a crude product that is an oily matter. Purify the crude product by basic silica gel column chromatography (petroleum ether/ethyl acetate = 15/1-ethyl acetate) to obtain a yellow oily compound **3** (900 g), and crystallize at low temperature from n-hexane (2.7 L) to obtain off-white solid compound **3** (360 g, yield 24%).

### Step 3

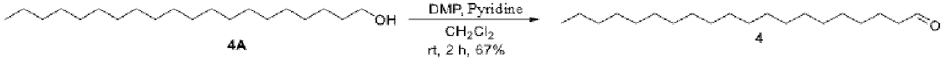

Dissolve 1-Eicosanol (**4A**, 1.0 kg, 3.4 mol, 1.0 eq) and pyridine (0.8 kg, 10.1 mol, 3.0 eq) in dichloromethane (15 L), cool to 10°C, and add Dess-Martin reagent (1.7 kg, 4.0 mol, 1.2 eq). React at room temperature for 2 h. TLC shows that compound **4A** has been completely consumed. Quench the reaction solution with saturated sodium sulfite (10 L), and extract the aqueous phas with dichloromethane (5 L). Combine the organic phases, dry over anhydrous sodium sulfate, filter, and concentrat to obtain a crude product, which is an oily matter. Purify the crude product by silica gel column chromatography (petroleum ether/ethyl acetate = 200/1 to 80/1) to obtain an off-white solid compound **4** (670 g, yield 67%).
**TLC**: PE/EA = 20/1 (2,4-dinitrophenylhydrazine)
**R_{f}** (Compound **4A**) = 0.3
**R_{f}** (Compound **4**) = 0.8

### Step 4

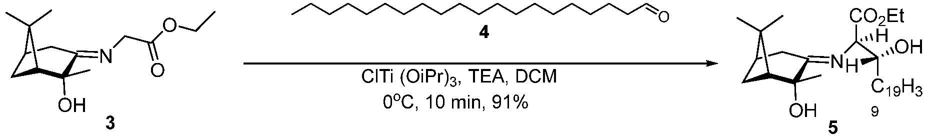

Dissolve compound **3** (0.98 kg, 3.9 mol, 1.0 eq) in dichloromethane (4 L). Under argon protection at 0°C, add a dichloromethane solution (4 L) of triisopropoxytitanium chloride (1.4 kg, 5.3 mol, 2.5 eq), triethylamine (1.2 L, 3.0 eq), and a dichloromethane (4 L) of compound **4** (0.69 kg, 2.3 mol, 1.1 eq) in sequence. After reacting for 10 min, TLC shows that compound **3** has been completely consumed, and add saturated brine (3 L) to quench the reaction. Filter the reaction solution to remove the titanium salt, rinse the filter cake with dichloromethane (2 L), and extract the aqueous phase with dichloromethane (2 L). Combine the organic phases, dry over anhydrous sodium sulfate, filter, and concentrat to obtain a crude product. Dissolve the crude product in ethyl acetate (2 L), filter to remove the insoluble matter, and concentrate to obtain an orange oily compound **5** (2.1 kg, yield 91%), which is a mixture of ethyl ester and isopropyl ester.
**TLC:** PE/EA = 3/1 (I₂)
**R_{f}** (Compound **3**) = 0.4
**R_{f}** (Compound **5**) = 0.6

### Step 5

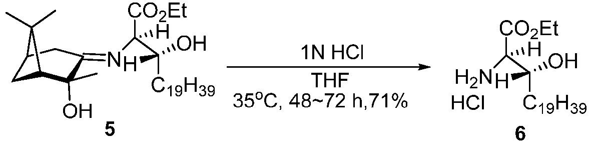

Dissolve compound **5** (3 kg, 5.5 mol) in tetrahydrofuran (12 L), add 1.0 M dilute hydrochloric acid (32 L) that has been prepared, and stir at 35°C for 48-72 h. TLC shows that compound **5** has been completely consumed, and directly concentrate the reaction solution to obtain a crude product that is a solid. Subject the crude product to slurrying with ethanol/ethyl acetate = 2/5 (14 L), and then dry to obtain a light yellow solid compound **6** (1.7 kg, yield 71%).
**TLC**: PE/EA = 3/1
**Rf** (Compound **5**) = 0.6
**Rf** (Compound **6**) = 0.1

### Step 6

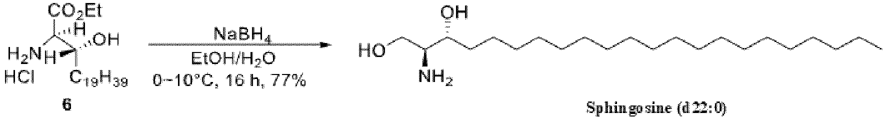

Disperse compound 6 (0.9 kg, 2.5 mol, 1.0 eq) in deionized water (5.4 L) and ethanol (16.2 L), cool to 0 to 10°C, and sadd odium borohydride (0.6 kg, 20.1 mol, 8.0 eq). React overnight at room temperature. LCMS shows that no starting material is present. Add saturated ammonium chloride (5 L) to quench the reaction, and stir for 30 min.

Combine the two batches of reaction solutions, and process as follows:
after the combination, filter the reaction solution, extract the aqueous phase with chloroform (10 L), and combine and concentrate the organic phases to obtain an off-white solid. after combining the solid with the filter cake, dissolve in deionized water (20 L) and methanol (5 L) at 100°C, and stir for 1 h. Cool naturally to room temperature, stir overnight, filter, and dry the filter cake to obtain a light yellow solid (1.8 kg). Dissolve the solid in methanol (30 L) under reflux, cool to room temperature, filter, and dry to obtain a light yellow solid (1.5 kg). Dissolve the solid in chloroform/methanol = 8/1 (100 L), remove the insoluble matter through a filtration membrane, and then concentrate the filtrate to obtain sphingosine (d22:0) (1.2 kg, yield 77%), which is an off-white solid.
**TLC**: PE/EA = 1/1 (I₂)
**Rf** (Compound **6**) = 0.4
**Rf** (sphingosine (d22:0)) = 0.1
**LC-MS**: 358.23 [M+1]+
**¹H NMR** (400 MHz, methanol-d4) δ 3.73 (dd, J = 8.0, 4.0 Hz, 1H), 3.52-3.45 (m, 2H), 2.77-2.74 (m, 1H), 1.51 (s, 2H), 1.27 (s, 34H), 0.88 (t, J = 6.8 Hz, 3H).

### Sphingosine (d23:1) & (d23:0)

### Synthesis route map

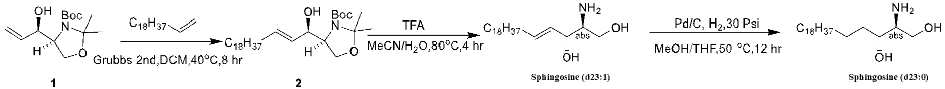

### Synthesis route:

### Step 1

### Tert-butyl (4S)-4-[(E,1R)-1-hydroxyhenicosane-2-enyl]-2,2-dimethyl-oxazolidine-3-carboxylate

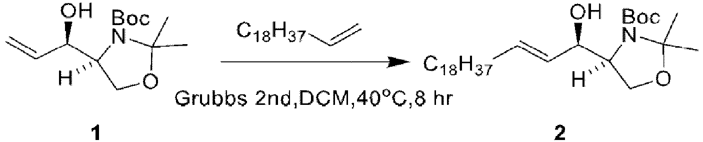

Degas a mixed liquid of tert-butyl (4S)-4-[(1R)-1-hydroxyallyl]-2,2-dimethyl-oxazolidine-3-carboxylate (400 mg, 1.55 mmol), eicos-1-ene (566.89 mg, 2.02 mmol), and benzal-[1,3-bis(2,4,6-trimethylphenyl)imidazolidin-2-ylidene]-dichloro-ruthenium; and tricyclohexylphosphine (131.97 mg, 155.45 µmol) in dichloromethane (6 mL), and replace with nitrogen three times. Stir the mixture at 40°C for 8 h under nitrogen protection. Purify, by silica gel flash column chromatography (eluent: 0 to 14% ethyl acetate in petroleum ether), the crude product after concentration of the reaction solution under reduced pressure to obtain tert-butyl (4S)-4-[(E,1R)-1-hydroxyhenicosane-2-enyl]-2,2-dimethyl-oxazolidine-3-carboxylate (280 mg, 549.23 µmol, yield 35.33%), which is a yellow oily matter.

**¹H NMR** (400 MHz, CDCl₃) δ = 5.78 - 5.69 (m, 1H), 5.48 - 5.36 (m, 1H), 4.16 - 3.80 (m, 4H), 2.08 - 2.00 (m, 2H), 1.76 - 1.25 (m, 31H), 1.25 - 1.22 (m, 15H), 0.92 - 0.84 (m, 3H).

### Step 2

### (E,2S,3R)-2-aminotricosane-4-ene-1,3-diol

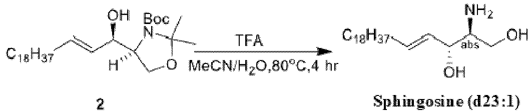

Add an aqueous solution (2 mL) of trifluoroacetic acid (626.24 mg, 5.49 mmol, 407.97 µL) to an acetonitrile solution (2 mL) of (4S)-4-[(E,1R)-1-hydroxyhenicosane-2-enyl]-2,2-dimethyl-oxazolidine-3-carboxylate (280 mg, 549.23 µmol). Stir the mixture at 80°C for 4 h. Purify the crude product obtained after concentration of the reaction solution under reduced pressure by prep-HPLC (chromatographic column: Phenomenex Luna C8 50*40 mm*5 µm; mobile phase: [water (formic acid)-methanol]; gradient: 50% to 85% B in 20 min), and freeze dry to obtain (E,2S,3R)-2-aminotricosane-4-ene-1,3-diol (4 mg, 8.27 µmol, yield 0.15%, trifluoroacetate), which is a white solid.

**¹H NMR** (400 MHz, CD₃OD) δ = 8.54 (br s, 1H), 5.90 - 5.79 (m, 1H), 5.53 - 5.43 (m, 1H), 4.27 (t, *J* = 5.6 Hz, 1H), 3.82 - 3.75 (m, 1H), 3.69 - 3.61 (m, 1H), 3.23 - 3.14 (m, 1H), 2.10 (q, *J* = 7.2 Hz, 2H), 1.46 - 1.40 (m, 2H), 1.29 (s, 30H), 0.90 (t, *J* = 6.8 Hz, 3H).

**¹⁹F NMR** (376.5 MHz, CD₃OD) δ = -76.926.

**LCMS** Rt = 1.107 min in 1.5 min Chromatography, 5-95AB ESI calculated value C₂₃H₄₈NO₂ [M+H]⁺ 370.4, and measured value 370.3

**HPLC** Rt = 3.661 min, in 8 min chromatography, ELSD, purity 100.000%.

### Step 3

### (2S,3R)-2-aminotricosane-1,3-diol

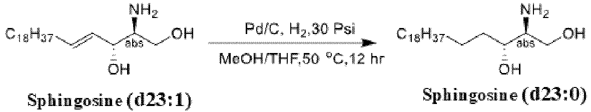

Under nitrogen protection, add wet palladium on carbon (143.96 mg, 135.27 µmol, 10% purity) to a solution of (E,2S,3R)-2-aminotricosane-4-ene-1,3-diol (100 mg, 270.55 µmol) in methanol (2 mL) and tetrahydrofuran (2 mL). Degas the suspension, and replace with hydrogen three times. Stir the mixture under hydrogen atmosphere (30 psi) at 50°C for 12 h. Add tetrahydrofuran (10 mL) to the mixture, and filter. Wash the filter cake three times with tetrahydrofuran (10 mL). Purify the crude product after concentration of the filtrate by prep-HPLC (chromatographic column: Phenomenex Luna C8 50*40 mm*5 µm; mobile phase: [water(formic acid)-methanol]; gradient: 50% to 85% B in 20 min) to obtain (2S,3R)-2-aminotricosane-1,3-diol (5.4 mg, 12.93 µmol, yield 4.80%, formate), which is a white solid.
**¹H NMR** (400 MHz, CD₃OD) δ = 8.55 (s, 1H), 3.82 - 3.75 (m, 1H), 3.65 - 3.55 (m, 2H), 3.01 - 2.89 (m, 1H), 1.56 - 1.50 (m, 2H), 1.33 - 1.28 (m, 36H), 0.90 (t, *J* = 6.4 Hz, 3H).
**LCMS** Rt = 1.118 min in 1.5 min Chromatography, 5-95AB ESI calculated value C₂₃H₅₀NO₂ [M+H]⁺ 372.4, and measured value 372.2
**HPLC** Rt = 3.820 min, in 8 min chromatography, ELSD, purity 94.809%.

### Sphingosine (d24:1) & (d24:0)

### Synthesis route map

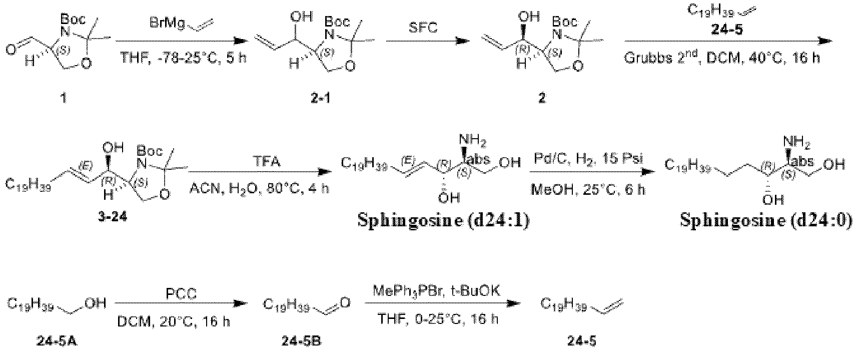

### Synthesis route:

### Step 1

### Tert-Butyl (4S)-4-(1-hydroxyallyl)-2,2-dimethyloxazolidine-3-carboxylate

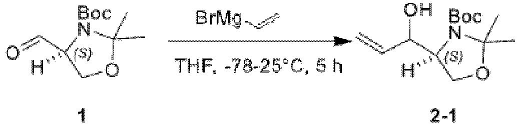

Add vinylmagnesium bromide (1 M, 49.07 mL, 1.5 eq) dropwise to a THF solution (75 mL) of tert-butyl (S)-4-formyl-2,2-dimethyloxazolidine-3-carboxylate (7.5 g, 32.71 mmol, 1 eq) at -78°C over half an hour. Stir the mixture at -78°C for 2 h, then heat to 25°C, and continue to stir for another 5 h. Quench the reaction mixture with a saturated aqueous ammonium chloride solution (50 mL), and extract the aqueous phase with ethyl acetate (50 mL×3). Wash the combined organic phases with saturated brine (15 mL), dry over sodium sulfate, filter, and concentrate. Purify the crude product by silica gel flash column chromatography (eluent: 0 to 25% ethyl acetate in petroleum ether) to obtain tert-butyl (4S)-4-(1-hydroxyallyl)-2,2-dimethyloxazolidine-3-carboxylate (7 g, 27.20 mmol, yield 83.1%), which is colorless and oily.

**¹H NMR** (400 MHz, CDCl₃) δ = 5.92 - 5.80 (m, 1H), 5.43 - 5.34 (m, 1H), 5.26 - 5.19 (m, 1H), 4.31 - 4.20 (m, 1H), 4.08 - 3.82 (m, 3H), 1.56 - 1.47 (m, 15H).

### Step 2

### Tert-Butyl (S)-4-((R)-1-hydroxyallyl)-2,2-dimethyloxazolidine-3-carboxylate

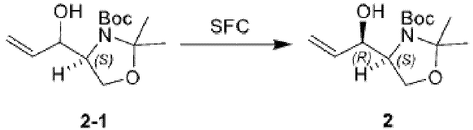

Purify tert-butyl (4S)-4-(1-hydroxyallyl)-2,2-dimethyloxazolidine-3-carboxylate (5 g) by SFC (column: DAICEL CHIRALCEL OX (250 mm x 30 mm, 10 µm); mobile phase: [CO₂-EtOH (0.1% NH₃H₂O)]; B%: 10%, isocratic elution mode) to obtain tert-butyl (S)-4-((R)-1-hydroxyallyl)-2,2-dimethyloxazolidine-3-carboxylate (2.1 g, 8.16 mmol, yield 42.0%, ee 100%), which is colorless and oily.

**¹H NMR** (400 MHz, CDCl₃) δ = 5.92 - 5.78 (m, 1H), 5.43 - 5.33 (d, *J =* 17.2 Hz, 1H), 5.26 - 5.17 (d, *J=* 10.4 Hz, 1H), 4.33 - 3.86 (m, 4H), 1.58 - 1.45 (m, 15H).

**SFC** Rt = 0.540 min in 3 min. Column: Chiral Column OX-3 50 x 4.6 mm I.D., 3 um Mobile Phase: Phase A (CO₂), Phase B: (EtOH (0.05% DEA)); Elution Gradient: B in A from 5% to 40% Flow Rate: 3 mL/min; Detector: PDA; Column Temperature: 35°C; Back Pressure: 100 Bar".

### Step 3

### Eicosanal

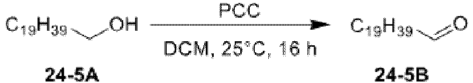

Add pyridinium chlorochromate (PCC) (6.75 g, 31.32 mmol, 1.1 eq) to a DCM solution (400 mL) of eicosan-1-ol (8.5 g, 28.47 mmol, 1 eq). Stir the mixture at 25°C for 16 h. Add silica gel (10 g) to the brown suspension. Stir the resulting mixture for 30 min, and purify the concentrated crude product by silica gel flash column chromatography (eluent: 0 to 5% ethyl acetate in petroleum ether) to obtain eicosanal (6.5 g, 21.04 mmol, yield 73.9%), which is a white solid.

**¹H NMR** (400 MHz, CDCl₃) δ = 9.77 (t, *J =* 2.0 Hz, 1H), 2.46 - 2.39 (m, 2H), 1.66 - 1.60 (m, 2H), 1.31 - 1.25 (m, 32H), 0.91 - 0.86 (t, *J* = 2.8 Hz, 3H).

### Step 4

### Heneicos-1-ene

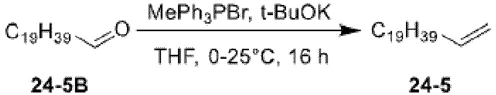

Add potassium tert-butoxide (2.27 g, 20.23 mmol, 3 eq) to a THF solution (40 mL) of methyl(triphenyl)phosphonium bromide (7.23 g, 20.23 mmol, 3 eq) at 0°C. Stir the resulting yellow suspension at 25°C for 1 h, and cool to 0°C again. Add a THF solution (20 mL) of eicosanal (2 g, 6.74 mmol, 1 eq) dropwise at 0°C. Stir the mixture at 25°C for 16 h. Add water (10 mL) to the mixture, and extract with ethyl acetate (15 mL x 3). Wash the combined organic phases with saturated brine (5 mL), dry over sodium sulfate, filter, and concentrate. Purify the crude product by silica gel flash column chromatography (eluent: petroleum ether) to obtain heneicos-1-ene (1.7 g, 5.77 mmol, yield 85.5%), which is a colorless solid.

**¹H NMR** (400 MHz, CDCl₃) δ = 5.89 - 5.76 (m, 1H), 5.04 - 4.90 (m, 2H), 2.08 - 2.01 (m, 2H), 1.40 - 1.36 (m, 2H), 1.30 - 1.24 (m, 32H), 0.91 - 0.87 (t, *J* = 5.2 Hz, 3H).

### Step 5

### Tert-butyl (4S)-4-[(E,1R)-1-hydroxydocosane-2-enyl]-2,2-dimethyl-oxazolidine-3-carboxylate

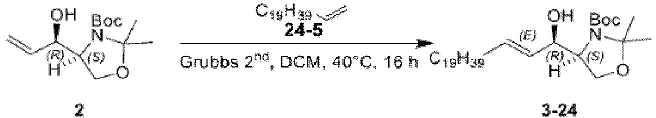

Under nitrogen protection, add benzal-[1,3-bis(2,4,6-trimethylphenyl)imidazolidine-2-methylene]-dichloro-ruthenium; tricyclohexylphosphine (98.98 mg, 116.58 µmol, 0.1 eq) to a mixture of tert-butyl (4S)-4-[(1R)-1-hydroxyallyl]-2,2-dimethyl-oxazolidine-3-carboxylate (300.00 mg, 1.17 mmol, 1 eq) and heneicos-1-ene (446.43 mg, 1.52 mmol, 1.3 eq) in dichloromethane (10 mL). Stir the mixture at 40°C for 16 h. Purify, by silica gel flash column chromatography (eluent: 0 to 25% ethyl acetate in petroleum ether), the crude product obtained after concentration of the reaction solution under reduced pressure to obtain tert-butyl (4S)-4-[(E,1R)-1-hydroxydocosane-2-enyl]-2,2-dimethyl-oxazolidine-3-carboxylate (0.28 g, 534.53 µmol, yield 45.8%), which is a yellow solid.

**¹H NMR** (400 MHz, CDCl₃) δ = 5.81 - 5.69 (m, 1H), 5.50 - 5.40 (m, 1H), 4.22 - 3.83 (m, 4H), 2.09 - 2.01 (m, 2H), 1.56 - 1.48 (m, 15H), 1.31 - 1.22 (m, 34H), 0.91 - 0.86 (t, *J* = 6.4 Hz, 3H).

### Step 6

### (E,2S,3R)-2-aminotetracosane-4-ene-1,3-diol

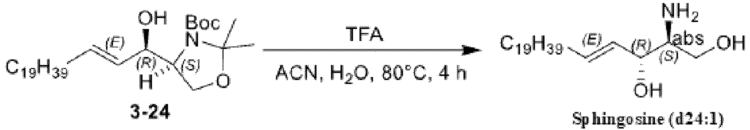

Add an aqueous solution (2 mL) of trifluoroacetic acid (30.70 mg, 269.24 µmol, 0.02 mL, 0.6 eq) to an acetonitrile solution (2 mL) of (4S)-4-[(E,1R)-1-hydroxydocosane-2-enyl]-2,2-dimethyl-oxazolidine-3-carboxylate (230.00 mg, 439.07 µmol, 1 eq). Stir the mixture at 80°C for 4 h. Add a saturated aqueous solution of sodium bicarbonate to the crude product obtained after vacuum concentration to adjust the pH to 8, filter, and dry the filter cake under vacuum to obtain (E,2S,3R)-2-aminotetracosane-4-ene-1,3-diol (120 mg, 307.78 µmol, yield 70.1%, purity 98.4%), which is a white solid.

**LCMS** Rt = 1.786 min in 3 min Chromatography, 30-100AB ESI calculated value C₂₄H₄₉NO₂ [M+1]⁺ 384.6, and measured value 384.4

**¹H NMR** (400 MHz, CDCl₃) δ = 5.85 - 5.63 (m, 1H), 5.57 - 5.43 (m, 1H), 4.24 - 3.93 (t, *J =* 6.0 Hz, 1H), 3.74 - 3.57 (m, 2H), 2.95 - 2.78 (m, 1H), 2.10 - 2.04 (m, 2H), 1.90 - 1.67 (m, 4H), 1.41 - 1.36 (m, 2H), 1.26 (s, 32H), 0.91 - 0.87 (t, *J =* 2.4 Hz, 3H).

### Step 7

### (2S,3R)-2-aminotetracosane-1,3-diol

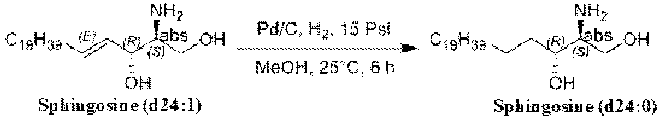

Under nitrogen protection, add wet palladium on carbon (40 mg, 37.59 µmol, 10% purity) to a methanol solution (6 mL) of (E,2S,3R)-2-aminotetracosane-4-ene-1,3-diol (200 mg, 521.31 µmol, 1 eq). Stir the mixture under hydrogen atmosphere at 25°C for 6 h. Filter the mixture. Purify the crude product after concentration of the filtrate by prep-HPLC (chromatographic column: Phenomenex Luna C18 150 x 25mm, 10 µm; mobile phase: [water(formic acid)-methanol]; gradient: 60% to 90% B in 8 min) to obtain (2S,3R)-2-aminotetracosane-1,3-diol (7 mg, 18.13 µmol, yield 3.4%, purity 99.9%), which is a white solid.

**LCMS** Rt = 1.86 min in 3 min Chromatography, 30-100AB ESI calculated value C₂₄H₅₁NO₂ [M+1]⁺ 386.6, and measured value 386.4

**¹H NMR** (400 MHz, CDCl₃) δ = 3.75 - 3.67 (s, 2H), 3.65 - 3.58 (s, 1H), 2.91 - 2.81 (s, 1H), 2.00 (br s, 4H), 1.51 - 1.48 (s, 2H), 1.28 - 1.25 (s, 38H), 0.90 - 0.87 (t, J = 5.6 Hz, 3H).

### Sphingosine (d25:0) & (d25:1)

### Synthesis route map

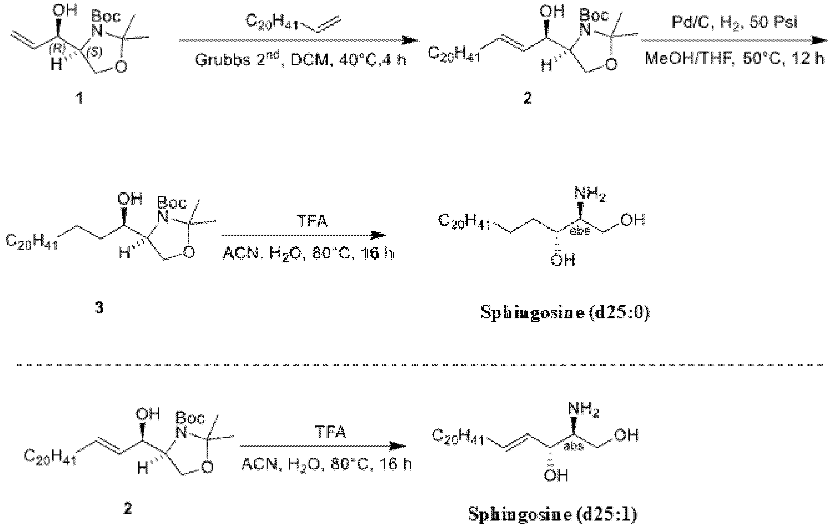

### Synthesis route:

### Step 1

### (4S)-4-[(E,1R)-1-hydroxytricosane-2-enyl]-2,2-dimethyl-oxazolidine-3-carboxylate

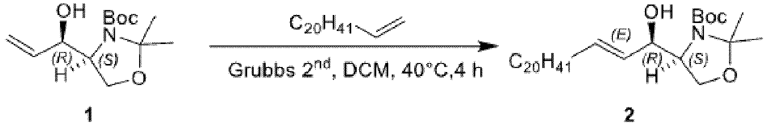

Degas a mixed liquid of tert-butyl (4S)-4-[(1R)-1-hydroxyallyl]-2,2-dimethyl-oxazolidine-3-carboxylate (1 g, 3.89 mmol), docos-1-ene (1.56 g, 5.05 mmol), and benzal-[1,3-bis(2,4,6-trimethylphenyl)imidazolidin-2-ylidene]-dichloro-ruthenium; and tricyclohexylphosphine (329.92 mg, 388.61 µmol) in dichloromethane (15 mL), and replace with nitrogen three times. Stir the mixture at 40°C for 4 h under nitrogen protection. Filter the reaction solution, and purify, by silica gel flash column chromatography (eluent: 20% ethyl acetate in petroleum ether), the crude product after concentration of the filtrate to obtain (4S)-4-[(E,1R)-1-hydroxytricosane-2-enyl]-2,2-dimethyl-oxazolidine-3-carboxylate (910 mg, 1.69 mmol, yield 43.54%), which is a brown oily matter.

**¹H NMR** (400 MHz, CDCl₃) δ = 5.80 - 5.70 (m, 1H), 5.50 - 5.40 (m, 1H), 4.24 - 3.85 (m, 4H), 2.10 - 2.00 (m, 2H), 1.51 (s, 15H), 1.28 (s, 36H), 0.90 (t, *J =* 6.4 Hz, 3H).

### Step 2

### (4S)-4-[(1R)-1-hydroxytricosyl]-2,2-dimethyl-oxazolidine-3-carboxylate

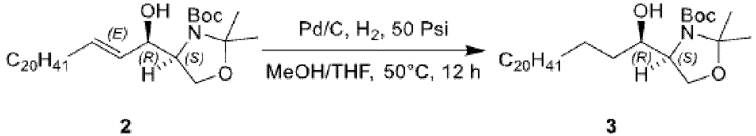

Add Pd/C (722.19 mg, 678.62 µmol, purity 10%) to a mixed solution of (4S)-4-[(E,1R)-1-hydroxytricosane-2-enyl]-2,2-dimethyl-oxazolidine-3-carboxylate (730 mg, 1.36 mmol) in methanol (4 mL) and tetrahydrofuran (4 mL) under nitrogen protection. Degas the suspension, and replace with hydrogen three times. Stir the mixture under hydrogen atmosphere (50 psi) at 50°C for 16 h. Add tetrahydrofuran (10 mL) to the mixture, and filter. Wash the filter cake three times with tetrahydrofuran (10 mL). Purify the crude product obtained by concentration of the filtrate by flash silica gel column chromatography (eluent: 20% ethyl acetate in petroleum ether) to obtain compound (4S)-4-[(1R)-1-hydroxytricosyl]-2,2-dimethyl-oxazolidine-3-carboxylate (560 mg, 1.04 mmol, yield 76.43%), which is a white solid.

**¹H NMR** (400 MHz, CDCl₃) δ = 4.20 - 3.75 (m, 4H), 1.65 - 1.55 (m, 15H), 1.27 (s, 42H), 0.95 - 0.85 (m, 3H).

### Step 3

### (2S,3R)-2-aminopentacosane-1,3-diol

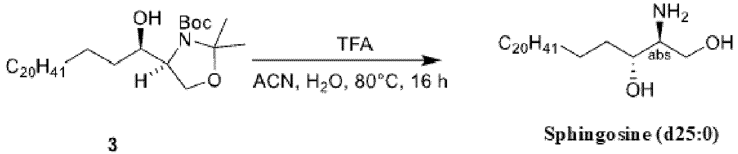

Add an aqueous solution (3 mL) of trifluoroacetic acid (591.36 mg, 5.19 mmol, 385.25 µL) to an acetonitrile solution (3 mL) of (4S)-4-[(1R)-1-hydroxytricosyl]-2,2-dimethyl-oxazolidine-3-carboxylate (560 mg, 1.04 mmol). Stir the mixture at 80°C for 4 h. Add a saturated aqueous solution of sodium bicarbonate to adjust the pH value to 8, and then filter. Stir the filter cake in HCl\dioxane (2 M, 10 mL) at room temperature, and filter. After slurrying with methanol (10 mL), freeze dry to obtain (2S,3R)-2-aminopentacosane-1,3-diol (241 mg, 552.56 µmol, yield 53.27%, hydrochloride), which is a white solid.

**MS** Rt = 1.92 - 2.14 min in 4 min Chromatography, 50-100AB ESI calculated value C₂₅H₅₄NO₂ [M+H]⁺ 400.4, and measured value 400.4

**¹H NMR** (400 MHz, DMSO-*d⁶*) δ = 7.90 - 7.70 (m, 3H), 5.10 - 4.85 (m, 2H), 3.75 - 3.65 (m, 2H), 3.60 - 3.50 (m, 1H), 3.10 - 3.00(m, 1H), 1.50 - 1.35 (m, 2H), 1.27 (s, 40H), 0.88 (t, *J* = 6.8 Hz, 3H).

### Step 4

### (E,2S,3R)-2-aminopentacosane-4-ene-1,3-diol

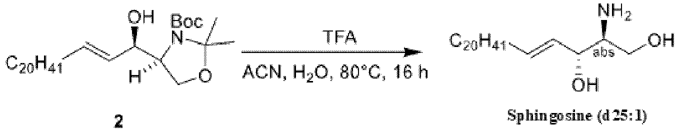

Add an aqueous solution (1 mL) of trifluoroacetic acid (169.59 mg, 1.49 mmol, 110.48 µL) to an acetonitrile solution (1 mL) of (4S)-4-[(E,1R)-1-hydroxytricosane-2-enyl]-2,2-dimethyl-oxazolidine-3-carboxylate (80 mg, 148.74 µmol). Stir the mixture at 80°C for 16 h. Purify the crude product after concentration of the reaction solution by prep-HPLC (chromatographic column: Phenomenex Luna C8 50*40 mm*5 µm; mobile phase: [water(formic acid)-methanol]; gradient: 30% to 60% B in 20 min) to obtain (E,2S,3R)-2-aminopentacosane-4-ene-1,3-diol (9.7 mg, 21.86 µmol, yield 14.70%, formate), which is a white solid.

**LCMS** Rt = 1.140 min in 1.5 min Chromatography, 5-95AB ESI calculated value C₂₅H₅₂NO₂ [M+H]⁺ 398.4, and measured value 398.3.

**¹H NMR** (400 MHz, CD₃OD) δ = 8.57 (s, 1H), 5.94 - 5.80 (m, 1H), 5.55 - 5.45 (m, 1H), 4.25 - 4.21 (m, 1H), 3.81 - 3.75 (m, 1H), 3.72 - 3.61 (m, 1H), 3.16 - 3.05 (m, 1H), 2.23 - 2.15 (m, 2H), 1.51 - 1.41 (m, 2H), 1.31 (s, 34H), 0.92 (t, *J* = 6.8 Hz, 3H).

### Sphingosine (d26:0) & (d26:1)

### Synthesis route map

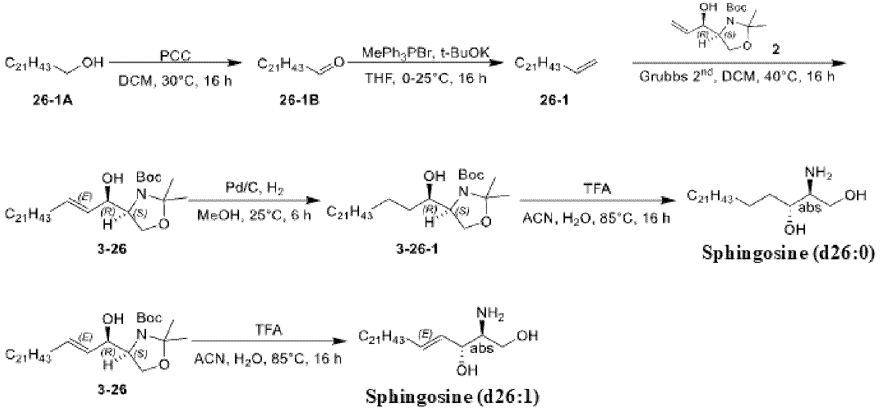

### Synthesis route:

### Step 1

### Docosanal

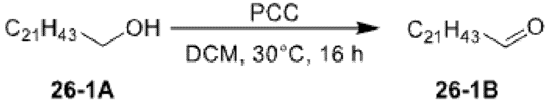

Add pyridinium chlorochromate (PCC) (7.92 g, 36.74 mmol, 1.2 eq) to a DCM solution (400 mL) of docosan-1-ol (10 g, 30.62 mmol, 1 eq). Stir the mixture at 30°C for 16 h. Add silica gel (20 g) to the brown suspension. Stir the resulting mixture for 30 min, and purify the concentrated crude product by silica gel flash column chromatography (eluent: 0 to 10% ethyl acetate in petroleum ether) to obtain docosanal (7.3 g, 22.49 mmol, yield 73.4%), which is a white solid.

**¹H NMR** (400 MHz, CDCl₃) δ = 9.78 - 9.76 (t, J =2.0 Hz, 1H), 2.48 - 2.38 (m, 2H), 1.67 - 1.59 (m, 2H), 1.26 (s, 36H), 0.93 - 0.83 (t, *J* = 6.4 Hz, 3H).

### Step 2

### Tricos-1-ene

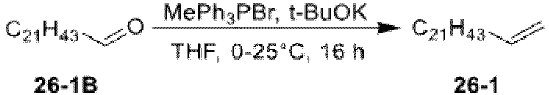

Add potassium tert-butoxide (4.15 g, 36.97 mmol, 3 eq) to a THF solution (40 mL) of methyl(triphenyl)phosphonium bromide (13.21 g, 36.97 mmol, 3 eq) at 0°C. Stir the resulting yellow suspension at 25°C for 1 h, and cool to 0°C again. Add dropwise a THF solution (40 mL) of docosanal (4 g, 12.32 mmol, 1 eq) at 0°C. Stir the mixture at 25°C for 16 h. Add water (30 mL) to the mixture, and extract with ethyl acetate (40 mL x 3). Wash the combined organic phases with saturated brine (15 mL), dry over sodium sulfate, filter, and concentrate. Purify the crude product by silica gel flash column chromatography (eluent: petroleum ether) to obtain tricos-1-ene (3.4 g, 10.54 mmol, yield 85.5%), which is a white solid.

**¹H NMR** (400 MHz, CDCl₃) δ = 5.88 - 5.77 (m, 1H), 5.03 - 4.97 (m, 1H), 4.96 - 4.91 (m, 1H), 2.08 - 2.02 (m, 2H), 1.41 - 1.36 (m, 2H), 1.29 - 1.25 (m, 36H), 0.91 - 0.87 (t, *J* = 6.8 Hz, 3H).

### Step 3

### (4S)-4-[(E,1R)-1-hydroxytetracosane-2-enyl]-2,2-dimethyl-oxazolidine-3-carboxylate

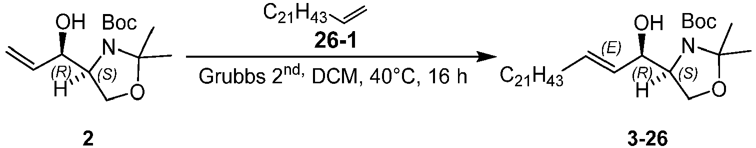

Under nitrogen protection, add benzal-[1,3-bis(2,4,6-trimethylphenyl)imidazolidine-2-methylene]-dichloro-ruthenium; tricyclohexylphosphine (188.64 mg, 222.20 µmol, 0.1 eq) to tert-butyl (4S)-4-[(1R)-1-hydroxyallyl]-2,2-dimethyl-oxazolidine-3-carboxylate (571.78 mg, 2.22 mmol, 1 eq) and tricos-1-ene (2 g, 6.20 mmol, 2.79 eq) in dichloromethane (20 mL). Stir the mixed liquid at 40°C for 16 h. Purify, by silica gel flash column chromatography (eluent: 0 to 25% ethyl acetate in petroleum ether), the crude product after concentration of the reaction solution to obtain (4S)-4-[(E,1R)-1-hydroxytetracosane-2-enyl]-2,2-dimethyl-oxazolidine-3-carboxylate (0.6 g, 1.09 mmol, yield 48.9%), which is a brown solid.

**¹HNMR** (400 MHz, CDCl₃) δ = 5.79 - 5.71 (m, 1H), 5.48 (m, 1H), 4.19 - 3.87 (m, 4H), 2.08 - 2.02 (m, 2H), 1.54 - 1.49 (m, 15H), 1.26 (s, 38H), 0.91 - 0.86 (t, *J* = 6.0 Hz, 3H).

### Step 4

### (4S)-4-[(1R)-1-hydroxytetracosyl]-2,2-dimethyl-oxazolidine-3-carboxylate

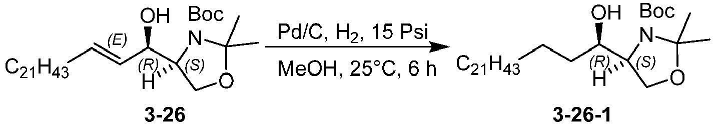

Add Pd/C (40 mg, 37.59 µmol, purity 10%) to a methanol solution (6 mL) of (4S)-4-[(E,1R)-1-hydroxytetracosane-2-enyl]-2,2-dimethyl-oxazolidine-3-carboxylate (300 mg, 543.59 µmol, 1 eq) under nitrogen protection. Stir the mixture under hydrogen atmosphere at 25°C for 6 h. Filter, and concentrate the filtrate to obtain compound (4S)-4-[(1R)-1-hydroxytetracosyl]-2,2-dimethyl-oxazolidine-3-carboxylate (0.28 g, 505.51 µmol, yield 93.0%), which is a white solid.

**¹H NMR** δ = 4.07 - 3.77 (m, 4H), 3.60 - 3.46 (s, 1H), 1.60 - 1.58 (s, 2H), 1.54 - 1.49 (m, 15H), 1.26 (s, 42H), 0.91 - 0.87 (t*, J =* 6.4 Hz, 3H).

### Step 5

### (2S,3R)-2-aminohexacosane-1,3-diol

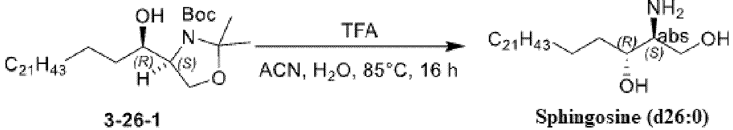

Add an acetonitrile solution (3 mL) of (4S)-4-[(1R)-1-hydroxytetracosyl]-2,2-dimethyl-oxazolidine-3-carboxylate (260 mg, 469.40 µmol, 1 eq) to an aqueous solution (3 mL) of trifluoroacetic acid (92.10 mg, 807.73 µmol, 1.72 eq). Stir the mixture at 85°C for 16 h. Concentrate the reaction solution, and add the resulting crude product into a saturated aqueous sodium bicarbonate solution to adjust the pH to 8. Purify the filtrate after filtration by prep-HPLC (chromatographic column: Welch Xtimate C1 100 x 30 mm, 5 µm; mobile phase: [water (formic acid)-methanol]; gradient: 65%-95% B in 8 min) to obtain (2S,3R)-2-aminohexacosane-1,3-diol (10 mg, 23.42 µmol, yield 5.0%, purity 96.88%), which is a white solid.

**LCMS** Rt = 1.989 min in 3 min Chromatography, 30-100AB ESI calculated value C₂₆H₅₅NO₂ [M+1]⁺ 414.7, and measured value 414.4

**¹H NMR** (400 MHz, CD₃OD) δ = 8.56 - 8.50 (s, 1H), 3.86 - 3.80 (dd, J = 4.0, 11.2 Hz, 1H), 3.79 - 3.74 (m, 1H), 3.73 - 3.66 (dd, J = 8.8, 11.6 Hz, 1H), 3.21 - 3.14 (m, 1H), 1.51 - 1.45 (m, 2H), 1.35 - 1.28 (m, 42H), 0.92 - 0.88 (t, J = 6.4 Hz, 3H).

### Step 6

### (E,2S,3R)-2-aminohexacosane-4-ene-1,3-diol

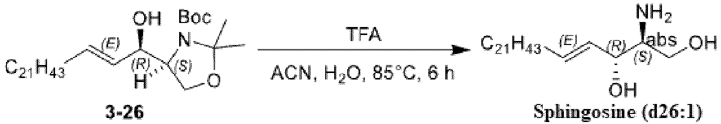

Add an acetonitrile solution (3 mL) of (4S)-4-[(E,1R)-1-hydroxytetracosane-2-enyl]-2,2-dimethyl-oxazolidine-3-carboxylate (300 mg, 543.59 µmol, 1 eq) to an aqueous solution (3 mL) of trifluoroacetic acid (92.10 mg, 807.73 µmol, 1.49 eq). Stir the mixture at 85°C for 6 h. Concentrate the reaction solution, and add the resulting crude product into a saturated aqueous sodium bicarbonate solution to adjust the pH to 8. Purify the filtrate after filtration by prep-HPLC (chromatographic column: Welch Xtimate C1 100 x 30 mm, 5 µm; mobile phase: [water (formic acid)-methanol]; gradient: 65%-95% B in 20 min), and freeze dry to obtain (E,2S,3R)-2-aminohexacosane-4-ene-1,3-diol (12.7 mg, 30.82 µmol, yield 5.6%, purity 99.9%), which is a white solid.

**LCMS** Rt = 1.839 min in 3 min Chromatography, 30-100AB ESI calculated value C₂₆H₅₃NO₂ [M+1]⁺ 412.7, and measured value 412.4

**¹H NMR** (400 MHz, CD₃OD) δ = 8.59 - 8.50 (s, 1H), 5.83 - 5.72 (m, 1H), 5.53 - 5.44 (m, 1H), 4.12 - 4.06 (m, 1H), 3.76 - 3.68 (m, 1H), 3.60 - 3.51 (m, 1H), 2.97 - 2.88 (m, 1H), 2.12 - 2.06 (m, 2H), 1.44 - 1.40 (m, 2H), 1.33 - 1.27 (m, 36H), 0.93 - 0.87 (t, J = 5.2 Hz, 3H).

### Sphingosine (d27:1) & (d27:0)

### Synthesis route map

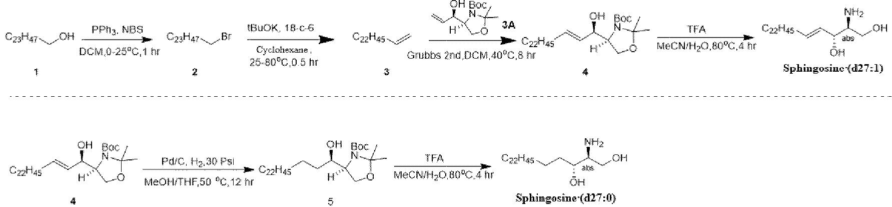

### Synthesis route:

### Step 1

### Tetracosane-1-bromide

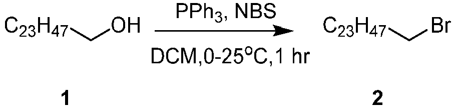

Add triphenylphosphine (4.44 g, 16.92 mmol) to a DCM solution (50 mL) of tetracosan-1-ol (5 g, 14.10 mmol). Then, add N-bromosuccinimide (3.01 g, 16.92 mmol) to the mixture at 0°C. Stir the mixture at 25°C for 1 h. Add saturated Na₂SO₃ solution (50 mL), and extract the aqueous phase with DCM (50 mL x 3). Wash the combined organic phases with saturated brine (100 mL), dry with anhydrous Na₂SO₄, filter, and concentrate. Purify the crude product by silica gel flash column chromatography (eluent: petroleum ether) to obtain tetracosane-1-bromide (3.8 g, 9.10 mmol, yield 64.55%), which is a white solid.

**¹H NMR** (400 MHz, CDCl₃) δ = 3.41 (t, *J =* 6.8 Hz, 2H), 1.92 - 1.82 (m, 2H), 1.48 - 1.39 (m, 2H), 1.36 - 1.13 (m, 40H), 0.89 (t, *J* = 6.8 Hz, 3H).

### Step 2

### Tetracos-1-ene

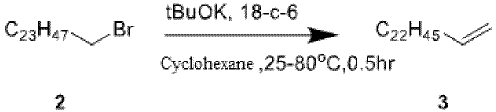

At 25°C, add potassium tert-butoxide (2.45 g, 21.84 mmol) to a cyclohexane solution (40 mL) of tetracosane-1-bromide (3.8 g, 9.10 mmol) and 18-crown-6 (577.32 mg, 2.18 mmol). Stir the mixture at 80°C for 0.5 h. Add the mixture into water (50 mL), and extract the aqueous layer with EtOAc (50 mL x 3). Wash the combined organic phases with saturated brine (100 mL), dry with anhydrous Na₂SO₄, filter, and concentrate under reduced pressure. Purify the crude product by silica gel flash column chromatography (eluent: petroleum ether) to obtain tetracos-1-ene (2.1 g, 6.24 mmol, yield 68.55%), which is a white solid.

**¹H NMR** (400 MHz, CDCl₃) δ = 5.98 - 5.73 (m, 1H), 5.05 - 4.92 (m, 2H), 2.10 - 2.00 (m, 2H), 1.41 - 1.37 (m, 2H), 1.30 - 1.28 (m, 38H), 0.89 (t, *J* = 6.8 Hz, 3H).

### Step 3

### (4S)-4-[(E,1R)-1-hydroxypentacosane-2-enyl]-2,2-dimethyl-oxazolidine-3-carboxylate

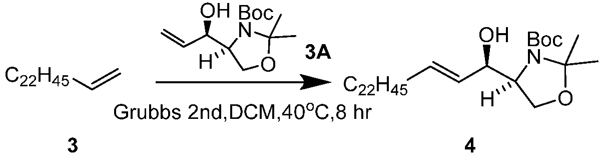

Under nitrogen protection, degas a mixed liquid of tert-butyl (4S)-4-[(1R)-1-hydroxyallyl]-2,2-dimethyl-oxazolidine-3-carboxylate (504.51 mg, 1.96 mmol), tetracos-1-ene (600 mg, 1.78 mmol), and benzal-[1,3-bis(2,4,6-trimethylphenyl)imidazolidin-2-ylidene]-dichloro-ruthenium; and tricyclohexylphosphine (151.32 mg, 178.23 µmol) in dichloromethane (6 mL), and replace with nitrogen three times. Stir the mixture at 40°C for 8 h under nitrogen protection. Combine this reaction solution with another batch of the reaction solution prepared from tetracos-1-ene (600 mg). Purify, by silica gel flash column chromatography (eluent: 0 to 14% ethyl acetate in petroleum ether), the crude product after concentration to obtain (4S)-4-[(E,1R)-1-hydroxypentacosane-2-enyl]-2,2-dimethyl-oxazolidine-3-carboxylate (880 mg, 1.56 mmol, yield 43.62%), which is a brown oily matter.

**¹H NMR** (400 MHz, CDCl₃) δ = 5.82 - 5.68 (m, 1H), 5.47 - 5.38 (m, 1H), 4.15 - 3.74 (m, 4H), 1.49 - 1.25 (m, 57H), 0.89 - 0.85 (m, 3H).

### Step 4

### (E,2S,3R)-2-aminoheptacosane-4-ene-1,3-diol

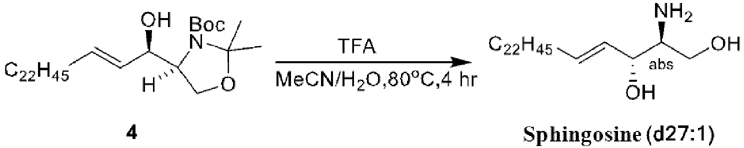

Add an aqueous solution (3 mL) of trifluoroacetic acid (1.77 g, 15.55 mmol, 1.16 mL) to an acetonitrile solution (3 mL) of (4S)-4-[(E,1R)-1-hydroxypentacosane-2-enyl]-2,2-dimethyl-oxazolidine-3-carboxylate (880 mg, 1.56 mmol). Stir the mixture at 80°C for 4 h. Concentrate the reaction solution to obtain (E,2S,3R)-2-aminoheptacosane-4-ene-1,3-diol (660 mg, 1.55 mmol, yield 99.70%). Purify (330 mg, 775.14 µmol) thereof by prep-HPLC (chromatographic column: Welch Xl timate C4 100 x 30 x 10 µm; mobile phase: [water (formic acid)-methanol]; gradient: 70% to 55% B in 20 min) to obtain (E,2S,3R)-2-aminoheptacosane-4-ene-1,3-diol (10.8 mg, 20.01 µmol, yield 2.57%, trifluoroacetate), which is a white solid.

**¹H NMR** (400 MHz, CD₃OD) δ = 8.52 (br s, 1H), 5.96 - 5.74 (m, 1H), 5.58 - 5.43 (m, 1H), 4.34 - 4.26 (m, 1H), 3.80 - 3.60 (m, 2H), 3.21 - 3.14 (m, 1H), 2.15 - 2.05 (m, 2H), 1.43 - 1.39 (m, 2H), 1.34 - 1.26 (m, 38H), 0.93 - 0.85 (m, 3H).

**¹⁹F NMR** (376.5 MHz, CD₃OD) δ = -76.907.

**LCMS** Rt = 1.191 min in 1.5 min Chromatography, 5-95AB ESI calculated value C₂₇H₅₆NO₂ [M+H]⁺ 426.4, and measured value 426.3

**HPLC** Rt = 4.980 min, in 8 min chromatography, ELSD, purity98.085%.

### Step 5

### (4S)-4-[(1R)-1-hydroxypentacosyl]-2,2-dimethyl-oxazolidine-3-carboxylate

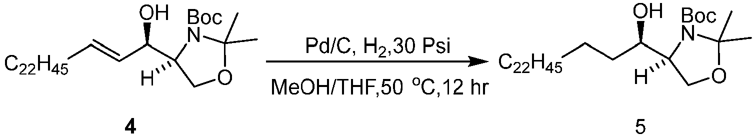

Add Pd/C (188.05 mg, 176.71 µmol, purity 10%) to a mixed solution of (4S)-4-[(E,1R)-1-hydroxypentacosane-2-enyl]-2,2-dimethyl-oxazolidine-3-carboxylate (1 g, 1.77 mmol) in methanol (5 mL) and tetrahydrofuran (5 mL) under nitrogen protection. Degas the suspension, and replace with hydrogen three times. Stir the mixture under hydrogen atmosphere (30 psi) at 50°C for 12 h. Add tetrahydrofuran (50 mL) to the mixture, and filter. Wash the filter cake three times with tetrahydrofuran (30 mL). Purify the crude product obtained by concentration of the filtrate by silica gel flash column chromatography (eluent: 0 to 14% ethyl acetate in petroleum ether) to obtain compound (4S)-4-[(1R)-1-hydroxypentacosyl]-2,2-dimethyl-oxazolidine-3-carboxylate (600 mg, 1.06 mmol, yield 59.79%), which is a yellow solid.

**¹H NMR** (400 MHz, CDCl₃) δ = 4.26 - 3.75 (m, 4H), 3.72 - 3.36 (m, 1H), 1.58 (s, 9H), 1.49 (s, 6H), 1.45-1.19 (m, 46H), 0.88 (t, *J* = 6.8 Hz, 3H).

### Step 6

### (2S,3R)-2-aminoheptacosane-1,3-diol

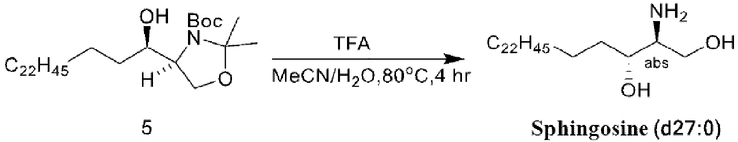

Add an aqueous solution (3 mL) of trifluoroacetic acid (602.30 mg, 5.28 mmol, 392.38 µL) to an acetonitrile solution (3 mL) of (4S)-4-[(E,1R)-1-hydroxypentacosane-2-enyl]-2,2-dimethyl-oxazolidine-3-carboxylate (600 mg, 1.06 mmol). Stir the mixture at 80°C for 4 h. Add a saturated aqueous solution of sodium bicarbonate to adjust the pH value to 8. Filter, and dry the filter cake under reduced pressure. At room temperature, place the filter cake in hydrochloric acid/dioxane (2 M, 10 mL), stir, and concentrate. Slurry the crude product with methanol (10 mL), and filter. Freeze dry the filter cake to obtain compound (2S,3R)-2-aminoheptacosanyl-1,3-diol (105 mg, 215.07 µmol, yield 20.36%, purity 95.082%, hydrochloride), which is a white solid.

**¹H NMR** (400 MHz, DMSO-*d*₆) δ = 8.04 (br s, 3H), 5.03 (br s, 2H), 3.78 - 3.55 (m, 3H), 3.06 - 2.94 (m, 1H), 1.46 - 1.39 (m, 2H), 1.37 - 1.15 (m, 44H), 0.86 (t, *J* = 6.8 Hz, 3H).

**LCMS** Rt = 5.664 min in 7 min Chromatography, 50-100AB ESI calculated value C₂₇H₅₈NO₂ [M+H]⁺ 428.4, and measured value 428.4

**HPLC** Rt = 5.630 min, in 8 min chromatography, ELSD, purity 95.082%.

### Sphingosine (d28:1) & (d28:0)

### Synthesis route map

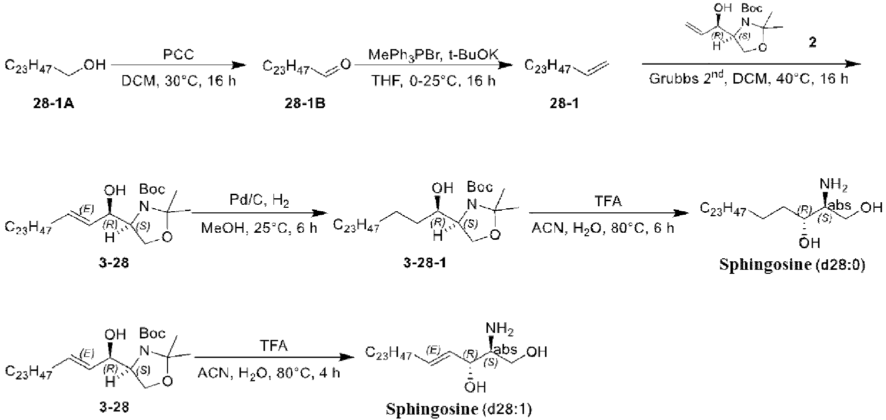

### Synthesis route:

### Step 1

### Tetracosanal

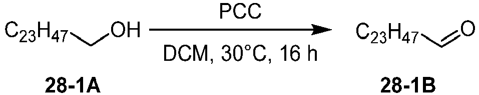

Add pyridinium chlorochromate (PCC) (7.29 g, 33.84 mmol, 1.5 eq) to a DCM solution (400 mL) of tetracosan-1-ol (8 g, 22.56 mmol, 1 eq). Stir the mixture at 30°C for 16 h. Add silica gel (20 g) to the brown suspension. Stir the resulting mixture for 30 min, and purify the concentrated crude product by silica gel flash column chromatography (eluent: 0 to 10% ethyl acetate in petroleum ether) to obtain tetracosanal (5.65 g, 16.03 mmol, yield 71.0%), which is a white solid.

**¹H NMR** (400 MHz, CDCl₃) δ = 9.78 - 9.76 (t, *J* =2.0 Hz, 1H), 2.48 - 2.38 (m, 2H), 1.67 - 1.59 (m, 2H), 1.26 (s, 40H), 0.90 - 0.86 (t, *J* = 6.8 Hz, 3H).

### Step 2

### Pentacos-1-ene

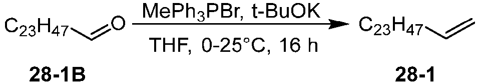

At 0°C, added potassium tert-butoxide (6.36 g, 56.72 mmol, 5 eq) to a THF solution (15 mL) of methyl(triphenyl)phosphonium bromide (20.26 g, 56.72, 5 eq). Stir the resulting yellow suspension at 25°C for 1 h, and cool to 0°C again. Add a THF solution (40 mL) of tetracosanal (4 g, 11.34 mmol, 1 eq) dropwise at 0°C. Stir the mixture at 25°C for 16 h. Add water (30 mL) to the mixture, and extract with ethyl acetate (40 mL x 3). Wash the combined organic phases with saturated brine (15 mL), dry over sodium sulfate, filter, and concentrate. Purify the crude product by silica gel flash column chromatography (eluent: petroleum ether) to obtain pentacos-1-ene (3.20 g, 9.13 mmol, yield 80.4%), which is a light yellow solid.

**¹H NMR** (400 MHz, CDCl₃) δ = 5.88 - 5.77 (m, 1H), 5.03 - 4.91 (m, 2H), 2.06 - 2.03 (m, 2H), 1.29 - 1.25 (m, 42H), 0.91 - 0.86 (t, *J* = 6.4 Hz, 3H).

### Step 3

### (4S)-4-[(E,1R)-1-hydroxyhexacosane-2-enyl]-2,2-dimethyl-oxazolidine-3-carboxylate

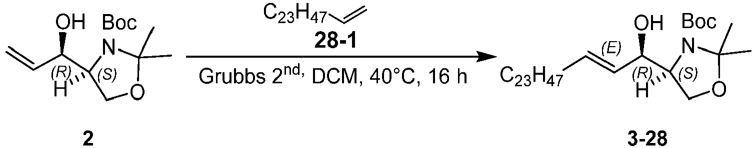

Under nitrogen protection, add benzal-[1,3-bis(2,4,6-trimethylphenyl)imidazolidine-2-methylene]-dichloro-ruthenium; tricyclohexylphosphine (183.44 mg, 216.07 µmol, 0.1 eq) to tert-butyl (4S)-4-[(1R)-1-hydroxyallyl]-2,2-dimethyl-oxazolidine-3-carboxylate (556 mg, 2.16 mmol, 1 eq) and pentacos-1-ene (2.11 g, 6.03 mmol, 2.79 eq) in dichloromethane (20 mL). Stir the mixed liquid at 40°C for 16 h. Purify, by silica gel flash column chromatography (eluent: 0 to 25% ethyl acetate in petroleum ether), the crude product after concentration of the reaction solution to obtain (4S)-4-[(E,1R)-1-hydroxyhexacosane-2-enyl]-2,2-dimethyl-oxazolidine-3-carboxylate (438 mg, 755.26 µmol, yield 34.9%), which is a yellow solid.

**¹HNMR** (400 MHz, CDCl₃) δ = 5.76 - 5.71 (m, 1H), 5.48 (m, 1H), 4.19 - 3.87 (m, 4H), 2.08 - 2.02 (m, 2H), 1.54 - 1.49 (m, 15H), 1.26 (s, 44H), 0.91 - 0.86 (t, *J* = 6.4 Hz, 3H).

### Step 4

### (4S)-4-[(1R)-1-hydroxyhexacosyl]-2,2-dimethyl-oxazolidine-3-carboxylate

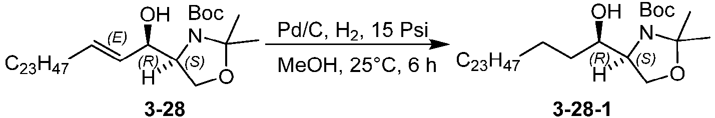

Add Pd/C (30 mg, 28.19 µmol, purity 10%) to a methanol solution (3 mL) of (4S)-4-[(E,1R)-1-hydroxyhexacosane-2-enyl]-2,2-dimethyl-oxazolidine-3-carboxylate (300 mg, 517.30 µmol, 1 eq) under nitrogen protection. Stir the mixture under hydrogen atmosphere at 25°C for 6 h. Filter the mixture. Concentrate the filtrate to obtain compound (4S)-4-[(1R)-1-hydroxyhexacosyl]-2,2-dimethyl-oxazolidine-3-carboxylate (231 mg, 396.94 µmol, yield 76.7%), which is a white solid.

**¹H NMR** δ = 4.07 - 3.77 (m, 4H), 3.60 - 3.46 (s, 1H), 1.60 - 1.58 (s, 2H), 1.54 - 1.49 (m, 15H), 1.26 (s, 48H), 0.91 - 0.87 (t, *J* = 6.4 Hz, 3H).

### Step 5

### (2S,3R)-2-aminooctacosane-1,3-diol

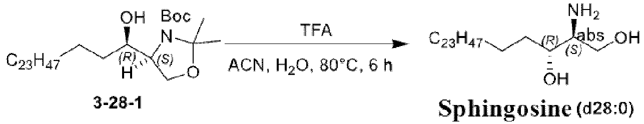

Add an aqueous solution (3 mL) of trifluoroacetic acid (92.10 mg, 807.75 µmol, 60 µL, 2.03 eq) to an acetonitrile solution (3 mL) of (4S)-4-[(1R)-1-hydroxyhexacosyl]-2,2-dimethyl-oxazolidine-3-carboxylate (231 mg, 396.94 µmol, 1 eq). Stir the mixture at 80°C for 6 h. Concentrate the reaction solution, add a saturated aqueous sodium bicarbonate solution to adjust the pH to 8, and filter. Purify the filtrate by prep-HPLC (chromatographic column: Welch Xl timate C1 100 x 30 mm, 5 µm; mobile phase: [water (formic acid)-methanol]; gradient: 65% to 95% B in 8 min) to obtain (2S,3R)-2-aminooctacosane-1,3-diol (9 mg, 20.37 µmol, yield 5.1%, purity 100%), which is a white solid.
**LCMS** Rt = 2.854 min in 3 min Chromatography, 30-100AB ESI calculated value C₂₈H₅₉NO₂ [M+1]⁺ 442.7, and measured value 442.4
**¹H NMR** (400 MHz, CD₃OD) δ = 8.54 (s, 1H), 3.86 - 3.80 (dd, *J =* 7.2, 11.2 Hz, 1H), 3.77 - 3.74 (m, 1H), 3.73 - 3.66 (dd, *J* = 8.8, 11.6 Hz, 1H), 3.16 - 3.13 (m, 1H), 1.52 - 1.45 (m, 2H), 1.35 - 1.28 (m, 46H), 0.92 - 0.88 (t, *J =* 6.0 Hz, 3H).

### Step 6

### (E,2S,3R)-2-aminooctacosane-4-ene-1,3-diol

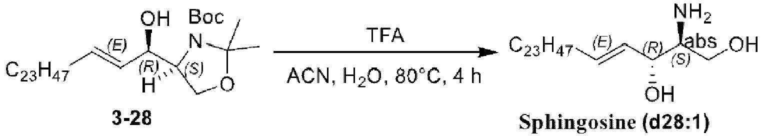

Add an acetonitrile solution (4 mL) of (4S)-4-[(E,1R)-1-hydroxyhexacosane-2-enyl]-2,2-dimethyl-oxazolidine-3-carboxylate (360 mg, 620.76 µmol, 1 eq) to an aqueous solution (7 mL) of trifluoroacetic acid (110.52 mg, 969.30 µmol, 72.00 µL, 1.56 eq). Stir the mixture at 80°C for 4 h. Concentrate the reaction solution, add a saturated aqueous sodium bicarbonate solution to adjust the pH to 8, and filter. Purify the filtrate by prep-HPLC (chromatographic column: Welch Xtimate C1 100 x 30 mm, 5 µm; mobile phase: [water (formic acid)-methanol]; gradient: 60% to 90% B in 8 min) to obtain (E,2S,3R)-2-aminooctacosane-4-ene-1,3-diol (6.99 mg, 15.90 µmol, yield 2.3%, purity 100%), which is a white solid.
**LCMS** Rt = 0.478 min in 0.8 min Chromatography, 30-100AB ESI calculated value C₂₈H₅₇NO₂ [M+1]⁺ 440.7, and measured value 440.4
**¹H NMR** (400 MHz, CDCl₃) δ = 5.85 - 5.63 (m, 1H), 5.57 - 5.43 (m, 1H), 4.24 - 3.93 (m, 1H), 3.74 - 3.57 (m, 2H), 2.95 - 2.78 (m, 1H), 2.45 - 2.10 (m, 4H), 2.10 - 1.90 (m, 2H), 1..6 - 1.41 (m, 2H), 1.4 - 1.0 (s, 40H), 0.91 - 0.70 (m, 3H).

### Sphingosine (d29:1) & (d29:0)

### Synthesis route map

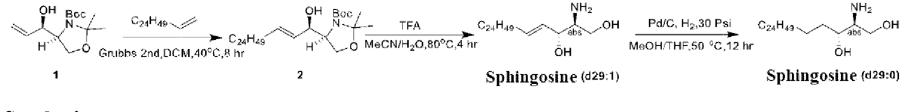

### Synthesis route:

### Step 1

### (4S)-4-[(E,1R)-1-hydroxyheptacosane-2-enyl]-2,2-dimethyl-oxazolidine-3-carboxylate

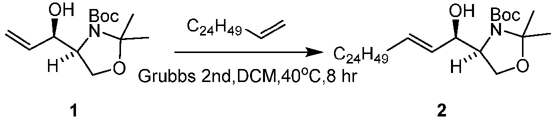

Degas a mixed liquid of tert-butyl (4S)-4-[(1R)-1-hydroxyallyl]-2,2-dimethyl-oxazolidine-3-carboxylate (500 mg, 1.94 mmol), hexacos-1-ene (921.20 mg, 2.53 mmol), and benzal-[1,3-bis(2,4,6-trimethylphenyl)imidazolidin-2-ylidene]-dichloro-ruthenium; and tricyclohexylphosphine (164.96 mg, 194.31 µmol) in dichloromethane (6 mL), and replace with nitrogen three times. Stir the mixture at 40°C for 8 h under nitrogen protection. Purify, by silica gel flash column chromatography (eluent: 0 to 14% ethyl acetate in petroleum ether), the crude product after concentration of the reaction solution to obtain (4S)-4-[(E,1R)-1-hydroxyheptacosane-2-enyl]-2,2-dimethyl-oxazolidine-3-carboxylate (280 mg, 471.41 µmol, yield 24.26%), which is a yellow oily matter.

**¹H NMR** (400 MHz, CDCl₃) δ = 5.81 - 5.67 (m, 1H), 5.50 - 5.38 (m, 1H), 4.20 - 3.84 (m, 4H), 2.06 - 2.00 (m, 2H), 1.60 - 1.26 (m, 44H), 1.25 - 1.24 (m, 15H), 0.88 (t, *J* = 6.4 Hz, 3H).

### Step 2

### (E,2S,3R)-2-aminononacosane-4-ene-1,3-diol

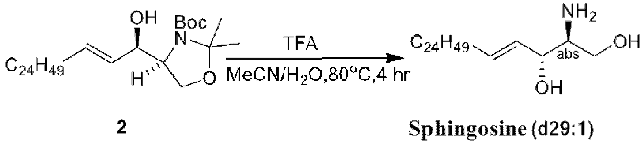

Add an aqueous solution (3 mL) of trifluoroacetic acid (537.50 mg, 4.71 mmol, 350.16 µL) to an acetonitrile solution (3 mL) of (4S)-4-[(E,1R)-1-hydroxyheptacosane-2-enyl]-2,2-dimethyl-oxazolidine-3-carboxylate (280 mg, 471.41 µmol). Stir the mixture at 80°C for 4 h. Add a saturated aqueous solution of sodium bicarbonate to the reaction solution to adjust the pH value to 8. Filter, and wash the filter cake three times with water (10 mL). Dry the filter cake to obtain (E,2S,3R)-2-aminononacosane-4-ene-1,3-diol (200 mg, 440.74 µmol, yield 93.49%), which is a brown solid. Purify (70 mg, 154.26 µmol) thereof twice by prep-HPLC (chromatographic column: Welch Xltimate C4 100 x 30 x 10µm; mobile phase: [water (formic acid)-methanol]; gradient: 70% to 85% B in 20 min) to obtain (E,2S,3R)-2-aminononacosane-4-ene-1,3-diol (4.3 mg, 8.25 µmol, yield 0.05%, purity 95.870%, formate), which is a white solid.
**¹H NMR** (400 MHz, DMSO-*d*₆) δ = 8.43 (br s, 3H), 5.77 - 5.18 (m, 2H), 3.89 - 3.77 (m, 1H), 3.63 - 3.35 (m, 1H), 3.22 - 3.20 (m, 1H), 2.74 - 2.58 (m, 1H), 2.04 - 1.98 (m, 2H), 1.40 -1.16 (m, 44H), 0.90 - 0.83 (m,3H).
**LCMS** Rt = 5.960 min in 7 min Chromatography, 50-100AB ESI calculated value C₂₉H₆₀NO₂ [M+H]⁺ 454.5, and measured value 454.5
**HPLC** Rt = 5.996 min in 15 min chromatography, ELSD, purity 95.870%.

### Step 3

### (2S,3R)-2-aminononacosane-1,3-diol

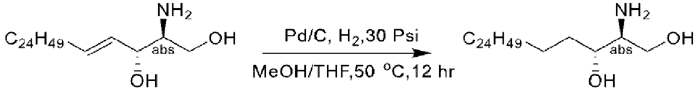

### Sphingosine (d29:1) Sphingosine (d29:0)

Add Pd/C (152.44 mg, 143.24 µmol, purity 10%) to a mixed solution of (E,2S,3R)-2-aminononacosane-4-ene-1,3-diol (130 mg, 286.48 µmol) in methanol (2 mL) and tetrahydrofuran (2 mL) under nitrogen protection. Degas the reaction solution, and replace with hydrogen three times. Stir the mixture under hydrogen atmosphere (30 psi) at 50°C for 12 h. Add tetrahydrofuran (10 mL) to the reaction solution. Wash the filter cake three times with tetrahydrofuran (10 mL). Concentrate the filtrate, and purify by prep-HPLC (chromatographic column: Welch Xltimate C4 100 x 30 x 10 µm; mobile phase: [water (formic acid)-methanol]; gradient: 70% to 85% B in 20 min) to obtain compound (2S,3R)-2-aminononacosane-1,3-diol (6.4 mg, 12.54 µmol, yield 4.38%, purity 98.296%, formate), which is a white solid.

**¹H NMR** (400 MHz, DMSO-*d*₆) δ = 8.37 (s, 3H), 3.64 - 3.45 (m, 1H), 3.28 - 3.27 (m, 2H), 2.71 - 2.60 (m, 1H), 1.45 - 1.23 (m, 50H), 0.88 - 0.82 (m, 3H).

**LCMS** Rt = 6.199 min in 7 min Chromatography, 50-100AB ESI calculated value C₂₉H₆₂NO₂ [M+H]⁺ 456.5, and measured value 456.5.

**HPLC** Rt = 6.735 min in 15min chromatography, ELSD, purity 98.296%.

### Sphingosine (d30:0)

### Synthesis route map:

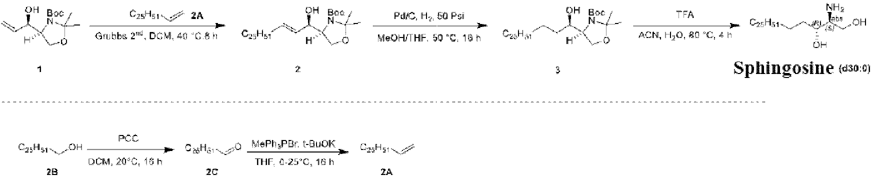

### Synthesis route:

### Step 1

### Tert-butyl (4S)-4-[(E,1R)-1-hydroxyoctacosane-2-enyl]-2,2-dimethyl-oxazolidine-3-carboxylate

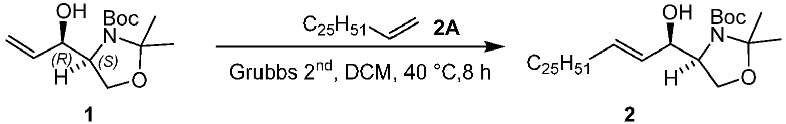

Add benzal-[1,3-bis(2,4,6-trimethylphenyl)imidazolidine-2-methylene]-dichloro-ruthenium; tricyclohexylphosphine (224.17 mg, 264.05 µmol) to a dichloromethane solution (10 mL) of tert-butyl (4S)-4-[(1R)-1-hydroxyallyl]-2,2-dimethyl-oxazolidine-3-carboxylate (500 mg, 1.94 mmol) and hexacos-1-ene (1.30 g, 3.43 mmol). Degas the mixed liquid, and replace with hydrogen three times. Stir the mixture at 40°C for 8 h under nitrogen protection. Purify, by silica gel flash column chromatography (eluent: 0 to 10% ethyl acetate in petroleum ether), the crude product after concentration of the reaction solution to obtain tert-butyl (4S)-4-[(E,1R)-1-hydroxyoctacosane-2-enyl]-2,2-dimethyl-oxazolidine-3-carboxylate (280 mg, 460.54 µmol, yield 17.44%), which is a brown solid.

**¹H NMR** (400 MHz, CDCl₃) δ = 5.81 - 5.67 (m, 1H), 5.50 - 5.35 (m, 1H), 4.25 - 3.77 (m, 4H), 2.10 - 1.91 (m, 2H), 1.58 - 1.45 (m, 15H), 1.40 - 1.08 (m, 46H), 0.88 (t, *J* = 6.8 Hz, 3H).

### Step 2

### Tert-butyl (4S)-4-[(1R)-1-hydroxyoctacosyl]-2,2-dimethyl-oxazolidine-3-carboxylate

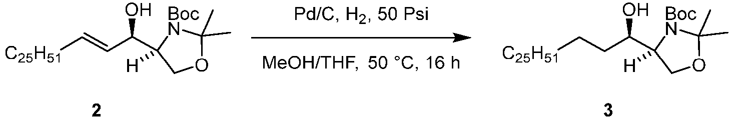

Add Pd/C (50 mg, purity 10%) to a mixed solution of tert-butyl (4S)-4-[(E,1R)-1-hydroxyoctacosane-2-enyl]-2,2-dimethyl-oxazolidine-3-carboxylate (280 mg, 460 µmol) in tetrahydrofuran (10 mL) and methanol (5 mL) under nitrogen protection. Degas the suspension, and replace with hydrogen three times. Stir the mixture under hydrogen atmosphere (50 psi) at 50°C for 16 h. Filter, and wash the filter cake three times with tetrahydrofuran (30 mL). Purify the crude product obtained by concentration of the filtrate by silica gel plate chromatography (dispersing agent: petroleum ether: ethyl acetate 4:1) to obtain compound tert-butyl (4S)-4-[(1R)-1-hydroxyoctacosyl]-2,2-dimethyl-oxazolidine-3-carboxylate (160 mg, 262.29 µmol, yield 56.95%), which is a white solid.

**¹H NMR** (400 MHz, CDCl₃) δ = 4.15-3.43 (m, 4H), 1.58 (s, 6H), 1.49 (s, 9H), 1.45 - 1.10 (m, 52H), 0.88 (t, *J =* 7.2 Hz, 3H).

### Step 3

### (2S,3R)-2-aminotriacontane-1,3-diol

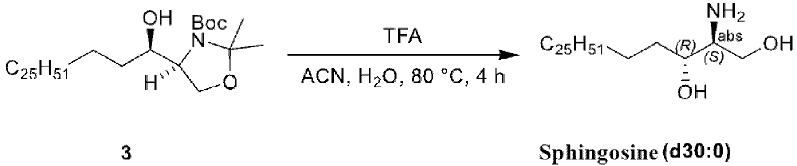

Add trifluoroacetic acid (307 mg, 2.69 mmol, 0.2 mL) to mixed solvents of an acetonitrile solution (1.5 mL) of tert-butyl(4S)-4-[(1R)-1-hydroxyoctacosyl]-2,2-dimethyl-oxazolidine-3-carboxylate (160 mg, 262 µmol) and water (1.5 mL). Stir the mixture at 80°C for 4 h under nitrogen atmosphere. Add a saturated aqueous solution of sodium bicarbonate to the reaction solution to adjust the pH value to 8. Filter in batches, and rinse the filter cake with methanol (5 mL). Disperse the filter cake in hydrochloric acid/dioxane (2 M, 5 mL), and stir for 20 min. Slurry the crude product with methanol (3 mL), and filter. Freeze dry the filter cake to obtain compound (2S,3R)-2-aminotriacontane-1,3-diol (53.8 mg, 92.15 µmol, yield 35.13%, trifluoroacetate), which is a white solid.

**MS** Rt = 0.464 min in 0.8 min Chromatography, 30-100 AB, ESI calculated value C30H64NO2 [M+H]⁺ 470.5, and measured value 470.4.

**¹H NMR** (400 MHz, DMSO-d6) δ = 7.71 (br s, 3H), 4.95 - 4.82 (m, 2H), 3.74 - 3.64 (m, 2H), 3.62 - 3.53 (m, 1H), 3.10 -3.01 (m, 1H), 1.32 - 1.25 (m, 52H), 0.87 (t, J = 6.8 Hz, 3H).

**¹⁹F NMR** (282 MHz, DMSO-d6) δ = -73.413.

### Step 4

### Hexacosanal

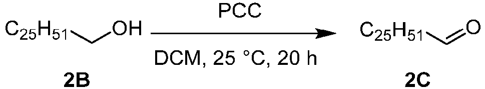

Add silica gel (5 g) and pyridinium chlorochromate (PCC) (5.63 g, 26.12 mmol) to a DCM solution (350 mL) of hexacosan-1-ol (5 g, 13.06 mmol). Stir the mixture at 25°C for 20 h. Filter the reaction solution through diatomaceous earth, and concentrate the filtrate to obtain hexacosanal (4.97 g, 13.06 mmol), which is a white solid with no need for further purification.

**¹H NMR** (400 MHz, CDCl₃) δ = 9.76 (s, 1H), 2.45 - 2.34 (m, 2H), 1.70 - 1.57 (m, 2H), 1.34 - 1.20 (m, 44H), 0.88 (t, *J =* 6.8 Hz, 3H).

### Step 5

### Heptacos-1-ene

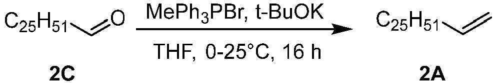

At 0°C, add potassium tert-butoxide (4.39 g, 39.17 mmol) to a THF solution (120 mL) of methyl(triphenyl)phosphonium bromide (13.99 g, 39.17 mmol). Stir the resulting suspension at 25°C for 1 h, and cool to 0°C again. At 0°C, add dropwise a THF solution (60 mL) of hexacosanal (4.97 g, 13.06 mmol). Stir the mixture at 25°C for 16 h. Add water (100 mL) to the mixture, and extract with ethyl acetate (100 mL x 3). Wash the combined organic phases with saturated brine (200 mL), dry over anhydrous sodium sulfate, filter, and concentrate. Purify the crude product by silica gel flash column chromatography (eluent: petroleum ether) to obtain heptacos-1-ene (3.85 g, 10.17 mmol, yield 77.87%), which is a white solid.

**¹H NMR** (400 MHz, CDCl₃) δ = 5.88 - 5.75 (m, 1H), 5.04 - 4.88 (m, 2H), 2.04 (q, J = 7.2 Hz, 2H), 1.45 - 1.32 (m, 2H), 1.40 - 1.15 (m, 44H), 0.88 (t, J = 6.8 Hz, 3H).

### Sphingosine (d30:1)

### Synthesis route map

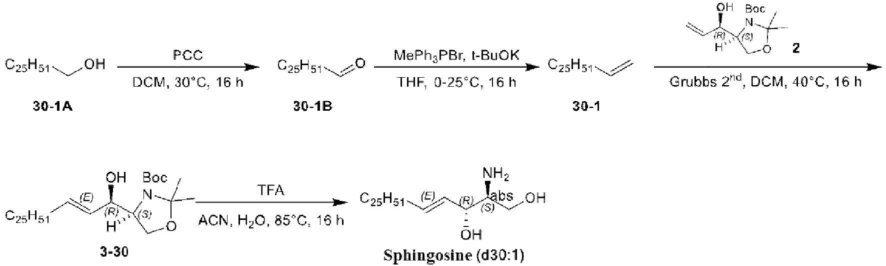

### Synthesis route:

### Step 1

### Hexacosanal

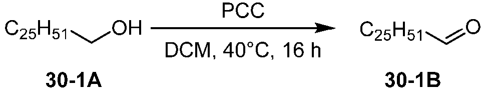

Add pyridinium chlorochromate (PCC) (1.61 g, 7.38 mmol, 1.5 eq) to a DCM solution (85 mL) of hexacosan-1-ol (1.9 g, 4.96 mmol, 1 eq). Stir the mixture at 40°C for 16 h. Add silica gel (3.5 g) to the brown suspension. Stir the resulting mixture for 30 min, and purify the concentrated crude product by silica gel flash column chromatography (eluent: 0 to 10% ethyl acetate in petroleum ether) to obtain hexacosanal (690 mg, 1.81 mmol, yield 36.5%), which is a white solid.

**¹H NMR** (400 MHz, CDCl₃) δ = 9.80 - 9.75 (t, *J* = 2.0 Hz, 1H), 2.48 - 2.38 (m, 2H), 1.60 (br s, 2H), 1.29 - 1.25 (m, 44H), 0.91 - 0.87 (t, *J* = 6.8 Hz, 3H).

### Step 2

### Heptacos-1-ene

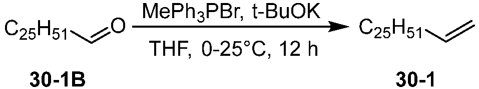

Add potassium tert-butoxide (530.57 mg, 4.73 mmol, 3 eq) to a THF solution (5 mL) of methyl(triphenyl)phosphonium bromide (1.69 g, 4.73 mmol, 3 eq) at 0°C. Stir the resulting yellow suspension at 25 °C for 1 h, and cool to 0°C again. Add a THF solution (5 mL) of hexacosanal (0.6 g, 1.58 mmol, 1 eq) dropwise at 0°C. Stir the mixture at 25°C for 12 h. Add water (10 mL) to the mixture, and extract with ethyl acetate (15 mL x 3). Wash the combined organic phases with saturated brine (5 mL), dry over sodium sulfate, filter, and concentrate. Purify the crude product by silica gel flash column chromatography (eluent: petroleum ether) to obtain heptacos-1-ene (0.31 g, 0.87 mmol, yield 45.0%), which is a colorless solid.

**¹H NMR** (400 MHz, CDCl₃) δ = 5.89 - 5.75 (m, 1H), 5.04 - 4.97 (d, *J =* 17.2 Hz, 1H), 4.97 - 4.90 (d, *J* = 10.0 Hz, 1H), 2.08 - 2.02 (m, 2H), 1.40 - 1.36 (m, 2H), 1.27 (s, 44H), 0.91 - 0.87 (t, *J* = 6.4 Hz, 3H).

### Step 3

### (4S)-4-[(E,1R)-1-hydroxyoctacosane-2-enyl]-2,2-dimethyl-oxazolidine-3-carboxylate

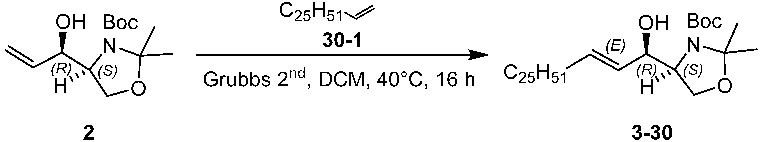

Under nitrogen protection, add benzal-[1,3-bis(2,4,6-trimethylphenyl)imidazolidine-2-methylene]-dichloro-ruthenium; tricyclohexylphosphine (24.10 mg, 28.39 µmol, 0.1 eq) to tert-butyl (4S)-4-[(1R)-1-hydroxyallyl]-2,2-dimethyl-oxazolidine-3-carboxylate (146.12 mg, 567.85 µmol, 2 eq) and heptacos-1-ene (300 mg, 792.15 µmol, 2.79 eq) in dichloromethane (15 mL). Stir the mixed liquid at 40°C for 16 h. Purify, by silica gel flash column chromatography (eluent: 0 to 25% ethyl acetate in petroleum ether), the crude product after concentration of the reaction solution to obtain (4S)-4-[(E,1R)-1-hydroxyoctacosane-2-enyl]-2,2-dimethyl-oxazolidine-3-carboxylate (55 mg, 90.46 µmol, yield 31.9%), which is a gray solid.

**¹H NMR** (400 MHz, CDCl₃) δ = 5.79 - 5.71 (m, 1H), 5.48 (br s, 1H), 4.19 - 3.87 (m, 4H), 2.08 - 2.02 (m, 2H), 1.54 - 1.49 (m, 15H), 1.26 (m, 46H), 0.91 - 0.86 (t, *J* = 6.4 Hz, 3H).

### Step 4

### (E,2S,3R)-2-aminotriacontyl-4-ene-1,3-diol

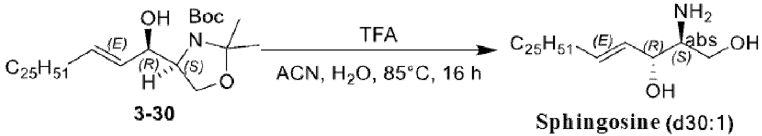

Add an acetonitrile solution (1 mL) of (4S)-4-[(E,1R)-1-hydroxyoctacosane-2-enyl]-2,2-dimethyl-oxazolidine-3-carboxylate (45 mg, 74.01 µmol, 1 eq) to an aqueous solution (1 mL) of trifluoroacetic acid (30.70 mg, 269.24 µmol, 0.02 mL, 3.64 eq). Stir the mixture at 85°C for 16 h. Add a saturated aqueous solution of sodium bicarbonate to the concentrated reaction solution to adjust the pH value to 8. Filter, and dry the filter cake to obtain (E,2S,3R)-2-aminotriacontyl-4-ene-1,3-diol (9 mg, 18.57 µmol, yield 25.1%, purity 96.5%), which is a white solid.

**LCMS** Rt = 2.066 min in 3 min chromatogram, 30-100 ESI calculated value C₃₀H₆₁NO₂ [M+1]⁺ 468.8, and measured value 468.5.

**¹H NMR** (400 MHz, CDCl₃) δ = 5.83 - 5.70 (m, 1H), 5.55 - 5.42 (m, 1H), 4.15 - 4.00 (m, 1H), 3.77 - 3.59 (m, 2H), 2.98 - 2.83 (m, 1H), 2.10 - 2.04 (m, 2H), 1.82 - 1.73 (m, 4H), 1.43 - 1.39 (m, 2H), 1.26 (br s, 44H), 0.91 - 0.87 (t, *J =* 6.4 Hz, 3H).

### Sphingosine (d31:1) & (d31:0)

### Synthesis route map

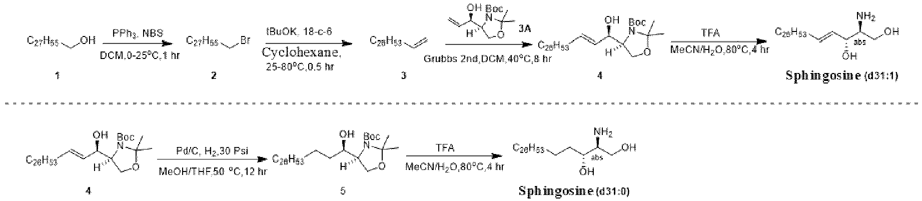

### Synthesis route:

### Step 1

### Octacosane-1-bromide

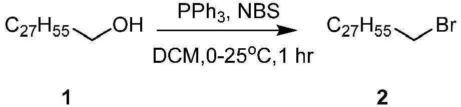

Added PPh₃ (3.83 g, 14.61 mmol) to a DCM solution (50 mL) of octacosan-1-ol (5 g, 12.17 mmol). Then, add N-bromosuccinimide (2.60 g, 14.61 mmo) to the mixture at 0°C. Stir the mixture at 25°C for 1 h. Add a saturated Na₂SO₃ solution (50 mL), and extract the aqueous phase with DCM (50 mL x 3). Wash the combined organic phases with saturated brine (100 mL), dry with anhydrous Na₂SO₄, filter, and concentrate under reduced pressure. Purify the crude product by silica gel flash column chromatography (eluent: petroleum ether) to obtain ctacosane-1-bromide (5.77 g, 12.18 mmol, yield 100.00%), which is a white solid.

**¹H NMR** (400 MHz, CDCl₃) δ = 3.42 (t, *J* = 7.6 Hz, 2H), 1.95 - 1.80 (m, 2H), 1.44 (s, 2H), 1.30 - 1.24 (m, 48H), 0.89 (t, *J* = 6.8 Hz, 3H).

### Step 2

### Octacos-1-ene

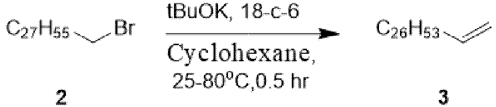

Add potassium tert-butoxide (2.71 g, 24.17 mmol) to a cyclohexane solution (5 mL) of octacosane-1-bromide (4.77 g, 10.07 mmol) and 18-crown-6 (638.85 mg, 2.42 mmol) at 25°C. Stir the mixture at 80°C for 0.5 h. Add the mixture into water (50 mL), and extract the aqueous phase with EtOAc (50 mL x 3). Wash the combined organic phases with saturated brine (100 mL), dry with anhydrous Na₂SO₄, filter, and concentrate under reduced pressure. Purify the crude product by silica gel flash column chromatography (eluent: petroleum ether) to obtain octacos-1-ene (3.15 g, 8.02 mmol, yield 79.64%), which is a white solid.

**¹H NMR** (400 MHz, CDCl₃) δ = 5.87 - 5.70 (m, 1H), 5.07 - 4.85 (m, 2H), 1.25 - 1.24 (m, 50H), 0.86 (t, *J* = 6.8 Hz, 3H).

### Step 3

### (4S)-4-[(E,1R)-1-hydroxynonacosane-2-enyl]-2,2-dimethyl-oxazolidine-3-carboxylate

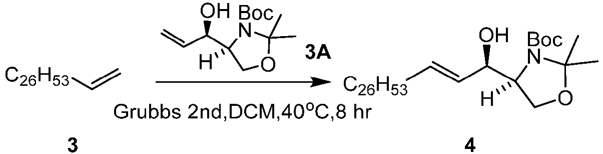

Degas a mixed liquid of tert-butyl (4S)-4-[(1R)-1-hydroxyallyl]-2,2-dimethyl-oxazolidine-3-carboxylate (800 mg, 3.11 mmo), octacos-1-ene (1.59 g, 4.04 mmol), and benzal-[1,3-bis(2,4,6-trimethylphenyl)imidazolidin-2-ylidene]-dichloro-ruthenium; and tricyclohexylphosphine (263.94 mg, 310.89 µmol) in dichloromethane (12 mL), and replace with nitrogen three times. Stir the mixture at 40°C for 8 h under nitrogen protection. Purify, by silica gel flash column chromatography (eluent: 0 to 14% ethyl acetate in petroleum ether), the crude product after concentration of the reaction solution to obtain (4S)-4-[(E,1R)-1-hydroxynonacosane-2-enyl]-2,2-dimethyl-oxazolidine-3-carboxylate (450 mg, 723.46 µmol, yield 23.27%), which is a yellow oily matter.

**¹H NMR** (400 MHz, CDCl₃) δ = 5.78 - 5.70 (m, 1H), 5.50 - 5.38 (m, 1H), 4.08 - 3.69 (m, 4H), 2.09 - 2.00 (m, 2H), 1.79 - 1.29 (m, 48H), 1.25 - 1.24 (m, 15H), 0.89 - 0.86 (m, 3H).

### Step 4

### (E,2S,3R)-2-aminohentriacontyl-4-ene-1,3-diol

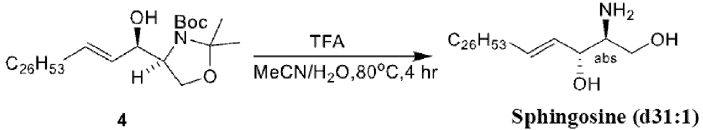

Add an aqueous solution (3 mL) of trifluoroacetic acid (824.88 mg, 7.23 mmol, 537.38 µL) to an acetonitrile solution (3 mL) of (4S)-4-[(E,1R)-1-hydroxynonacosane-2-enyl]-2,2-dimethyl-oxazolidine-3-carboxylate (450 mg, 723.46 µmol). Stir the mixture at 80°C for 4 h. Add a saturated aqueous solution of sodium bicarbonate to the reaction solution to adjust the pH value to 8. Filter, wash the filter cake three times with water (10 mL), and dry under reduced pressure to obtain (E,2S,3R)-2-aminohentriacontyl-4-ene-1,3-diol (340 mg, 705.64 µmol, yield 97.54%), which is a brown solid. Purify (170 mg, 352.82 µmol) thereof twice by prep-HPLC (chromatographic column: Welch Xltimate C4 100 x 30 x 10 µm; mobile phase: [water (formic acid)-methanol]; gradient: 70% to 90% B in 20 min) to obtain compound (E,2S,3R)-2-aminohentriacontyl-4-ene-1,3-diol (2 mg, 3.64 µmol, yield 1.03%, purity 96.127%, formate), which is a white solid.

**¹H NMR** (400 MHz, DMSO-*d*₆) δ = 8.21 (br s, 2H), 5.60 - 5.25 (m, 2H), 3.65 - 3.46 (m, 2H), 3.40 - 3.37 (m, 1H), 2.69 - 2.66 (m, 1H), 2.03 - 1.97 (m, 2H), 1.52 - 1.46 (m, 2H), 1.30 - 1.23 (m, 46H), 0.88 - 0.85 (m, 3H).

**LCMS** Rt = 6.247 min in 7 min chromatogram, 50-100AB ESI calculated value C₃₁H₆₄NO₂ [M+H]⁺ 482.5, and measured value 482.5.

**HPLC** Rt = 7.018 min in 15 min chromatography, ELSD, purity 96.127%.

### Step 5

### (4S)-4-[(1R)-1-hydroxynonacosyl]-2,2-dimethyl-oxazolidine-3-carboxylate

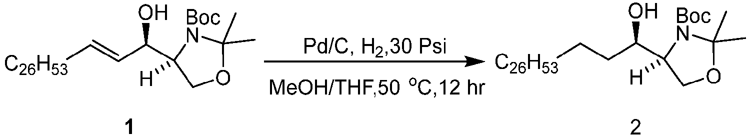

Add Pd/C (171.09 mg, 160.77 µmol, purity 10%) to a mixed solution of (4S)-4-[(E,1R)-1-hydroxynonacosane-2-enyl]-2,2-dimethyl-oxazolidine-3-carboxylate (1 g, 1.61 mmol) in methanol (5 mL) and tetrahydrofuran (5 mL) under nitrogen protection. Degas the mixture, and replace with hydrogen three times. Stir the mixture under hydrogen atmosphere (30 psi) at 50°C for 12 h. Add tetrahydrofuran (50 mL) to the reaction solution. Filter the mixture, and wash the filter cake three times with tetrahydrofuran (30 mL). Purify the crude product after concentration of the filtrate by silica gel flash column chromatography (eluent: 0 to 14% ethyl acetate in petroleum ether) to obtain compound (4S)-4-[(1R)-1-hydroxynonacosyl]-2,2-dimethyl-oxazolidine-3-carboxylate (280 mg, 448.70 µmol, yield 27.91%), which is a yellow solid.

**¹H NMR** (400 MHz, CDCl₃) δ = 4.18 - 3.72 (m, 4H), 3.67 - 3.31 (m, 1H), 1.57 (s, 9H), 1.49 (s, 6H), 1.48 - 1.13 (m, 54H), 0.88 (t, *J* =6.8 Hz,3H).

### Step 6

### (2S,3R)-2-aminohentriacontane-1,3-diol

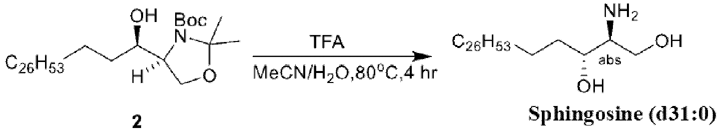

Add an aqueous solution (3 mL) of trifluoroacetic acid (255.80 mg, 2.24 mmol, 166.65 µL) to an acetonitrile solution (3 mL) of (4S)-4-[(1R)-1-hydroxynonacosyl]-2,2-dimethyl-oxazolidine-3-carboxylate (280 mg, 448.70 µmol). Stir the mixture at 80°C for 4 h. Add a saturated aqueous sodium bicarbonate solution to the reaction solution to adjust the pH to 8, and filter. Dry the filter cake under reduced pressure. At room temperature, place the filter cake in hydrochloric acid/dioxane (2 M, 10 mL), stir, and concentrate. Slurry the crude product with methanol (10 mL), and filter. Freeze dry the filter cake to obtain compound (2S,3R)-2-aminohentriacontane-1,3-diol (51 mg, 88.33 µmol, yield 19.69%, purity 90.112%, hydrochloride), which is a white solid.

**¹H NMR** (400 MHz, DMSO-*d*₆) δ = 7.83 (br s, 1.5H), 5.04 - 4.89 (m, 1H), 3.78 - 3.38 (m, 3H), 3.04 - 2.99 (m, 1H), 1.46 - 1.42 (m, 2H), 1.32 - 1.24 (m, 52H), 0.86 (t, *J* = 7.6 Hz, 3H).

**LCMS** Rt = 6.614 min in 7 min chromatogram, 50-100AB ESI calculated value C₃₁H₆₆NO₂ [M+H]⁺ 484.5, and measured value 484.5.

**HPLC** Rt = 6.672 min, in 8 min chromatography, ELSD, purity 90.112%.

### Sphingosine (d32:1) & (d32:0)

### Synthesis route map

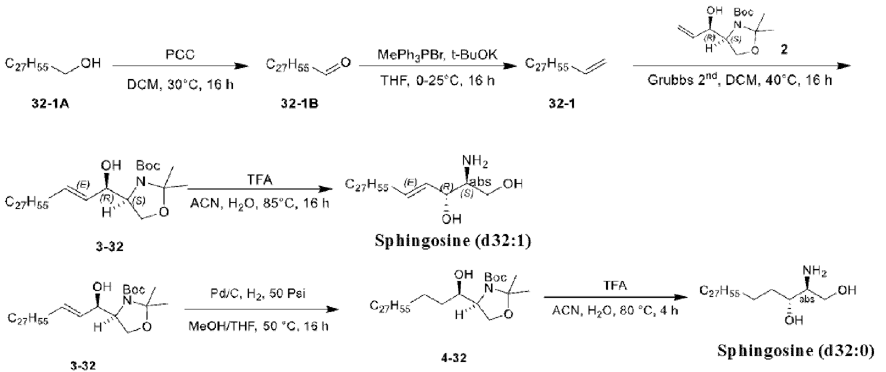

### Synthesis route:

### Step 1

### Octacosanal

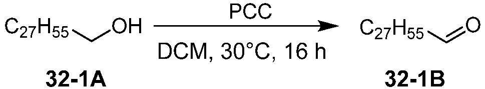

Add pyridinium chlorochromate (PCC) (6.30 g, 29.21 mmol, 1.2 eq) to a DCM solution (400 mL) of octacosan-1-ol (10 g, 24.35 mmol, 1 eq). Stir the mixture at 30°C for 16 h. Add silica gel (15 g) to the brown suspension. Stir the resulting mixture for 30 min, and purify the concentrated crude product by silica gel flash column chromatography (eluent: 0 to 10% ethyl acetate in petroleum ether) to obtain octacosanal (4.5 g, 11.01 mmol, yield 45.2%), which is a white solid.

**¹H NMR** (400 MHz, CDCl₃) δ = 9.79 - 9.76 (s, 1H), 2.46 - 2.39 (m, 2H), 1.67 - 1.61 (m, 2H), 1.31 - 1.25 (m, 48H), 0.91 - 0.87 (t, *J* = 6.4 Hz, 3H).

### Step 2

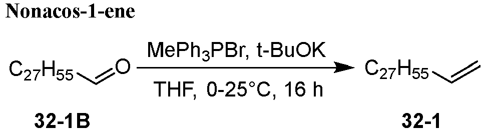

Add potassium tert-butoxide (3.71 g, 33.03 mmol, 3 eq) to a THF solution (40 mL) of methyl(triphenyl)phosphonium bromide (11.80 g, 33.03 mmol, 3 eq) at 0°C. Stir the resulting yellow suspension at 25°C for 1 h, and cool to 0°C again. Add a THF solution (50 mL) of octacosanal (4.5 g, 11.01 mmol, 1 eq) dropwise at 0°C. Stir the mixture at 25°C for 16 h. Add water (30 mL) to the mixture, and extract with ethyl acetate (40 mL x 3). Wash the combined organic phases with saturated brine (15 mL), dry with sodium sulfate, filter, and concentrate. Purify the crude product by silica gel flash column chromatography (eluent: petroleum ether) to obtain nonacos-1-ene (3.4 g, 8.36 mmol, yield 75.9%), which is a white solid.

**¹H NMR** (400 MHz, CDCl₃) δ = 5.91 - 5.75 (m, 1H), 5.07 - 4.88 (m, 2H), 2.08 - 2.01 (m, 2H), 1.40 - 1.36 (m, 2H), 1.28 - 1.25 (m, 48H), 0.91 - 0.87 (t, *J* = 6.4 Hz, 3H).

### Step 3

### (4S)-4-[(E,1R)-1-hydroxytriacontyl-2-enyl]-2,2-dimethyl-oxazolidine-3-carboxylate

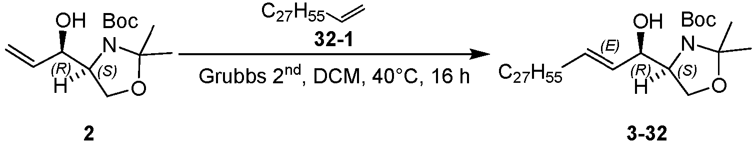

Under nitrogen protection, add benzal-[1,3-bis(2,4,6-trimethylphenyl)imidazolidine-2-methylene]-dichloro-ruthenium; tricyclohexylphosphine (149.61 mg, 176.23 µmol, 0.1 eq) to tert-butyl (4S)-4-[(1R)-1-hydroxyallyl]-2,2-dimethyl-oxazolidine-3-carboxylate (453.48 mg, 1.76 mmol, 1 eq) and nonacos-1-ene (2 g, 4.92 mmol, 2.79 eq) in dichloromethane (20 mL). Stir the mixed liquid at 40°C for 16 h. Purify, by silica gel flash column chromatography (eluent: 0 to 25% ethyl acetate in petroleum ether), the crude product after concentration of the reaction solution under reduced pressure to obtain (4S)-4-[(E,1R)-1-hydroxytriacontyl-2-enyl]-2,2-dimethyl-oxazolidine-3-carboxylate (0.3 g, 471.67 µmol, yield 26.8%), which is a brown solid.

**¹H NMR** (400 MHz, CDCl₃) δ = 5.79 - 5.68 (m, 1H), 5.48 - 5.41 (m, 1H), 4.10 - 3.76 (m, 4H), 2.05 - 2.01 (m, 2H), 1.54 - 1.49 (m, 15H), 1.28 - 1.24 (m, 50H), 0.91 - 0.86 (t, *J* = 6.4 Hz, 3H).

### Step 4

### (E,2S,3R)-2-aminodotriacontyl-4-ene-1,3-diol

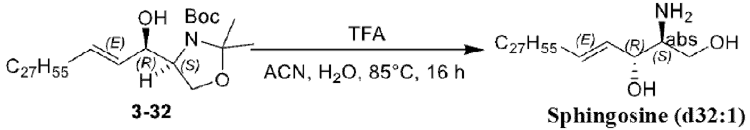

Add an acetonitrile solution (3 mL) of (4S)-4-[(E,1R)-1-hydroxytriacontyl-2-enyl]-2,2-dimethyl-oxazolidine-3-carboxylate (0.3 g, 471.67 µmol, 1 eq) to an aqueous solution (3 mL) of trifluoroacetic acid (92.10 mg, 807.73 µmol, 60.00 µL, 1.71 eq). Stir the mixture at 85°C for 16 h. Add a saturated aqueous solution of sodium bicarbonate to the concentrated reaction solution to adjust the pH value to 8. Filter, and dry the filter cake under reduced pressure to obtain (E,2S,3R)-2-aminodotriacontyl-4-ene-1,3-diol (110 mg, 216.29 µmol, yield 45.9%, purity 97.5%), which is a white solid.

**LCMS** Rt = 2.137 min in 3 min chromatogram, 30-100AB ESI calculated value C₃₂H₆₅NO₂ [M+1]⁺ 496.8, and measured value 496.5.

**¹H NMR** (400 MHz, CDCl₃) δ = 5.82 - 5.70 (m, 1H), 5.53 - 5.42 (m, 1H), 4.14 - 3.97 (s, 1H), 3.74 - 3.58 (m, 2H), 2.96 - 2.82 (s, 1H), 2.10 - 2.03 (m, 2H), 1.91 - 1.70 (m, 4H), 1.41 - 1.36 (m, 2H), 1.29 - 1.24 (m, 48H), 0.91 - 0.86 (m, 3H).

### Step 5

### Tert-butyl (4S)-4-[(1R)-1-hydroxytriacontyl]-2,2-dimethyl-oxazolidine-3-carboxylate

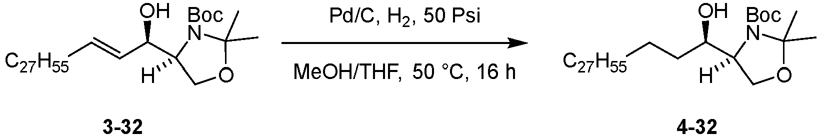

Under nitrogen protection, add Pd/C (50 mg, purity 10%) to a mixed solution of tert-butyl (4S)-4-[(E,1R)-1-hydroxytriacontyl-2-enyl]-2,2-dimethyl-oxazolidine-3-3-carboxylate (300 mg, 471.67 µmol) in tetrahydrofuran (5 mL) and methanol (5 mL), degas the suspension, replace with hydrogen three times, and then stir at 50°C under hydrogen atmosphere (50 psi) for 16 hr. Filter, and wash the filter cake three times with tetrahydrofuran (50 mL). Concentrate the filtrate to obtain tert-butyl (4S)-4-[(1R)-1-hydroxytriacontyl]-2,2-dimethyl-oxazolidine-3-carboxylate (250 mg, 391.81 µmol), which is a brown solid.

**¹H NMR** (400 MHz, CDCl₃) δ = 4.15-3.67 (m, 4H), 1.56 (s, 6H), 1.43 (s, 9H), 1.38 - 1.15 (m, 56H), 0.88 (t, *J* = 6.8 Hz, 3H).

### Step 6

### (2S,3R)-2-aminodotriacontane-1,3-diol

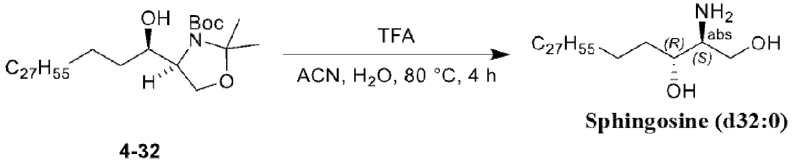

Add trifluoroacetic acid (307.00 mg, 2.69 mmol, 0.2 mL) to mixed solvents of an acetonitrile solution (2 ml) of tert-butyl(4S)-4-[(1R)-1-hydroxytriacontyl]-2,2-dimethyl-oxazolidine-3-carboxylate (250 mg, 391.81 µmol) and water (2 ml). Stir the mixture at 80°C for 4 h. Add a saturated aqueous solution of sodium bicarbonate to the reaction solution to adjust the pH value to 8. Filter in batches, and rinse the filter cake with methanol (5 mL). Disperse the filter cake in hydrochloric acid/dioxane (2 M, 5 mL), stir, and then concentrate. Slurry the crude product with methanol (10 mL), and filter. Freeze dry the filter cake to obtain compound (2S,3R)-2-aminodotriacontane-1,3-diol (44.7 mg, 83.65 umol, yield 21.35%, hydrochloride) in the form of a white solid.

**MS** Rt = 0.497 min in 0.8 min chromatogram, 30-100 AB, ESI calculated value C₃₂H₆₈NO₂ [M+H]⁺ 498.5, and measured value 498.6.

**¹H NMR** (400 MHz, DMSO-*d₆*) δ = 7.76 (s, 3H), 4.90 (s, 2H), 3.74 - 3.64 (m, 2H), 3.63 - 3.53 (m, 1H), 3.10 -3.00 (m, 1H), 1.48 - 1.35 (m, 2H), 1.41 - 1.15 (m, 54H), 0.87 (t, *J* = 6.8 Hz, 3H).

### Sphingosine (d33:1) & (d33:0)

### Synthesis route map

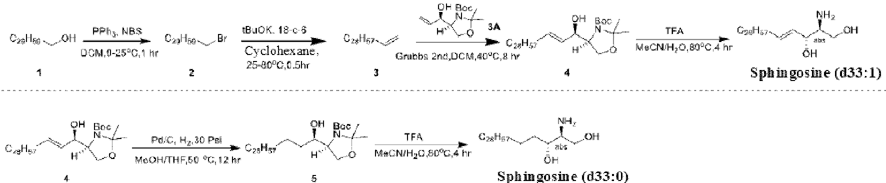

### Synthesis route:

### Step 1

### Triacontane-1-bromide

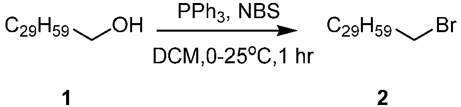

Add triphenylphosphine (2.15 g, 8.20 mmol) to a DCM solution (50 mL) of triacontan-1-ol (3 g, 6.84 mmol). Then, add N-bromosuccinimide (1.46 g, 8.20 mmol) to the mixture at 0°C. Stir the mixture at 25°C for 1 h. Add a saturated Na₂SO₃ solution (50 mL), and extract the aqueous phase with DCM (50 mL x 3). Wash the combined organic phases with saturated brine (100 mL), dry with anhydrous Na₂SO₄, filter, and concentrate under reduced pressure. Purify the crude product by silica gel flash column chromatography (eluent: petroleum ether) to obtain triacontane-1-bromide (3.43 g, 6.84 mmol, yield 100.00%), which is a white solid.

**¹H NMR** (400 MHz, CDCl₃) δ = 3.42 (t, *J* = 6.8 Hz, 2H), 1.86 (q, *J =* 7.2 Hz, 2H), 1.47 - 1.15 (m, 54H), 0.89 (t, *J =* 6.8 Hz, 3H).

### Step 2

### Triacont-1-ene

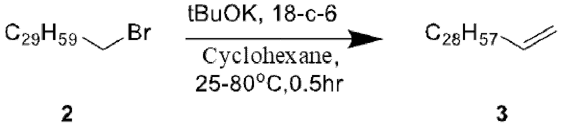

Add potassium tert-butoxide (1.30 g, 11.62 mmol) to a cyclohexane solution (5 mL) of triacontane-1-bromide (2.43 g, 4.84 mmol) and 18-crown-6 (307.25 mg, 1.16 mmol) at 25°C. Stir the mixture at 80°C for 0.5 h. Add the mixture into water (50 mL), and extract the aqueous phase with EtOAc (50 mL x 3). Wash the combined organic phases with saturated brine (100 mL), dry with anhydrous Na₂SO₄, filter, and concentrate under reduced pressure. Purify the crude product by silica gel flash column chromatography (eluent: petroleum ether) to obtain triacont-1-ene (1.35 g, 3.21 mmol, yield 66.24%), which is a white solid.

**¹H NMR** (400 MHz, CDCl₃) δ = 5.95 - 5.68 (m, 1H), 5.02 - 4.83 (m, 2H), 1.26 - 1.24 (m, 54H), 0.86 (t, *J* = 6.8 Hz, 3H).

### Step 3

### (4S)-4-[(E,1R)-1-hydroxyhentriacontyl-2-enyl]-2,2-dimethyl-oxazolidine-3-carboxylate

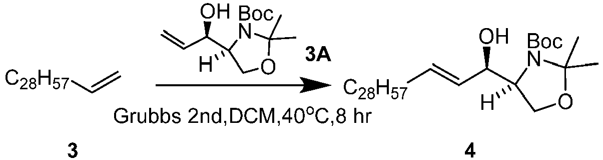

Degas a mixed liquid of tert-butyl (4S)-4-[(1R)-1-hydroxyallyl]-2,2-dimethyl-oxazolidine-3-carboxylate (800 mg, 3.11 mmol), triacont-1-ene (1.70 g, 4.04 mmol), and benzal-[1,3-bis(2,4,6-trimethylphenyl)imidazolidin-2-ylidene]-dichloro-ruthenium; and tricyclohexylphosphine (263.94 mg, 310.89 µmol) in dichloromethane (12 mL), and replace with nitrogen three times. Stir the mixture at 40°C for 8 h under nitrogen protection. Purify, by silica gel flash column chromatography (eluent: 0 to 14% ethyl acetate in petroleum ether), the crude product after concentration of the reaction solution to obtain (4S)-4-[(E,1R)-1-hydroxyhentriacontyl-2-enyl]-2,2-dimethyl-oxazolidine-3-carboxylate (260 mg, 399.96 µmol, yield 12.86%), which is a yellow liquid.

**¹H NMR** (400 MHz, CDCl₃) δ = 5.78 - 5.55 (m, 1H), 5.47 - 5.23 (m, 1H), 4.09 - 3.75 (m, 4H), 2.02 - 1.87 (m, 2H), 1.75 - 1.20 (m, 52H), 1.18 - 1.17 (m, 15H), 0.83 - 0.79 (m, 3H).

### Step 4

### (E,2S,3R)-2-aminotritriacontyl-4-ene-1,3-diol

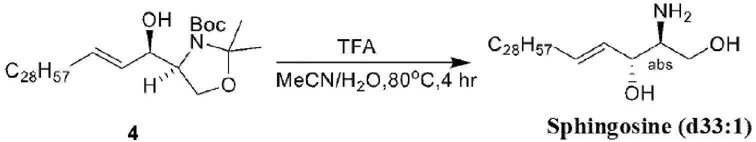

Add an aqueous solution (3 mL) of trifluoroacetic acid (420.95 mg, 3.69 mmol, 274.24 µL) to an acetonitrile solution (3 mL) of (4S)-4-[(E,1R)-1-hydroxyhentriacontyl-2-enyl]-2,2-dimethyl-oxazolidine-3-carboxylate (240 mg, 369.19 µmol). Stir the mixture at 80°C for 4 h. Add a saturated aqueous solution of sodium bicarbonate to the reaction solution to adjust the pH value to 8. Filter, wash the filter cake three times with water (10 mL), and dry under reduced pressure to obtain (E,2S,3R)-2-aminotritriacontyl-4-ene-1,3-diol (180 mg, 353.02 µmol, yield 95.62%). Purify (90 mg, 176.51 µmol) thereof by prep-HPLC (chromatographic column: Welch Xltimate C4 100 x 30 x 10 µm; mobile phase: [water (formic acid)-methanol]; gradient: 70% to 90% B in 20 min) to obtain compound (E,2S,3R)-2-aminotritriacontyl-4-ene-1,3-diol (7.8 mg, 14.03 µmol, yield 7.95%, formate), which is a white solid.

**¹H NMR** (400 MHz, DMSO-*d*₆) δ = 8.27 (br s, 1H), 5.66 - 5.55 (m, 1H), 5.50 - 5.40 (m, 1H), 3.93 - 3.85 (m, 1H), 3.48 - 3.47 (m, 2H), 2.71 - 2.65 (m, 1H), 2.04 - 1.95 (m, 2H), 1.36 - 1.31 (m, 2H), 1.30 - 1.21 (m, 50H), 0.89 - 0.83 (m, 3H).

**LCMS** Rt = 1.336 min in 1.5 min chromatogram, 5-95AB ESI calculated value C₃₃H₆₈NO₂ [M+H]⁺ 510.5, and measured value 510.4.

**HPLC** Rt = 7.948 min in 15 min chromatography, ELSD, purity 98.751%.

### Step 5

### (4S)-4-[(1R)-1-hydroxyhentriacontyl]-2,2-dimethyl-oxazolidine-3-carboxylate

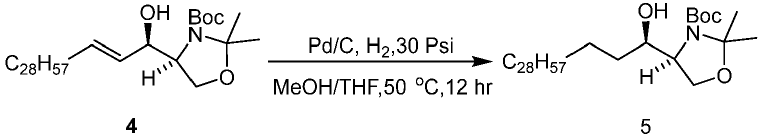

Add Pd/C (45.84 mg, 43.07 µmol, purity 10%) to a mixed solution of (4S)-4-[(E,1R)-1-hydroxyhentriacontyl-2-enyl]-2,2-dimethyl-oxazolidine-3-carboxylate (280 mg, 430.72 µmol) in methanol (5 mL) and tetrahydrofuran (5 mL) under nitrogen protection. Degas the mixture, and replace with hydrogen three times. Stir the mixture under hydrogen atmosphere (30 psi) at 50°C for 12 h. Add tetrahydrofuran (50 mL) to the reaction solution. Filter the mixture, and wash the filter cake three times with tetrahydrofuran (30 mL). Purify the crude product after concentration of the filtrate by silica gel flash column chromatography (eluent: 0 to 14% ethyl acetate in petroleum ether) to obtain compound (4S)-4-[(1R)-1-hydroxyhentriacontyl]-2,2-dimethyl-oxazolidine-3-carboxylate (240 mg, 368.05 µmol, yield 85.45%), which is a yellow oily matter.

**¹H NMR** (400 MHz, CDCl₃) δ = 4.06 - 3.85 (m, 4H), 3.74 - 3.49 (m, 1H), 1.59 (s, 6H), 1.49 (s, 9H), 1.48 - 1.13 (m, 58H), 0.88 (t, *J* =6.8 Hz,3H).

### Step 6

### (2S,3R)-2-aminotritriacontane-1,3-diol

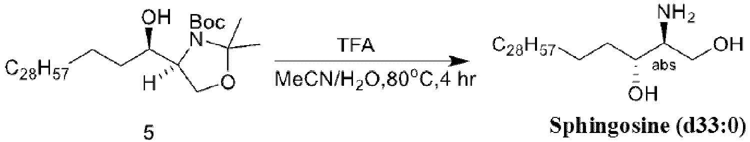

Add an aqueous solution (3 mL) of trifluoroacetic acid (209.83 mg, 1.84 mmol, 136.69 µL) to an acetonitrile solution (3 mL) of (4S)-4-[(1R)-1-hydroxyhentriacontyl]-2,2-dimethyl-oxazolidine-3-carboxylate (240 mg, 368.05 µmol). Stir the mixture at 80°C for 4 h. Add a saturated aqueous sodium bicarbonate solution to the reaction solution to adjust the pH to 8, and filter. Dry the filter cake under reduced pressure. At room temperature, place the filter cake in hydrochloric acid/dioxane (4 M, 10 mL), stir, and concentrate. Slurry the crude product with methanol (10 mL), and filter. Freeze dry the filter cake to obtain compound (2S,3R)-2-aminotritriacontane-1,3-diol (226.3 mg, 349.87 µmol, yield 95.06%, purity 84.780%, hydrochloride), which is a white solid.

**¹H NMR** (400 MHz, DMSO-*d*₆) δ = 7.89 (br s, 2H), 4.98 (br s, 1H), 3.73 - 3.56 (m, 2H), 3.54 - 3.40 (m, 1H), 3.04 - 2.99 (m, 1H), 1.45 - 1.41 (m, 2H), 1.34 - 1.21 (m, 56H), 0.87 (t, *J=* 6.4 Hz, 3H).

**HPLC** Rt = 7.079 min, in 8 min chromatography, ELSD, purity 84.780%.

**MS** Rt = 2.97 -3.14 min in 4 min chromatogram, 50-100AB ESI calculated value C₃₃H₇₀NO₂ [M+H]⁺ 512.5328, and measured value 512.5456.

### Sphingosine (d34:0) & (d34:1)

### Synthesis route map:

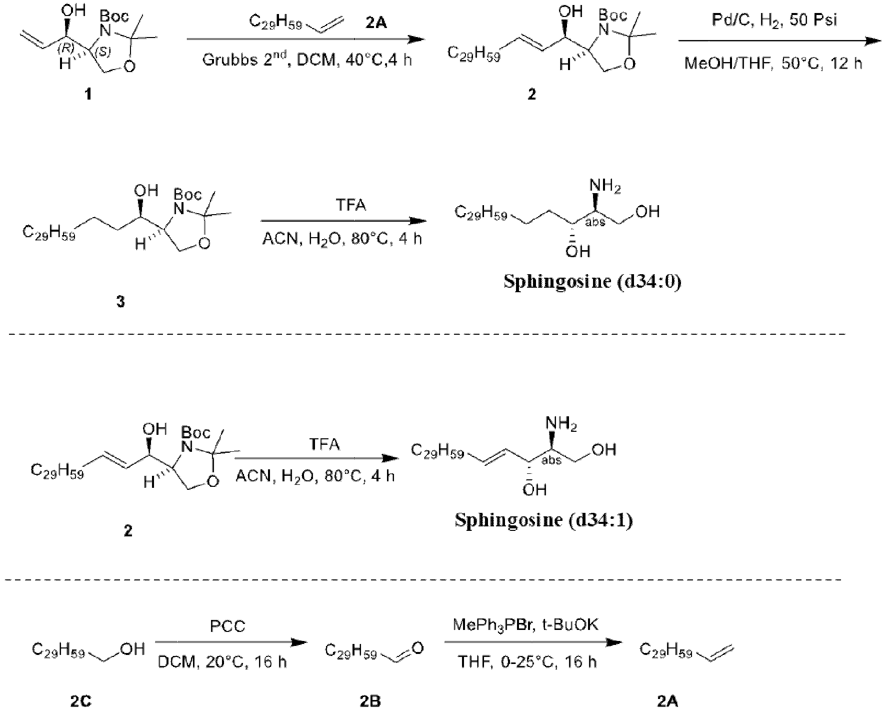

### Synthesis route:

### Step 1

### (4S)-4-[(E,1R)-1-hydroxydotriacontyl-2-enyl]-2,2-dimethyl-oxazolidine-3-carboxylate

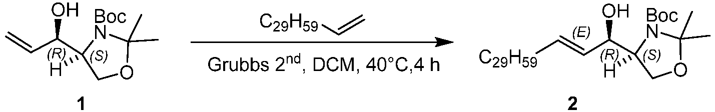

Degas a mixed liquid of tert-butyl (4S)-4-[(1R)-1-hydroxyallyl]-2,2-dimethyl-oxazolidine-3-carboxylate (800 mg, 3.11 mmol), hentricont-1-ene (1.2 g, 2.76 mmol), and benzal-[1,3-bis(2,4,6-trimethylphenyl)imidazolidin-2-ylidene]-dichloro-ruthenium; and tricyclohexylphosphine (234.30 mg, 275.98 µmol) in dichloromethane (12 mL), and replace with nitrogen three times. Stir the mixed liquid at 40°C for 4 h. Filter the reaction solution, and purify, by silica gel flash column chromatography (eluent: 20% ethyl acetate in petroleum ether), the crude product after concentration of the filtrate to obtain (4S)-4-[(E,1R)-1-hydroxydotriacontyl-2-enyl]-2,2-dimethyl-oxazolidine-3-carboxylate (1 g, 1.51 mmol, yield 54.56%), which is a brown oily matter.

**¹H NMR** (400 MHz, CDCl₃) δ = 5.80 - 5.70 (m, 1H), 5.55 - 5.40 (m, 1H), 4.10-3.75 (m, 4H), 2.07 (s, 2H), 1.51 (s, 15H), 1.27 (s, 54H), 0.95 - 0.85 (m, 3H).

### Step 2

### (4S)-4-[(1R)-1-hydroxydotriacontyl]-2,2-dimethyl-oxazolidine-3-carboxylate

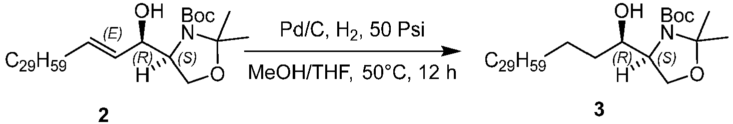

Add Pd/C (800 mg, 751.40 µmol, purity 10%) to a mixed solution of (4S)-4-[(E,1R)-1-hydroxydotriacontyl-2-enyl]-2,2-dimethyl-oxazolidine-3-carboxylate (1 g, 1.51 mmol) in methanol (10 mL) and tetrahydrofuran (10 mL) under nitrogen protection. Degas the mixture, and replace with hydrogen three times. Stir the mixture under hydrogen atmosphere (50 psi) at 50°C for 16 h. Add tetrahydrofuran (10 mL) to the reaction solution. Filter the mixture, and wash the filter cake three times with tetrahydrofuran (10 mL). Purify the crude product after concentration of the filtrate by silica gel flash column chromatography (eluent: 20% ethyl acetate in petroleum ether) to obtain compound (4S)-4-[(1R)-1-hydroxydotriacontyl]-2,2-dimethyl-oxazolidine-3-carboxylate (460 mg, 690.58 µmol, yield 45.86%), which is a white solid.

**¹H NMR** (400 MHz, CDCl₃) δ = 4.05-3.82 (m, 4H), 1.67 (s, 15H), 1.28 (s, 60H), 0.95-0.84 (m, 3H).

### Step 3

### (2S,3R)-2-aminotetratriacontane-1,3-diol

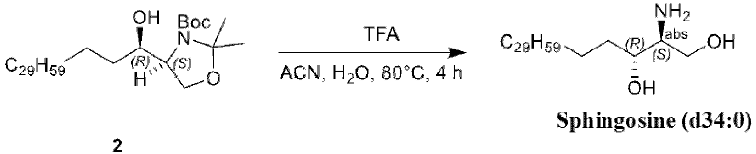

Add an aqueous solution (3 mL) of trifluoroacetic acid (393.70 mg, 3.45 mmol, 256.48 µL) to an acetonitrile solution (3 mL) of (4S)-4-[(1R)-1-hydroxydotriacontyl]-2,2-dimethyl-oxazolidine-3-carboxylate (460 mg, 690.58 µmol). Stir the mixture at 80°C for 4 h. Add a saturated aqueous sodium bicarbonate solution to the reaction solution to adjust the pH to 8, and filter. Dry the filter cake under reduced pressure. At room temperature, place the filter cake in hydrochloric acid/dioxane (2 M, 10 mL), stir for 10 min, and concentrate. Slurry the crude product with methanol (10 mL), and filter. Freeze dry the filter cake to obtain compound (2S,3R)-2-aminotetratriacontane-1,3-diol (377.4 mg, 671.06 µmol, yield 97.17%, hydrochloride), which is a white solid.
**MS** Rt = 1.06-1.43 min in 4 min Chromatography, 80-100 AB, ESI calculated value C₃₄H₇₂NO₂ [M+H]⁺ 526.5, and measured value 526.6
**¹H NMR** (400 MHz, DMSO-*d*⁶) δ = 8.10 - 7.90 (m, 3H), 5.10 - 4.85 (m, 1H), 3.75 - 3.65 (m, 2H), 3.65 - 3.55 (m, 1H), 3.04 -3.00 (m, 1H), 1.45 - 1.35 (m, 2H), 1.27 (s, 60H), 0.90 -0.80 (m, 3H).

### Step 4

### (E,2S,3R)-2-aminotetratriacontyl-4-ene-1,3-diol

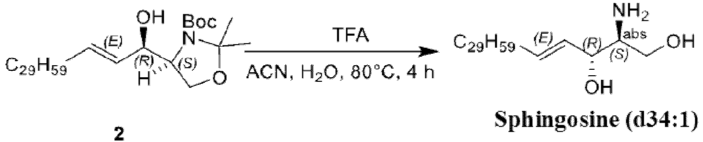

Add an aqueous solution (4 mL) of trifluoroacetic acid (824.12 mg, 7.23 mmol, 536.89 µL) to an acetonitrile solution (4 mL) of (4S)-4-[(E,1R)-1-hydroxydotriacontyl-2-enyl]-2,2-dimethyl-oxazolidine-3-carboxylate (480 mg, 722.79). Stir the mixture at 80°C for 4 h. Add a saturated aqueous sodium bicarbonate solution to the reaction solution to adjust the pH to 8, and filter. Dry the filter cake under reduced pressure to obtain (E,2S,3R)-2-aminotetratriacontyl-4-ene-1,3-diol (300 mg, 572.61 µmol, yield 79.22%). Purify the stock solution by prep-HPLC (chromatographic column: Welch Xltimate C4 100 x 30 x 10 µm; mobile phase: [water (formic acid)-methanol]; gradient: 70% to 90% B in 20 min) to obtain compound (E,2S,3R)-2-aminotetratriacontyl-4-ene-1,3-diol (8.2 mg, 14.39 µmol, yield 1.99%, formate), which is a white solid.

**LCMS** Rt = 1.342 min in 1.5 min chromatogram, 5-95AB ESI calculated value C₃₄H₇₀NO₂ [M+H]⁺ 524.5, and measured value 525.4.

**¹H NMR** (400 MHz, DMSO-*d*⁶) δ = 8.35 (s, 2H), 5.65 - 5.40 (m, 3H), 5.40 - 5.30 (m, 1H), 3.90 - 3.87 (m, 1H), 3.50 - 3.46 (m, 1H), 3.35 - 3.25 (m, 1H), 2.70 - 2.66 (m, 1H), 2.05 - 1.95 (m, 2H), 1.27 (s, 54H), 0.90-0.80 (m, 3H).

### Step 5

### Triacontanal

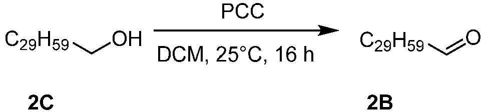

Add pyridinium chlorochromate (PCC) (1.08 g, 5.01 mmol) and silica gel (3.00 g, 49.93 mmol) to a DCM solution (100 mL) of triacontan-1-ol (2 g, 4.56 mmol). Stir the mixture at 25°C for 16 h. Filter the mixture, and purify the crude product after concentration of the filtrate by silica gel flash chromatography (eluent: 0 to 30% ethyl acetate in petroleum ether) to obtain triacontanal (1 g, 2.29 mmol, yield 50.23%), which is a brown solid.

**¹H NMR** (400 MHz, CDCl₃) δ = 9.85 - 9.75 (m, 1H), 2.50 - 2.35 (m, 2H), 1.70 - 1.60 (m, 2H), 1.30 - 1.20 (m, 52H), 0.95 - 0.85 (m, 3H).

### Step 6

### Hentriacont-1-ene

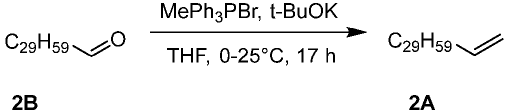

At 0°C, add potassium tert-butoxide (1.85 g, 16.48 mmol) to a THF solution (50 mL) of methyl(triphenyl)phosphonium bromide (5.89 g, 16.48 mmol). Stir the resulting yellow suspension at 25°C for 1 h, and cool to 0°C again. Add dropwise a THF solution (25 mL) of triacontanal (2.4 g, 5.49 mmol) at 0°C. Stir the mixture at 25°C for 16 h. Combine the reaction solution with another batch prepared from triacontanal (1 g). Add water (50 mL) to the mixture, and extract with ethyl acetate (50 mL x 3). Wash the combined organic phases with saturated brine (10 mL), dry with sodium sulfate, filter, and concentrate. Purify the crude product by flash silica gel chromatography (eluent: petroleum ether) to obtain hentriacont-1-ene (1.1 g, 2.53 mmol, yield 32.42%), which is a colorless oily matter.

**¹H NMR** (400 MHz, CDCl₃) δ = 5.90 - 5.80 (m, 1H), 5.05 - 4.93 (m, 2H), 2.09 - 2.02 (m, 2H), 1.28 (s, 54H), 0.91 (t, *J =* 6.8 Hz, 3H).

### Sphingosine (d35:1) & (d35:0)

### Synthesis route map:

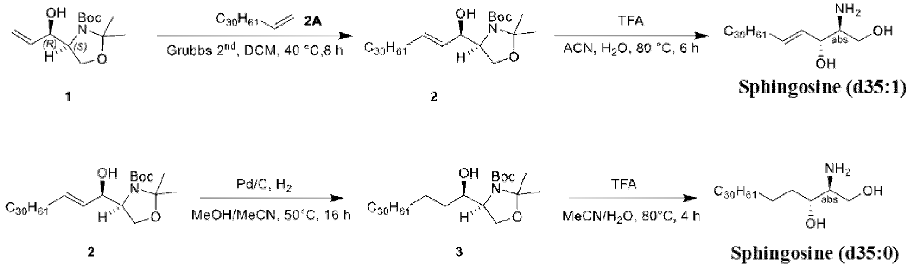

### Synthesis route:

### Step 1

### Tert-butyl (4S)-4-[(E,1R)-1-hydroxytritriacontyl-2-enyl]-2,2-dimethyl-oxazolidine-3-carboxylate

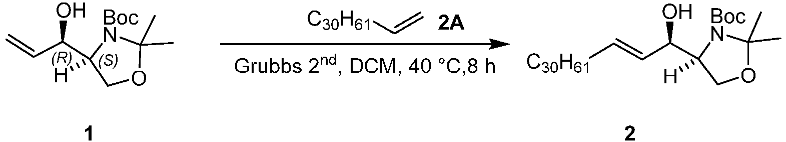

Add Grubbs second-generation catalyst benzyl-[1,3-bis(2,4,6-trimethylphenyl)imidazolidine-2-acyl]-dichlororuthenium-tricyclohexylphosphine (164.96 mg, 194.31 umol) to a mixed liquid of tert-butyl (4S)-4-[(1R)-1-hydroxyalkenyl]-2,2-dimethyl-oxazolidine-3-carboxylate (500 mg, 1.94 mmol) and dotriacont-1-ene (1.13 g, 2.53 mmol) in dichloromethane (7.5 mL), degas the mixed liquid, replace with nitrogen three times, and then stir at 40°C under nitrogen atmosphere for 8 h. Purify, by silica gel flash column chromatography (eluent: 0 to 15% ethyl acetate in petroleum ether), the crude product after concentration of the reaction solution to obtain tert-butyl (4S)-4-[(E,1R)-1-hydroxytritriacontyl-2-enyl]-2,2-dimethyl-oxazolidine-3-carboxylate (90.0 mg, 132.72 umol, yield 6.83%), which is a white solid.

**¹H NMR** (400 MHz, CDCl₃) δ = 5.78 - 5.68 (m, 1H), 5.48 - 5.35 (m, 1H), 4.24 - 3.76 (m, 4H), 2.07 - 1.98 (m, 2H), 1.55 - 1.47 (m, 15H), 1.40 - 1.11 (m, 56H), 0.88 (t, *J =* 6.8 Hz, 3H).

### Step 2

### (E,2S,3R)-2-aminopentatriacontyl-4-ene-1,3-diol

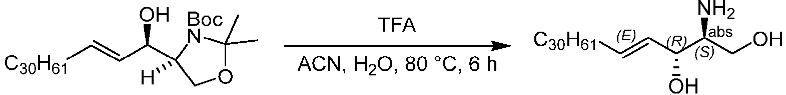

Add trifluoroacetic acid (307 mg, 2.69 mmol, 0.2 mL) to a mixed liquid of tert-butyl (4S)-4-[(E,1R)-1-hydroxytritriacontyl-2-enyl]-2,2-dimethyl-oxazolidine-3-carboxylate (90.0 mg, 132.7 µmol) in acetonitrile (1.5 mL) and water (1.5 mL), and stir the mixed liquid at 80°C for 6 h. Add a saturated aqueous solution of sodium bicarbonate to the reaction solution to adjust the pH value to 8. Filter in batches, and rinse the filter cake with methanol (5 mL). Disperse the filter cake in hydrochloric acid/dioxane (2 M, 5 mL), and stir for 20 min. Slurry the concentrated crude product with methanol (3 mL), and filter. Freeze dry the filter cake to obtain compound (E,2S,3R)-2-aminopentatriacontyl-4-ene-1,3-diol (15.0 mg, 26.11 umol, yield 19.68%, hydrochloride), which is a white solid.

**MS** Rt = 0.519 min in 0.8 min chromatogram, 30-100 AB, ESI calculated value C₃₅H₇₂NO₂ [M+H]⁺ 538.5, and measured value 538.5.

**¹H NMR** (400 MHz, DMSO-*d₆*) δ = 7.81 - 7.39 (m, 3H), 5.79 - 5.66 (m, 1H), 5.51 - 5.41 (m, 1H), 5.24 - 5.14 (m, 1H), 4.93 - 4.81 (m, 1H), 4.26 - 4.16 (m, 1H), 3.69 - 3.59 (m, 1H), 3.58 - 3.47 (m, 1H), 3.58 - 3.47 (m, 1H), 3.18 - 3.07 (m, 1H), 2.07 - 1.99 (m, 2H), 1.42 - 1.32 (m, 2H), 1.40 - 1.15 (m, 56H), 0.87 (t, *J =* 6.8 Hz, 3H).

### Step 3

### Tert-butyl (4S)-4-[(1R)-1-hydroxytritriacontyl]-2,2-dimethyl-oxazolidine-3-carboxylate

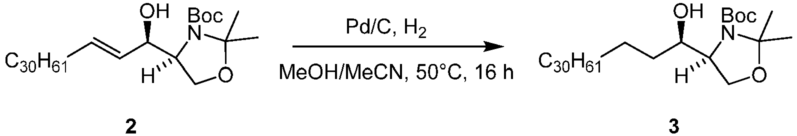

Under nitrogen protection, add Pd/C (117.70 mg, 110.60 µmol, purity 10%) to a mixed solution of tert-butyl (4S)-4-[(E,1R)-1-hydroxytritriacontyl-2-enyl]-2,2-dimethyl-oxazolidine-3-3-carboxylate (150 mg, 221.20 µmol) in tetrahydrofuran (2 mL) and methanol (2 mL), degas the suspension, replace with hydrogen three times, and then stir at 50°C under hydrogen atmosphere (50 psi) for 16 hr. Add tetrahydrofuran (10 mL). Filter, and wash the filter cake three times with tetrahydrofuran (10 mL). Purify, by silica gel flash column chromatography (eluent: 20% ethyl acetate in petroleum ether), the crude product after concentration of the filtrate to obtain tert-butyl (4S)-4-[(1R)-1-hydroxytritriacontyl-2-enyl]-2,2-dimethyl-oxazolidine-3-carboxylate (110 mg, 161.73 µmol, yield 73.12%), which is a white solid.

**¹H NMR** (400 MHz, CDCl₃) δ = 4.14 - 3.67 (m, 4H), 1.64 - 1.58 (m, 15H), 1.36 - 1.12 (m, 62H), 0.88 (t, *J =* 6.8 Hz, 3H).

### Step 4

### (2S,3R)-2-aminopentatriacontane-1,3-diol

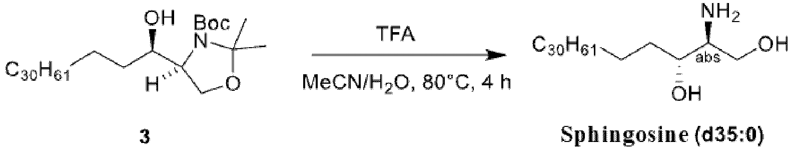

Add an aqueous solution (2 mL) of trifluoroacetic acid (92.20 mg, 808.66 µmol, 60.07 µL) to an acetonitrile solution (2 mL) of tert-butyl (4S)-4-[(1R)-1-hydroxytritriacontyl]-2,2-dimethyl-oxazolidine-3-carboxylate (110 mg, 161.73 µmol). Stir the mixture at 80°C for 4 h. Add a saturated aqueous solution of sodium bicarbonate to the reaction solution to adjust the pH value to 8. Filter the mixture in batches, and concentrate the filter cake under reduced pressure. Disperse the filter cake in hydrochloric acid/dioxane (2 M, 10 mL) at room temperature, and then concentrate. Slurry the crude product with methanol (10 mL), and filter. Freeze dry the filter cake to obtain compound (2S,3R)-2-aminopentatriacontane-1,3-diol (35.4 mg, 61.41 µmol, yield 37.97%, hydrochloride), which is a white solid.

**MS** Rt = 2.158 min in 3 min chromatography, ESI calculated value C₃₅H₇₄NO₂ [M+H]⁺ 540.6, and measured value 540.6.

**¹H NMR** (400 MHz, DMSO-*d₆*) δ = 7.23 - 6.74 (m, 3H), 4.39 - 4.19 (m, 2H), 3.16 - 3.06 (m, 2H), 2.95 - 2.88 (m, 1H), 2.70 - 2.64 (m, 1H), 0.86 - 0.77 (m, 4H), 0.69 - 0.66 (m, 58H), 0.30 - 0.24 (m, 3H).

### Sphingosine (d36:1) & (d36:0)

### Synthesis route map:

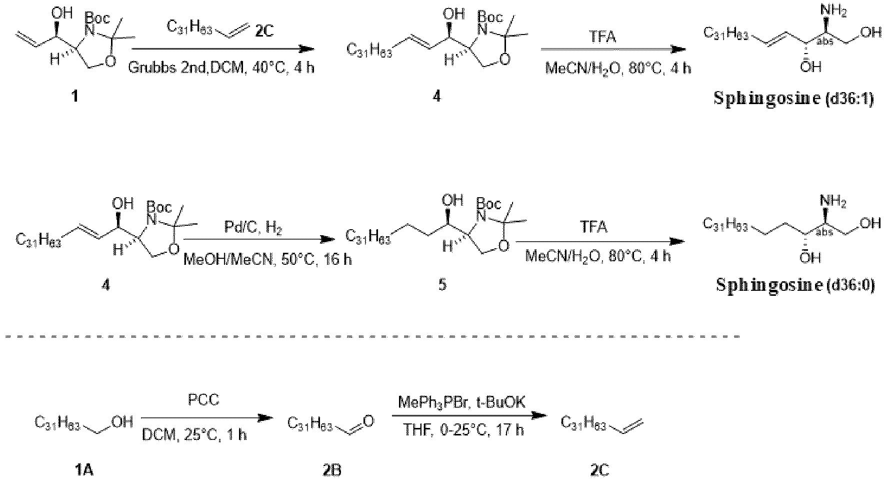

### Synthesis route:

### Step 1

### Dotriacontanal

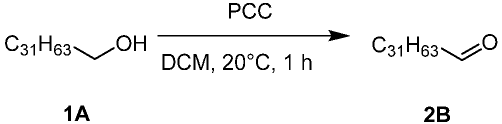

Add pyridinium chlorochromate (PCC) (10.16 g, 47.12 mmol) and silica gel (20.00 g, 332.87 mmol) to a DCM solution (1 L) of dotriacont-1-ol (20 g, 42.84 mmol). Stir the mixture at 25°C for 1 h. Combine the reaction solution with another batch of the reaction solution (prepared from 20 g of 1A). Filter, and concentrate the filtrate to obtain dotriacontanal (14 g, 30.12 mmol, yield 35.15%), which is a white solid.

**¹H NMR** (400 MHz, CDCl₃) δ = 9.79 (s, 1H), 2.45 - 2.38 (m, 2H), 2.03 - 1.96 (m, 2H), 1.27 - 1.30 (m, 56H), 0.88 (t, *J =* 6.8 Hz, 3H).

### Step 2

### Dotriacont-1-ene

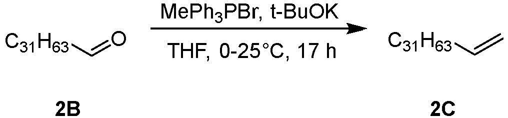

At 0°C, add potassium tert-butoxide (2.17 g, 19.36 mmol) to a THF solution (50 mL) of methyl(triphenyl)phosphonium bromide (6.92 g, 19.36 mmol). Stir the resulting yellow suspension at 25°C for 1 h, and cool to 0°C again. Add dropwise a THF solution (25 mL) of dotriacontanal (3 g, 6.45 mmol) at 0°C. Stir the mixture at 25°C for 16 h. Add water (50 mL) to the mixture, and extract with ethyl acetate (50 mL x 3). Wash the combined organic phases with saturated brine (50 mL), dry over anhydrous sodium sulfate, filter, and concentrate. Purify the crude product by silica gel flash column chromatography (eluent: petroleum ether) to obtain dotriacont-1-ene (880 mg, 1.90 mmol, yield 29.46%), which is a white solid.

**¹H NMR** (400 MHz, CDCl₃) δ = 5.85 - 5.77 (m, 1H), 5.04 - 4.92 (m, 1H), 4.90 - 4.86 (m, 1H), 2.09 - 1.95 (m, 2H), 1.31 - 1.25 (m, 58H), 0.88 (t, *J =* 6.8 Hz, 3H).

### Step 3

### Tert-butyl (4S)-4-[(E,1R)-1-hydroxytetratriacontyl-2-enyl]-2,2-dimethyl-oxazolidine-3-carboxylate

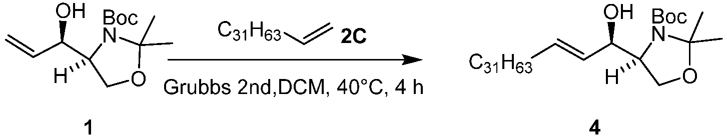

Under nitrogen atmosphere, add benzal-[1,3-bis(2,4,6-trimethylphenyl)imidazolidine-2-methylene]-dichloro-ruthenium; tricyclohexylphosphine (159.57 mg, 187.96 µmol) to a dichloromethane solution (12 mL) of tert-butyl (4S)-4-[(1R)-1-hydroxyallyl]-2,2-dimethyl-oxazolidine-3-carboxylate (532.02 mg, 2.07 mmol) and tritriacont-1-ene (870 mg, 1.88 mmol). Stir the mixture at 40°C for 4 h under nitrogen protection. Filter the reaction solution, and concentrate the filtrate. Purify, by silica gel flash column chromatography (eluent: 0 to 25% ethyl acetate in petroleum ether), the crude product to obtain tert-butyl (4S)-4-[(E,1R)-1-hydroxytetratriacontyl-2-enyl]-2,2-dimethyl-oxazolidine-3-carboxylate (70 mg, 101.13 µmol, yield 5.38%), which is a white solid.

**¹H NMR** (400 MHz, CDCl₃) δ = 5.80 - 5.70 (m, 1H), 5.48 - 5.40 (m, 1H), 4.17 - 3.95 (m, 4H), 2.09 - 1.98 (m, 2H), 1.61 - 1.55 (m, 15H), 1.31 - 1.24 (m, 58H), 0.88 (t, *J =* 6.8 Hz, 3H).

### Step 4

### (E,2S,3R)-2-aminohexatriacontyl-4-ene-1,3-diol

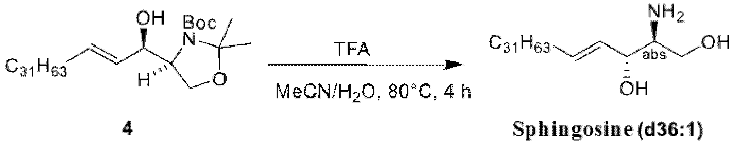

Add an aqueous solution (1 mL) of trifluoroacetic acid (57.66 mg, 505.67 µmol, 37.56 µL) to an acetonitrile solution (1 mL) of tert-butyl (4S)-4-[(E,1R)-1-hydroxytetratriacontyl-2-enyl]-2,2-dimethyl-oxazolidine-3-carboxylate (70 mg, 101.13 µmol). Stir the mixed liquid at 80°C for 4 h. Add a saturated aqueous solution of sodium bicarbonate to the reaction solution to adjust the pH value to 8. Filter in batches, and dry the filter cake under reduced pressure. Disperse the filter cake in hydrochloric acid/dioxane (2 M, 10 mL), and concentrate. Slurry with methanol (10 mL), and freeze dry to obtain compound (E, 2S, 3R)-2-aminohexatriacontyl-4-ene-1,3-diol (32 mg, 54.38 µmol, yield 53.77%, hydrochloride), which is a white solid.

**MS** Rt = 1.348 min in 1.5 min chromatogram, 5-95 AB, ESI calculated value C₃₆H₇₄NO₂ [M+H]⁺ 552.6, and measured value 552.5.

**¹H NMR** (400 MHz, DMSO-d⁶) δ = 7.82-7.58 (m, 2H), 5.84 - 5.68 (m, 1H), 5.50 -5.43 (m, 1H), 5.28 - 5.19 (m, 1H), 5.00 - 4.89 (m, 1H), 4.30 - 4.19 (m, 1H), 3.71 - 3.62 (m, 1H), 3.58 - 3.52 (m, 1H), 3.35-3.32(m, 1H), 2.08-2.00 (m, 2H), 1.31 - 1.25 (m, 58H), 0.88 (t, *J =* 6.8 Hz, 3H).

### Step 5

### Tert-butyl (4S)-4-[(1R)-1-hydroxytetratriacontyl]-2,2-dimethyl-oxazolidine-3-carboxylate

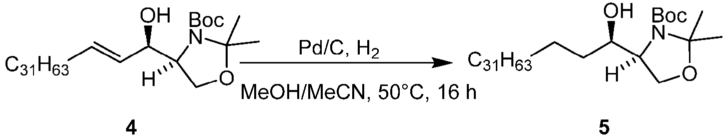

Add Pd/C (130.69 mg, 122.81 µmol, purity 10%) to a mixed solution of tert-butyl (4S)-4-[(E,1R)-1-hydroxytetratriacontyl-2-enyl]-2,2-dimethyl-oxazolidine-3-carboxylate (170 mg, 245.61 µmol) in tetrahydrofuran (2 mL) and methanol (2 mL) under nitrogen protection. Degas the suspension, and replace with hydrogen three times. Stir the mixture under hydrogen atmosphere (50 psi) at 50°C for 16 h. Add tetrahydrofuran (10 mL), and filter. Wash the filter cake three times with tetrahydrofuran (10 mL). Purify the crude product obtained by concentration of the filtrate by silica gel flash column chromatography (eluent: 20% ethyl acetate in petroleum ether) to obtain compound (4S)-4-[(1R)-1-hydroxytetratriacontyl]-2,2-dimethyl-oxazolidine-3-carboxylate (70 mg, 100.84 µmol, yield 41.06%), which is a white solid.

**¹H NMR** (400 MHz, CDCl₃) δ = 4.13 - 3.77 (m, 4H), 1.81 - 1.52 (m, 15H), 1.44 - 1.42 (m, 2H), 1.35 - 1.10 (m, 62H), 0.88 (t, *J =* 6.8 Hz, 3H).

### Step 6

### (2S,3R)-2-aminohexatriacontane-1,3-diol

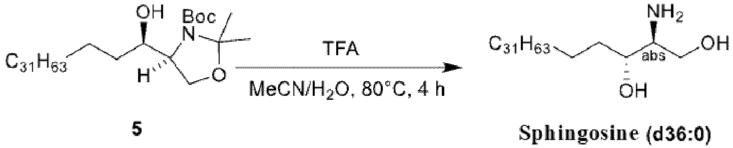

Add an aqueous solution (2 mL) of trifluoroacetic acid (57.49 mg, 504.20 µmol, 37.45 µL) to an acetonitrile solution (2 mL) of tert-butyl (4S)-4-[(1R)-1-hydroxytetratriacontyl]-2,2-dimethyl-oxazolidine-3-caiboxylate (70 mg, 100.84 µmol). Stir the mixture at 80°C for 4 h. Add a saturated aqueous solution of sodium bicarbonate to the reaction solution to adjust the pH value to 8. Filter in batches, and dry the filter cake under reduced pressure. Disperse the filter cake in hydrochloric acid/dioxane (2 M, 10 mL), and stir at room temperature. Slurry the crude product with methanol (10 mL), freeze dry to obtain compound (2S,3R)-2-aminohexatriacontane-1,3-diol (15.2 mg, 25.74 µmol, yield 25.53%, hydrochloride), which is a white solid.

**MS** Rt = 1.586 min in 3 min chromatography, ESI calculated value C₃₆H₇₆NO₂ [M+H]⁺ 554.6, and measured value 554.5.

**¹H NMR** (400 MHz, DMSO-*d⁶*) δ = 7.70 - 7.55 (m, 3H), 5.10 - 4.63 (m, 2H), 3.70 -3.62 (m, 2H), 3.58 - 3.51 (m, 1H), 3.18 - 3.10 (m, 1H), 1.40 - 1.38 (m, 2H), 1.28 - 1.25 (m, 62H), 0.88 (t, *J =* 6.8 Hz, 3H).

The lipids used in the examples of the present invention are sphingosines as shown in Table 1 below.

**Table 1: Sphingosines used in the present invention**

| Serial No. | Abbreviation | IUPAC name | Structure |
|---|---|---|---|
| | Sphingosine (d14:0) | (2S,3R)-2-aminotetradecane-1,3-diol | 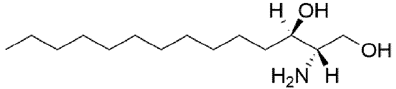 |
| | | ((2S,3R)-2-aminotetradecane-1,3-diol) | |
| | Sphingosine (d15:0) | (2S,3R)-2-aminopentadecane-1,3-diol | 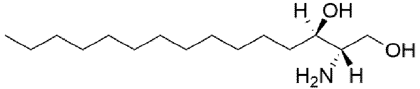 |
| | | ((2S,3R)-2-aminopentadecane-1,3-diol) | |
| | Sphingosine (d16:0) | (2S,3R)-2-aminohexadecane-1,3-diol | 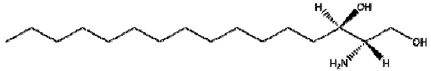 |
| | | ((2S,3R)-2-aminohexadecane-1,3-diol) | |
| | Sphingosine (d17:0) | (2S,3R)-2-aminoheptadecane-1,3-diol | 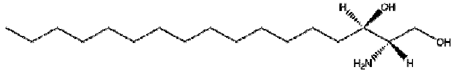 |
| | | ((2S,3R)-2-aminoheptadecane-1,3-diol) | |
| | Sphingosine (d18:0) | (2S,3R)-2-aminooctadecane-1,3-diol | 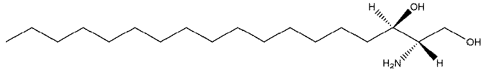 |
| | | ((2S,3R)-2-aminooctadecane-1,3-diol) | |
| | Sphingosine (d19:0) | (2S,3R)-2-aminononadecane-1,3-diol | 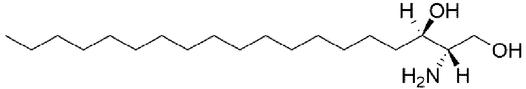 |
| | | ((2S,3R)-2-aminononadecane-1,3-diol) | |
| | Sphingosine (d20:0) | (2 S,3R)-2-aminoicosane-1,3-diol | 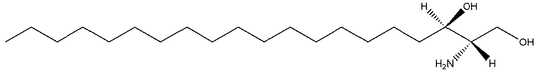 |
| | | ((2S,3R)-2-aminoicosane-1,3-diol) | |
| | Sphingosine (d21:0) | (2S,3R)-2-aminohenicosane-1,3-diol | 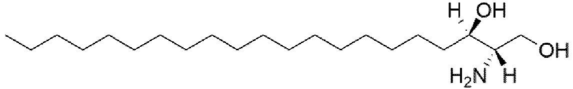 |
| | | ((2S,3R)-2-aminohenicosane-1,3-diol) | |
| | Sphingosine (d22:0) | (2S,3R)-2-aminodocosane-1,3-diol | 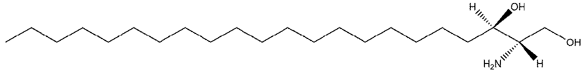 |
| | | ((2S,3R)-2-aminodocosane-1,3-diol) | |
| | Sphingosine (d23:0) | (2S,3R)-2-aminotricosane-1,3-diol | 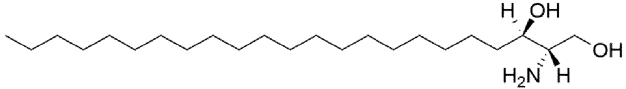 |
| | | ((2S,3R)-2-aminotricosane-1,3-diol) | |
| | Sphingosine (d24:0) | (2S,3R)-2-aminotetracosane-1,3-diol | 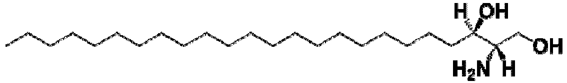 |
| | | ((2S,3R)-2-aminotetracosane-1,3-diol) | |
| | Sphingosine (d25:0) | (2S,3R)-2-aminopentacosane-1,3-diol | 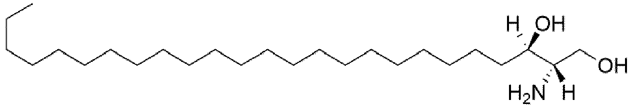 |
| | | ((2S,3R)-2-aminopentacosane-1,3-diol) | |
| | Sphingosine (d26:0) | (2S,3R)-2-aminohexacosane-1,3-diol | 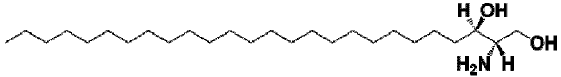 |
| | | ((2S,3R)-2-aminohexacosane-1,3-diol) | |
| | Sphingosine (d27:0) | (2S,3R)-2-aminoheptacosane-1,3-diol | 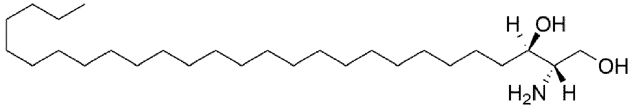 |
| | | ((2S,3R)-2-aminoheptacosane-1,3-dio) | |
| | Sphingosine (d28:0) | (2S,3R)-2-aminooctacosane-1,3-diol | 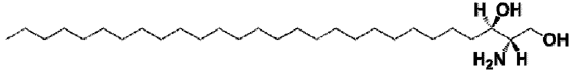 |
| | | ((2S,3R)-2-aminooctacosane-1,3-diol) | |
| | Sphingosine (d29:0) | (2S,3R)-2-aminononacosane-1,3-diol | 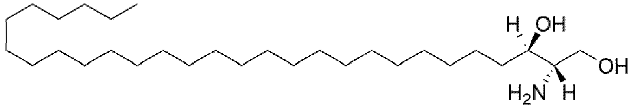 |
| | | ((2S,3R)-2-aminononacosane-1,3-diol) | |
| | Sphingosine (d30:0) | (2S,3R)-2-aminotriacontane-1,3-diol | 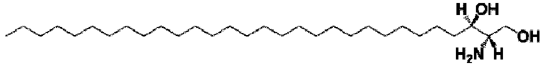 |
| | | ((2S,3R)-2-aminotriacontane-1,3-diol) | |
| | Sphingosine (d31:0) | (2S,3R)-2-aminohentriacontane-1,3-diol | 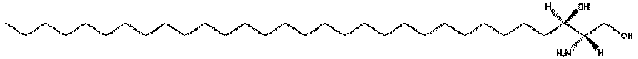 |
| | | ((2S,3R)-2-aminohentriacontane-1,3-diol) | |
| | Sphingosine (d32:0) | (2S,3R)-2-aminodotriacontane-1,3-diol | 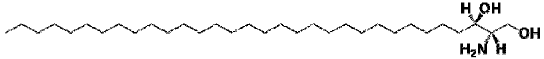 |
| | | ((2S,3R)-2-aminodotriacontane-1,3-diol) | |
| | Sphingosine (d33:0) | (2S,3R)-2-aminotritriacontane-1,3-diol | 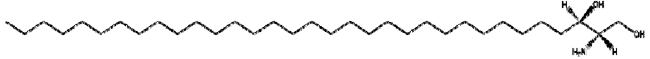 |
| | | ((2S,3R)-2-aminotritriacontane-1,3-diol) | |
| | Sphingosine (d34:0) | (2S,3R)-2-aminotetratriacontane-1,3-diol | 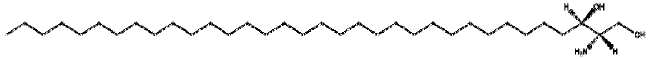 |
| | | ((2S,3R)-2-aminotetratriacontane-1,3-diol) | |
| | Sphingosine (d35:0) | (2S,3R)-2-aminopentatriacontane-1,3-diol | 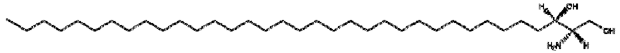 |
| | | ((2S,3R)-2-aminopentatriacontane-1,3-diol) | |
| | Sphingosine (d36:0) | (2S,3R)-2-aminohexatriacontane-1,3-diol | 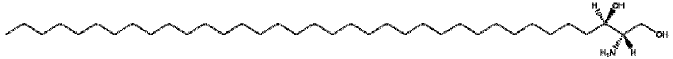 |
| | | ((2S,3R)-2-aminohexatriacontane-1,3-diol) | |
| | Sphingosine (d14:1) | (2S,3R,E)-2-aminotetradec-4-ene-1,3-diol | 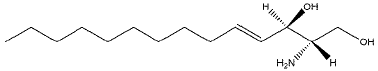 |
| | | ((2S,3R,E)-2-aminotetradec-4-ene-1,3-diol) | |
| | Sphingosine (d15:1) | (2S,3R,E)-2-aminopentadec-4-ene-1,3-diol | 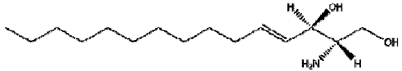 |
| | | ((2S,3R,E)-2-aminopentadec-4-ene-1,3-diol) | |
| | Sphingosine (d16:1) | (2S,3R,E)-2-aminohexadec-4-ene-1,3-diol | 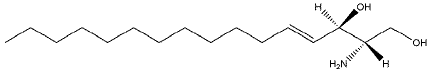 |
| | | ((2S,3R,E)-2-aminohexadec-4-ene-1,3-diol) | |
| | Sphingosine (d17:1) | (2S,3R,E)-2-aminoheptadec-4-ene-1,3-diol | 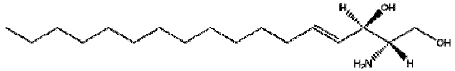 |
| | | ((2S,3R,E)-2-aminoheptadec-4-ene-1,3-diol) | |
| | Sphingosine (d18:1) | (2S,3R,E)-2-aminooctadec-4-ene-1,3-diol | 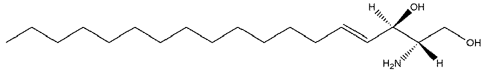 |
| | | ((2S,3R,E)-2-aminooctadec-4-ene-1,3-diol) | |
| | Sphingosine (d19:1) | (2S,3R,E)-2-aminononadec-4-ene-1,3-diol | 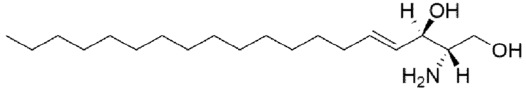 |
| | | ((2S,3R,E)-2-aminononadec-4-ene-1,3-diol) | |
| | Sphingosine (d20:1) | (2S,3R,E)-2-aminoicos-4-ene-1,3-diol | 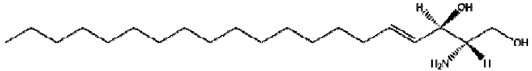 |
| | | ((2S,3R,E)-2-aminoicos-4-ene-1,3-diol) | |
| | Sphingosine (d21:1) | (2S,3R,E)-2-aminohenicos-4-ene-1,3-diol | 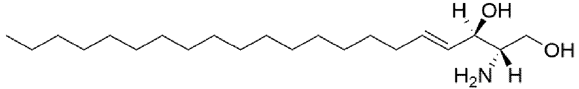 |
| | | ((2S,3R,E)-2-aminohenicos-4-ene-1,3-diol) | |
| | Sphingosine (d22:1) | (2S,3R,E)-2-aminodocos-4-ene-1,3-diol | 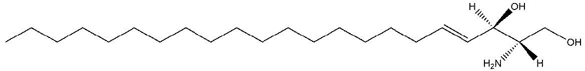 |
| | | ((2S,3R,E)-2-aminodocos-4-ene-1,3-diol) | |
| | Sphingosine (d23:1) | (2S,3R,E)-2-aminotricos-4-ene-1,3-diol | 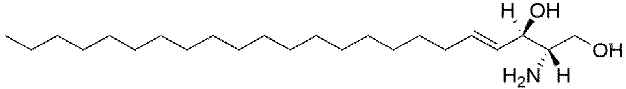 |
| | | ((2S,3R,E)-2-aminotricos-4-ene-1,3-diol) | |
| | Sphingosine (d24: 1) | (2S,3R,E)-2-aminotetracos-4-ene-1,3-diol | 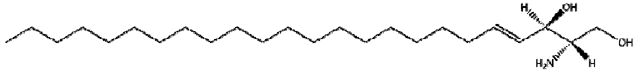 |
| | | ((2S,3R,E)-2-aminotetracos-4-ene-1,3-diol) | |
| | Sphingosine (d25:1) | (2S,3R,E)-2-aminopentacos-4-ene-1,3-diol | 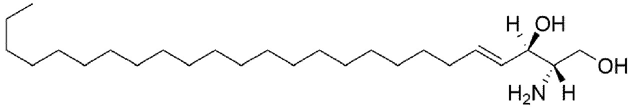 |
| | | ((2S,3R,E)-2-aminopentacos-4-ene-1,3-diol) | |
| | Sphingosine (d26: 1) | (2S,3R,E)-2-aminohexacos-4-ene-1,3-diol | 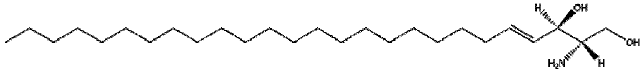 |
| | | ((2S,3R,E)-2-aminohexacos-4-ene-1,3-diol) | |
| | Sphingosine (d27:1) | (2S,3R,E)-2-aminoheptacos-4-ene-1,3-diol | 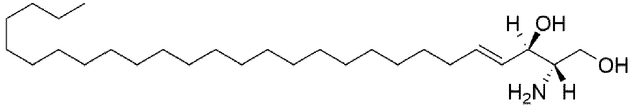 |
| | | ((2S,3R,E)-2-aminoheptacos-4-ene-1,3-diol) | |
| | Sphingosine (d28:1) | (2S,3R,E)-2-aminooctacos-4-ene-1,3-diol | 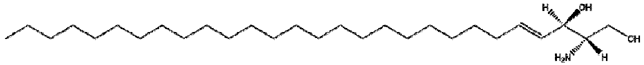 |
| | | ((2S,3R,E)-2-aminooctacos-4-ene-1,3-diol) | |
| | Sphingosine (d29:1) | (2S,3R,E)-2-aminononacos-4-ene-1,3-diol | 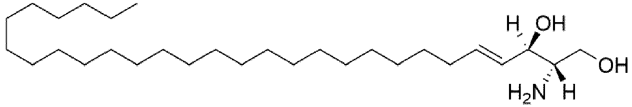 |
| | | ((2S,3R,E)-2-aminononacos-4-ene-1,3-diol) | |
| | Sphingosine (d30:1) | (2S,3R,E)-2-aminotriacont-4-ene-1,3-diol | 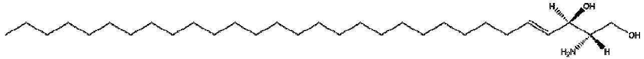 |
| | | ((2S,3R,E)-2-aminotriacont-4-ene-1,3-diol) | |
| | Sphingosine (d31:1) | (2S,3R,E)-2-aminohentriacont-4-ene-1,3-diol | 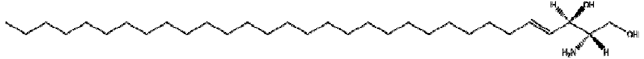 |
| | | ((2S,3R,E)-2-aminohentriacont-4-ene-1,3-diol) | |
| | Sphingosine (d32:1) | (2S,3R,E)-2-aminodotriacont-4-ene-1,3-diol | 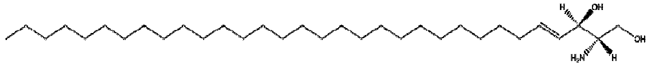 |
| | | ((2S,3R,E)-2-aminodotriacont-4-ene-1,3-diol) | |
| | Sphingosine (d33:1) | (2S,3R,E)-2-aminotritriacont-4-ene-1,3-diol | 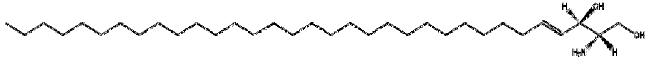 |
| | | ((2S,3R,E)-2-aminotritriacont-4-ene-1,3-diol) | |
| | Sphingosine (d34:1) | (2S,3R,E)-2-aminotetratriacont-4-ene-1,3-diol | 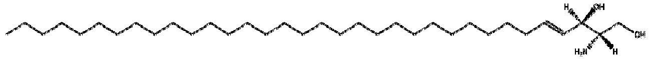 |
| | | ((2S,3R,E)-2-aminotetratriacont-4-ene-1,3-diol) | |
| | Sphingosine (d35:1) | (2S,3R,E)-2-aminopentatriacont-4-ene-1,3-diol | 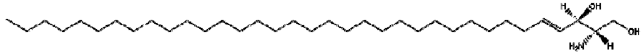 |
| | | ((2S,3R,E)-2-aminopentatriacont-4-ene-1,3-diol) | |
| | Sphingosine (d36:1) | (2S,3R,E)-2-aminohexatriacont-4-ene-1,3-diol | 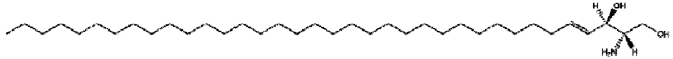 |
| | | ((2S,3R,E)-2-aminohexatriacont-4-ene-1,3-diol) | |

### Example 2. Study on in vitro delivery of small nucleic acids using lipid-nucleic acid complexes

### 1. Cell culture

Culture the human pancreatic acinar epithelial cancer cell line HPAC, human large cell lung cancer cell line H460, and human embryonic kidney cell line HEK293T (purchased from the Cell Resource Center, Institute of Basic Medicine, Chinese Academy of Medical Sciences) used in the experiments in a 37°C, 5%CO₂ incubator. Culture the HPAC cells in DMEM/F12 medium, the H460 cells in RPMI-1640 medium, and the 293T cells in DMEM medium containing 10% fetal bovine serum and 1% antibiotics (penicillin 100 U/mL & streptomycin 100 mg/mL). Culture the cells to the logarithmic growth phase with a cell density of 6 × 10⁵/1 mL medium/well; divide the cells into 12-well plates (1 mL medium/well), and incubate at 37°C overnight (12 h), and then conduct subsequent experiments.

### 2. Preparation of lipid-RNA mixtures

Add 5 µL of nucleic acid and 95 µL of DEPC-treated water to a 2 mL glass tube, mix homogeneously, add a certain amount of lipid monomer, mix thoroughly, heat in a 90°C water bath for 15 min, and then cool naturally to obtain a mixture of nucleic acid and lipid.

Illustratively, the RNA PGY-ssRNA-26 used in this example has the sequence UCCGGAAUGAUUGGGCGUAAAGCGU (SEQ ID NO: 1).

3. Detection of cell uptake of nucleic acids delivered by lipids inside cells by means of flow cytometry (CFlow)
1) Main experimental instruments and equipment:
   10 cm cell culture dishes, 12-well cell culture plates, pipettes, transfer tubes, optical microscope, flow cytometer CytoFLEX instrument (purchased from Beckman, USA)
2) Main experimental reagents:
   Model establishment and transfection: artificially synthesized lipid monomers and nucleic acids as shown in the table
3) Culture the human pancreatic acinar epithelial cancer cell line HPAC cells, human large cell lung cancer cell line H460 cells, and human embryonic kidney cell line HEK293T cells used in the experiment to the logarithmic growth phase, and then divide them into 12-well plates at a cell density of 6×10⁵/mL culture medium/well; incubate the 12-well plates at 37°C overnight (12 h), and then conduct subsequent experiments.
4) The experimental groups are as follows:
   a) Blank group: refers to untreated cells, and this group serves as a blank control group.
   b) Free uptake group: directly add 5 µL of fluorescently labeled nucleic acid solution (stock concentration 20 µM), and this group serves as the negative control group.
   c) Lipid-nucleic acid mixture treatment group: add the mixture of lipids and fluorescently labeled nucleic acids prepared in step 2 to the cells, and mix homogeneously. The final concentration of nucleic acids is 100 nM.
5) After incubating with cells for 9 h, wash the cells three times with PBS, resuspend the cells with PBS (self-prepared), and use a flow cytometer CytoFLEX instrument (purchased from Beckman, USA) to detect the fluorescence intensity of the cells in the sample wells.

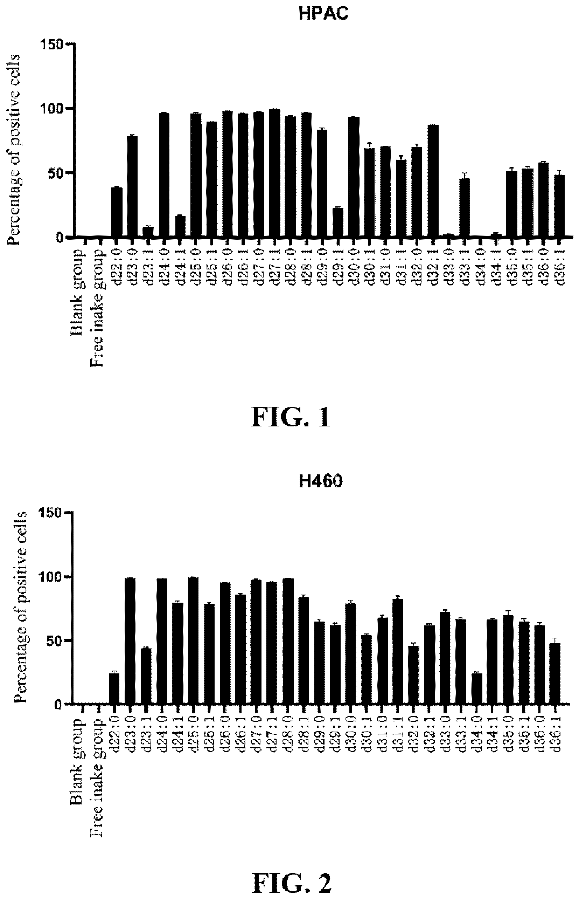

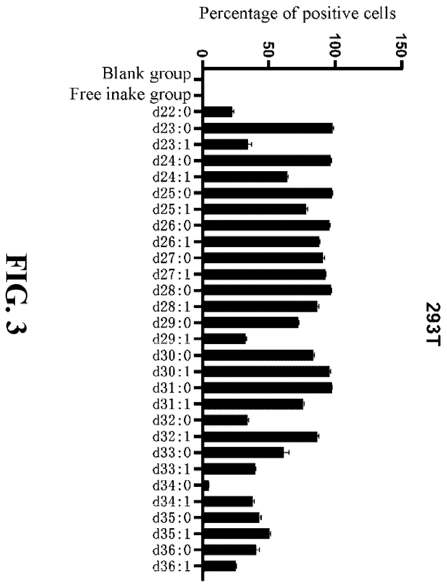

The results of in vitro small nucleic acid delivery in this example are shown in Tables 2 to 4 below and FIGS. 1 to 3.

All the small nucleic acids used in Tables 2 to 4 are PGY-ssRNA-26.

**Table 2**

| Lipid name | Blank Group | Free intake group | Lipid delivery group (1.25 ug/mL) | Delivery sequence | Cell lines |
|---|---|---|---|---|---|
| Sphingosine (d22:0) | 0.00% | 0.10% | 39.08% | PGY-ssRNA-26 | HPAC |
| Sphingosine (d23:0) | 0.00% | 0.10% | 78.91% | PGY-ssRNA-26 | HPAC |
| Sphingosine (d23:1) | 0.00% | 0.10% | 7.93% | PGY-ssRNA-26 | HPAC |
| Sphingosine (d24:0) | 0.00% | 0.10% | 96.61% | PGY-ssRNA-26 | HPAC |
| Sphingosine (d24:1) | 0.00% | 0.10% | 16.65% | PGY-ssRNA-26 | HPAC |
| Sphingosine (d25:0) | 0.00% | 0.10% | 96.17% | PGY-ssRNA-26 | HPAC |
| Sphingosine (d25:1) | 0.00% | 0.10% | 89.70% | PGY-ssRNA-26 | HPAC |
| Sphingosine (d26:0) | 0.00% | 0.10% | 97.75% | PGY-ssRNA-26 | HPAC |
| Sphingosine (d26:1) | 0.00% | 0.10% | 96.03% | PGY-ssRNA-26 | HPAC |
| Sphingosine (d27:0) | 0.00% | 0.10% | 97.35% | PGY-ssRNA-26 | HPAC |
| Sphingosine (d27:1) | 0.00% | 0.10% | 99.42% | PGY-ssRNA-26 | HPAC |
| Sphingosine (d28:0) | 0.00% | 0.10% | 94.40% | PGY-ssRNA-26 | HPAC |
| Sphingosine (d28:1) | 0.00% | 0.10% | 96.66% | PGY-ssRNA-26 | HPAC |
| Sphingosine (d29:0) | 0.00% | 0.10% | 83.57% | PGY-ssRNA-26 | HPAC |
| Sphingosine (d29:1) | 0.00% | 0.10% | 23.36% | PGY-ssRNA-26 | HPAC |
| Sphingosine (d30:0) | 0.00% | 0.10% | 93.63% | PGY-ssRNA-26 | HPAC |
| Sphingosine (d30:1) | 0.00% | 0.10% | 69.34% | PGY-ssRNA-26 | HPAC |
| Sphingosine (d31:0) | 0.00% | 0.10% | 70.5% | PGY-ssRNA-26 | HPAC |
| Sphingosine (d31:1) | 0.00% | 0.10% | 60.61% | PGY-ssRNA-26 | HPAC |
| Sphingosine (d32:0) | 0.00% | 0.10% | 70.16% | PGY-ssRNA-26 | HPAC |
| Sphingosine (d32:1) | 0.00% | 0.10% | 87.38% | PGY-ssRNA-26 | HPAC |
| Sphingosine (d33:0) | 0.00% | 0.10% | 2.48% | PGY-ssRNA-26 | HPAC |
| Sphingosine (d33:1) | 0.00% | 0.10% | 45.88% | PGY-ssRNA-26 | HPAC |
| Sphingosine (d34:0) | 0.00% | 0.10% | 0.55% | PGY-ssRNA-26 | HPAC |
| Sphingosine (d34:1) | 0.00% | 0.10% | 2.95% | PGY-ssRNA-26 | HPAC |
| Sphingosine (d35:0) | 0.00% | 0.10% | 51.46% | PGY-ssRNA-26 | HPAC |
| Sphingosine (d35:1) | 0.00% | 0.10% | 53.75% | PGY-ssRNA-26 | HPAC |
| Sphingosine (d36:0) | 0.00% | 0.10% | 58.22% | PGY-ssRNA-26 | HPAC |
| Sphingosine (d36:1) | 0.00% | 0.10% | 48.77% | PGY-ssRNA-26 | HPAC |

**Table 3**

| Lipid name | Blank Group | Free intake group | Lipid delivery group (1.25 ug/mL) | Delivery sequence | Cell lines |
|---|---|---|---|---|---|
| Sphingosine (d22:0) | 0.00% | 0.20% | 24.32% | PGY-ssRNA-26 | H460 |
| Sphingosine (d23:0) | 0.00% | 0.20% | 98.65% | PGY-ssRNA-26 | H460 |
| Sphingosine (d23:1) | 0.00% | 0.20% | 44.11% | PGY-ssRNA-26 | H460 |
| Sphingosine (d24:0) | 0.00% | 0.20% | 98.13% | PGY-ssRNA-26 | H460 |
| Sphingosine (d24: 1) | 0.00% | 0.20% | 79.66% | PGY-ssRNA-26 | H460 |
| Sphingosine (d25:0) | 0.00% | 0.20% | 99.26% | PGY-ssRNA-26 | H460 |
| Sphingosine (d25:1) | 0.00% | 0.20% | 78.43% | PGY-ssRNA-26 | H460 |
| Sphingosine (d26:0) | 0.00% | 0.20% | 95.2% | PGY-ssRNA-26 | H460 |
| Sphingosine (d26: 1) | 0.00% | 0.20% | 85.87% | PGY-ssRNA-26 | H460 |
| Sphingosine (d27:0) | 0.00% | 0.20% | 97.57% | PGY-ssRNA-26 | H460 |
| Sphingosine (d27:1) | 0.00% | 0.20% | 95.74% | PGY-ssRNA-26 | H460 |
| Sphingosine (d28:0) | 0.00% | 0.20% | 98.52% | PGY-ssRNA-26 | H460 |
| Sphingosine (d28:1) | 0.00% | 0.20% | 84.15% | PGY-ssRNA-26 | H460 |
| Sphingosine (d29:0) | 0.00% | 0.20% | 65.81% | PGY-ssRNA-26 | H460 |
| Sphingosine (d29:1) | 0.00% | 0.20% | 62.37% | PGY-ssRNA-26 | H460 |
| Sphingosine (d30:0) | 0.00% | 0.20% | 78.84% | PGY-ssRNA-26 | H460 |
| Sphingosine (d30:1) | 0.00% | 0.20% | 54.83% | PGY-ssRNA-26 | H460 |
| Sphingosine (d31:0) | 0.00% | 0.20% | 68.16% | PGY-ssRNA-26 | H460 |
| Sphingosine (d31:1) | 0.00% | 0.20% | 82.66% | PGY-ssRNA-26 | H460 |
| Sphingosine (d32:0) | 0.00% | 0.20% | 46.19% | PGY-ssRNA-26 | H460 |
| Sphingosine (d32:1) | 0.00% | 0.20% | 62.18% | PGY-ssRNA-26 | H460 |
| Sphingosine (d33:0) | 0.00% | 0.20% | 72.21% | PGY-ssRNA-26 | H460 |
| Sphingosine (d33:1) | 0.00% | 0.20% | 67.01% | PGY-ssRNA-26 | H460 |
| Sphingosine (d34:0) | 0.00% | 0.20% | 24.56% | PGY-ssRNA-26 | H460 |
| Sphingosine (d34:1) | 0.00% | 0.20% | 66.64% | PGY-ssRNA-26 | H460 |
| Sphingosine (d35:0) | 0.00% | 0.20% | 69.95% | PGY-ssRNA-26 | H460 |
| Sphingosine (d35:1) | 0.00% | 0.20% | 64.66% | PGY-ssRNA-26 | H460 |
| Sphingosine (d36:0) | 0.00% | 0.20% | 62.71% | PGY-ssRNA-26 | H460 |
| Sphingosine (d36:1) | 0.00% | 0.20% | 48.37% | PGY-ssRNA-26 | H460 |

**Table 4**

| Lipid name | Blank Group | Free intake group | Lipid delivery group (1.25 ug/mL) | Delivery sequence | Cell lines |
|---|---|---|---|---|---|
| Sphingosine (d22:0) | 0.00% | 0.60% | 22.99% | PGY-ssRNA-26 | 293T |
| Sphingosine (d23:0) | 0.00% | 0.60% | 98.16% | PGY-ssRNA-26 | 293T |
| Sphingosine (d23:1) | 0.00% | 0.60% | 34.51% | PGY-ssRNA-26 | 293T |
| Sphingosine (d24:0) | 0.00% | 0.60% | 96.77% | PGY-ssRNA-26 | 293T |
| Sphingosine (d24: 1) | 0.00% | 0.60% | 63.97% | PGY-ssRNA-26 | 293T |
| Sphingosine (d25:0) | 0.00% | 0.60% | 97.76% | PGY-ssRNA-26 | 293T |
| Sphingosine (d25:1) | 0.00% | 0.60% | 78.12% | PGY-ssRNA-26 | 293T |
| Sphingosine (d26:0) | 0.00% | 0.60% | 95.55% | PGY-ssRNA-26 | 293T |
| Sphingosine (d26: 1) | 0.00% | 0.60% | 87.97% | PGY-ssRNA-26 | 293T |
| Sphingosine (d27:0) | 0.00% | 0.60% | 91.06% | PGY-ssRNA-26 | 293T |
| Sphingosine (d27:1) | 0.00% | 0.60% | 92.77% | PGY-ssRNA-26 | 293T |
| Sphingosine (d28:0) | 0.00% | 0.60% | 96.93% | PGY-ssRNA-26 | 293T |
| Sphingosine (d28:1) | 0.00% | 0.60% | 86.5% | PGY-ssRNA-26 | 293T |
| Sphingosine (d29:0) | 0.00% | 0.60% | 72.18% | PGY-ssRNA-26 | 293T |
| Sphingosine (d29:1) | 0.00% | 0.60% | 32.74% | PGY-ssRNA-26 | 293T |
| Sphingosine (d30:0) | 0.00% | 0.60% | 83.38% | PGY-ssRNA-26 | 293T |
| Sphingosine (d30:1) | 0.00% | 0.60% | 95.79% | PGY-ssRNA-26 | 293T |
| Sphingosine (d31:0) | 0.00% | 0.60% | 97.4% | PGY-ssRNA-26 | 293T |
| Sphingosine (d31:1) | 0.00% | 0.60% | 75.77% | PGY-ssRNA-26 | 293T |
| Sphingosine (d32:0) | 0.00% | 0.60% | 34.02% | PGY-ssRNA-26 | 293T |
| Sphingosine (d32:1) | 0.00% | 0.60% | 86.77% | PGY-ssRNA-26 | 293T |
| Sphingosine (d33:0) | 0.00% | 0.60% | 61.39% | PGY-ssRNA-26 | 293T |
| Sphingosine (d33:1) | 0.00% | 0.60% | 40.07% | PGY-ssRNA-26 | 293T |
| Sphingosine (d34:0) | 0.00% | 0.60% | 5% | PGY-ssRNA-26 | 293T |
| Sphingosine (d34:1) | 0.00% | 0.60% | 38.31% | PGY-ssRNA-26 | 293T |
| Sphingosine (d35:0) | 0.00% | 0.60% | 43.03% | PGY-ssRNA-26 | 293T |
| Sphingosine (d35:1) | 0.00% | 0.60% | 50.79% | PGY-ssRNA-26 | 293T |
| Sphingosine (d36:0) | 0.00% | 0.60% | 40.66% | PGY-ssRNA-26 | 293T |
| Sphingosine (d36:1) | 0.00% | 0.60% | 25.23% | PGY-ssRNA-26 | 293T |

The results show that compared with the negative control group, the fluorescence offset in the experimental group increases, indicating that the cells take up more fluorescently labeled RNAs, that is, the amount of RNAs delivered into the cells by lipids is higher, which reflects a higher delivery efficiency. From the results in Tables 2 to 4 above, it can be seen that compared with the free uptake group, the fluorescence value of the experimental group is significantly shifted after the sphingosine lipid delivers 0.1 nmol nucleic acid at a concentration of 1.25 ug/mL, indicating that various sphingosine monomers can all effectively deliver sRNA into cells. Sphingosine lipid monomers are effective for nucleic acid delivery, especially the sphingosine of the present invention having a carbon chain length that is greater than 21.

## Claims

1. Use of a lipid composition in the preparation of a product for delivering nucleic acids, the lipid composition comprising one or more compounds having the following formula (I): 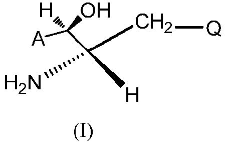 wherein
A is selected from linear C₁₀₋₃₄ alkyl groups and linear C₁₀₋₃₄ alkenyl groups, preferably linear C₁₀₋₃₂ alkyl groups and linear C₁₀₋₃₂ alkenyl groups;
Q is -OH, and preferably, the lipid composition comprises one or more compounds shown in Table 1.

2. The use according to claim 1, wherein the lipid composition comprises two or more compounds of formula (I), and optionally, the lipid composition further comprises other lipids than the compounds of formula (I).

3. The use according to claim 1 or 2, wherein the product is used to deliver a nucleic acid into the body of a subject, and preferably, the reagent is used to deliver a nucleic acid into the body of a subject via oral, intramuscular, intravenous, subcutaneous, transdermal, intraarterial, intraperitoneal, intrapulmonary, intracerebrospinal, intraarticular, intrasynovial, intrathecal, intraventricular, and/or inhalation route.

4. The use according to claim 1 or 2, wherein the product is used to deliver a nucleic acid to cells in vitro, preferably, to deliver a nucleic acid to cells in vitro by direct contact, and preferably, the cells are tumor cells.

5. The use according to any one of claims 1 to 4, wherein the nucleic acid is an RNA molecule or a DNA molecule, preferably,
the RNA molecule is a small RNA molecule, preferably, the RNA molecule is a small RNA molecule with a length of 14-32 nucleotides, and preferably, the nucleic acid molecule is a small RNA molecule with a length of 14, 15, 16, 17, 18, 19, 20, 21, 22, 23, 24, 25, 26, 27, 28, 29, 30, 31, or 32 nucleotides.

6. A pharmaceutical composition comprising a lipid composition and a nucleic acid molecule, wherein the lipid composition comprises one or more compounds of formula (I): 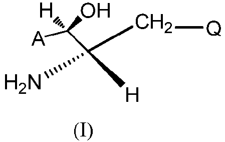 wherein
A is selected from linear C₁₀₋₃₄ alkyl groups and linear C₁₀₋₃₄ alkenyl groups, preferably linear C₁₀₋₃₂ alkyl groups and linear C₁₀₋₃₂ alkenyl groups;
Q is -OH,
and preferably, the lipid composition comprises one or more compounds shown in Table 1.

7. The pharmaceutical composition according to claim 6, wherein the nucleic acid molecule is an RNA molecule or a DNA molecule, preferably,
the nucleic acid molecule is a small RNA molecule, preferably, the nucleic acid molecule is a small RNA molecule with a length of 14-32 nucleotides, and preferably, the nucleic acid molecule is a small RNA molecule with a length of 14, 15, 16, 17, 18, 19, 20, 21, 22, 23, 24, 25, 26, 27, 28, 29, 30, 31, or 32 nucleotides.

8. The pharmaceutical composition according to any one of claims 6 to 7, wherein the mass ratio of the lipid composition to the nucleic acid molecule is 1:100 to 100:1.

9. The pharmaceutical composition according to claim 8, wherein the mass ratio of the lipid composition to the nucleic acid molecule is 1:100, 1:30, 1:10, 1:3, 1:1, 3:1, 10:1, 30:1, 100:1, or a range between any of the above ratios.

10. The pharmaceutical composition according to any one of claims 6 to 9, wherein the pharmaceutical composition further comprises a pharmaceutically acceptable carrier or excipient.

11. The pharmaceutical composition according to any one of claims 6 to 10, wherein the nucleic acid molecule is an RNA molecule or a DNA molecule for treatment; preferably, the nucleic acid molecule is used to treat a disease by targeting a specific target.

12. A method for preparing the pharmaceutical composition according to any one of claims 6 to 11, comprising the following steps:
1) mixing the lipid composition with the nucleic acid molecule;
wherein the lipid composition is as defined in any one of claims 6 and 8; and the nucleic acid molecule is as defined in any one of claims 7, 8, and 9.

13. The method according to claim 12, further comprising the following step:
2) at 25°C to 150°C,
preferably, at 25°C, 30°C, 35°C, 36°C, 37°C, 40°C, 45°C, 50°C, 55°C, 60°C, 65°C, 70°C, 75°C, 80°C, 85°C, 90°C, 95°C, 100°C, 110°C, 120°C, 130°C, 140°C, or 150°C, or a temperature in any range between these points,
heating the mixture obtained in step 1) of claim 12 for at least 5 min,
and preferably, heating for 6, 7, 8, 9, 10, 11, 12, 13, 14, 15, 16, 17, 18, 19, 20, 21, 22, 23, 24, 25, 26, 27, 28, 29, or 30 min.

14. Use of the pharmaceutical composition according to any one of claims 6 to 11 or the pharmaceutical composition prepared by the method according to any one of claims 12 to 13 in the preparation of a medicament for treating a disease in a subject, preferably, the medicament being administered to the subject by oral, intramuscular, intravenous, subcutaneous, transdermal, intraarterial, intraperitoneal, intrapulmonary, intracerebrospinal, intraarticular, intrasynovial, intrathecal, intraventricular, and/or inhalation route.

15. The use according to claim 14, wherein the disease is selected from cancer, inflammation, fibrotic diseases, autoimmune diseases, infections, congenital and hereditary diseases, connective tissue diseases, digestive system diseases, endocrine diseases, eye diseases, reproductive diseases, cardiovascular diseases, renal and urinary diseases, respiratory diseases, metabolic disorders, musculoskeletal diseases, nervous system diseases, and blood system diseases.

16. The use according to claim 15, wherein the cancer is selected from lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, kidney cancer, squamous cell carcinoma, or blood system cancer.

17. A composition for in vitro cell transfection, comprising a lipid composition and a nucleic acid molecule, wherein the lipid composition comprises one or more compounds of formula (I): 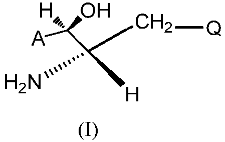 wherein
A is selected from linear C₁₀₋₃₄ alkyl groups and linear C₁₀₋₃₄ alkenyl groups, preferably linear C₁₀₋₃₂ alkyl groups and linear C₁₀₋₃₂ alkenyl groups;
Q is -OH;
and the nucleic acid molecule is a small RNA molecule.

18. A compound represented by the following formula (I): 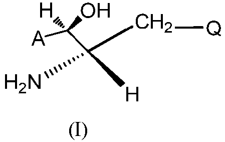 wherein
the A is selected from linear C₁₀₋₃₄ alkyl groups and linear C₁₀₋₃₄ alkenyl groups, preferably linear C₁₀₋₃₂ alkyl groups and linear C₁₀₋₃₂ alkenyl groups;
Q is -OH,
and preferably, the compound is a compound shown in Table 1.

19. A method for preparing the compound represented by formula (I) according to claim 18, wherein the method is selected from any one of the following methods a to c:
Method a. 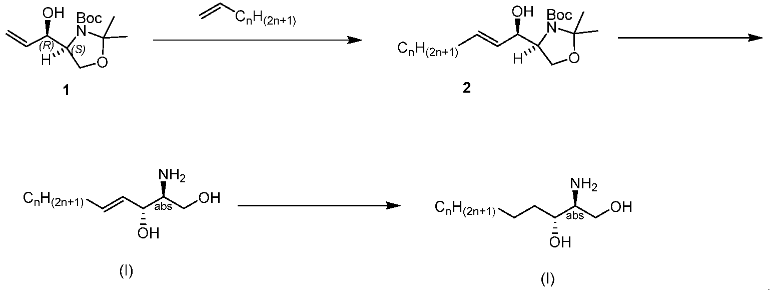 comprising:
Step a1. reacting a compound represented by formula 1 with an olefin to generate a compound represented by formula 2;
Step a2. converting the compound represented by formula 2 to a compound represented by formula (I) in which A is a linear alkenyl group; and
Optional step a3. reducing the compound represented by formula (I) in which A is a linear alkenyl group to obtain the compound represented by formula (I) in which A is a linear alkyl group;
wherein n=9, 10, 11, 12, 13, 14, 15, 16, 17, 18, 19, 20, 21, 22, 23, 24, 25, 26, 27, 28, 29, 30, 31, or 32;
Method b. 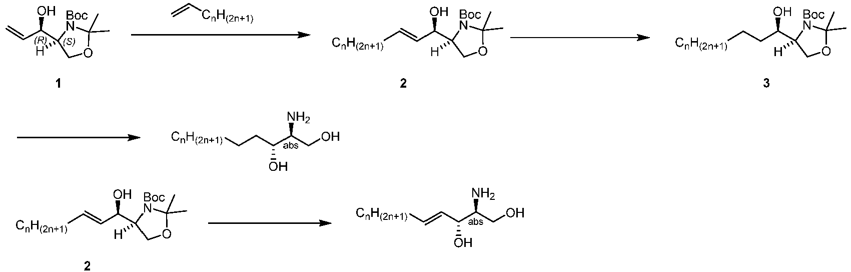 comprising:
Step b1. reacting a compound represented by formula 1 with an olefin to generate a compound represented by formula 2; and
Step b2-1. reducing the compound represented by formula 2 to obtain a compound represented by formula 3;
Step b3. converting the compound represented by formula 3 to a compound represented by formula (I) in which A is a linear alkyl group;
or
Step b2-2. converting the compound represented by formula 2 to a compound represented by formula (I) in which A is a linear alkenyl group;
wherein n=9, 10, 11, 12, 13, 14, 15, 16, 17, 18, 19, 20, 21, 22, 23, 24, 25, 26, 27, 28, 29, 30, 31, or 32;
Method c.
